# EUROPEAN PATENT SPECIFICATION

(11) **EP 3 875 151 B1**
(45) Date of publication and mention of the grant of the patent: **20.09.2023**
(21) Application number: 20160434.5
(22) Date of filing: 02.03.2020
(51) Int. Cl.: A61P 3/00, A61P 25/00, A61P 27/00, A61P 35/00, C07D 471/08, A61K 31/635, A61K 31/551

(54) **ALPHA-METHYL-SUBSTITUTED DIAZABICYCLO [4.3.1] DECANE DERIVATES FOR TREATMENT OF PSYCHIATRIC DISORDERS**
ALPHA-METHYL-SUBSTITUIERTE DIAZABICYCLO[4.3.1]DECAN-DERIVATE ZUR BEHANDLUNG VON PSYCHIATRISCHEN ERKRANKUNGEN
DÉRIVÉS DE DIAZABICYCLO [4.3.1] DÉCANE ALPHA-MÉTHYL-SUBSTITUÉS POUR LE TRAITEMENT DE TROUBLES PSYCHIATRIQUES

(43) Date of publication of application: 08.09.2021
(73) Proprietor: Technische Universität Darmstadt, 64289 Darmstadt (DE); Max-Planck-Gesellschaft zur Förderung der Wissenschaften e.V., 80539 München (DE)
(72) Inventor: Hausch, Felix, 64354 Reinheim (DE); Kolos, Jürgen, 64293 Darmstadt (DE); Pomplun, Sebastian, Cambridge, MA 02140 (US)
(74) Representative: Fuchs Patentanwälte Partnerschaft mbB

(56) References cited:
- EP-A1- 2 690 102
- EP-A1- 2 899 192
- WO-A1-2015/110271
- SEBASTIAN POMPLUN ET AL: "Chemogenomic Profiling of Human and Microbial FK506-Binding Proteins", JOURNAL OF MEDICINAL CHEMISTRY, vol. 61, no. 8, 26 April 2018 (2018-04-26) , pages 3660-3673, XP055594684, US ISSN: 0022-2623, DOI: 10.1021/acs.jmedchem.8b00137
- YANSONG WANG ET AL: "Increasing the Efficiency of Ligands for FK506-Binding Protein 51 by Conformational Control", JOURNAL OF MEDICINAL CHEMISTRY, vol. 56, no. 10, 23 May 2013 (2013-05-23), pages 3922-3935, XP055102631, ISSN: 0022-2623, DOI: 10.1021/jm400087k
- SEBASTIAN POMPLUN ET AL: "Rational Design and Asymmetric Synthesis of Potent and Neurotrophic Ligands for FK506-Binding Proteins (FKBPs)", ANGEWANDTE CHEMIE, INTERNATIONAL EDITION, vol. 54, no. 1, 2 January 2015 (2015-01-02), pages 345-348, XP055690340, DE ISSN: 1433-7851, DOI: 10.1002/anie.201408776
- MATTHIAS BISCHOFF ET AL: "Stereoselective Construction of the 5-Hydroxy Diazabicyclo[4.3.1]decane-2-one Scaffold, a Privileged Motif for FK506-Binding Proteins", ORGANIC LETTERS, vol. 16, no. 20, 6 October 2014 (2014-10-06), pages 5254-5257, XP055690330, ISSN: 1523-7060, DOI: 10.1021/ol5023195

## Description

The present invention relates to alpha-methyl substituted diazabicyclo-[4.3.1]-decane derivatives and stereo-isomeric forms, solvates, hydrates and/or pharmaceutically acceptable salts of these compounds as well as pharmaceutical compositions containing at least one of these alpha-methyl substituted bicyclic aza-amides derivatives together with pharmaceutically acceptable carrier, excipient and/or diluents. Said alpha-methyl substituted diazabicyclo-[4.3.1]-decane derivatives have been identified as especially potent inhibitors of the FK506 binding proteins (FKBPs), especially FKBP12, FKBP12.6, FKBP51 and FKBP52 or bacterial homologs like LpMIP, CpMIP or CtMIP, and are useful for the psychiatric, metabolic, infective, neurological and hematologial disorders as well as pain diseases and cancers.

### Background of the invention

The FK506-binding protein (FKBP) family of immunophilins consists of proteins with a variety of protein-protein interaction domains and versatile cellular functions. The bacterial homologs are also called Macrophage Infectivity potentiators (MIP). This highly conserved protein family binds with immunosuppressive drugs, such as FK506 and rapamycin. This protein family displays peptidyl propyl isomerase (PPlase) activity as seen with cyclophilins and parvulins. FKBP12, a 12 kD protein is the most widely studied member of this family.

The immunosuppressant drugs FK506, rapamycin, and cyclosporin are well known as potent T-cell specific immunosuppressants, and are effective against autoimmunity, transplant or graft rejection, inflammation, allergic responses, other autoimmune or immune-mediated diseases.

FK506 and rapamycin apart from binding to FKBP12 also interact and inhibit calcineurin (CaN) and mTOR, respectively, thereby mediating their immunosuppressive action.

FKBP12 and FKBP12.6 are regulators of ryanodine receptors and of receptors from the TGF/ALK family, and are involved for example in hematologoical and neurological disorder. The high molecular weight multidomain homologs of FKBP51 and FKBP 52, act as cochaperones for the heat shock protein 90 (Hsp90) and modulate the signal transduction of the glucocorticoid receptor by participating in the Heat shock protein 90 (Hsp90) - steroid receptor complex.

In this complex, FKBP51 and FKBP52 modulate the binding competence and signalling of steroid hormone receptors and thereby regulate the cellular responsiveness to circulating hormone levels. This is supported by a natural animal model (squirrel monkey) and by knockout mice, where the crucial role of FKPB51 and FKBP52 on the Glucocorticoid Receptor (GR) Progesterone Receptor (PR) or Androgen Receptor (AR) activity have been clearly demonstrated. Moreover, polymorphisms in the FKBP51-encoding gene of psychiatric patients have been associated with numerous stress-related psychiatric disorders, with diabetes and obesity, and with chronic pain states.

Bacterial FKBPs, also called MIP, are involved in various steps of the infectivity process or the replication of the bacterial pathogens.

The immunosuppressive compounds disclosed in the prior art suppress the immune system, by definition, and also exhibit other toxic side effects. Accordingly, there is a need for non-immunosuppressant, small molecule compounds, and compositions and methods for use of such compounds, that are useful in treating psychiatric disorders and neurodegenerative diseases, disorders and conditions.

Further studies led to α-ketoamide analogs of FK506 devoid of immunosuppressive activity.

WO 2015/110271 A1 describes diazabicyclo[4.3.1 ]decane derivatives (I), pharmaceutically acceptable salts of these compounds and pharmaceutical compositions containing at least one of these compounds together with pharmaceutically acceptable carrier, excipient and/or diluents. Said diazabicyclo[4.3.1]decane derivatives are specific inhibitors of the FK506 binding proteins (FKBP's) and can be used for prophylaxis and/or treatment of psychiatric disorders and neurodegenerative diseases, disorders and conditions.

EP 2 899 192 A1 describes also diazabicyclo[4.3.1]decane derivatives of formula (I), pharmaceutically acceptable salts of these compounds and pharmaceutical compositions containing at least one of these compounds together with pharmaceutically acceptable carrier, excipient and/or diluents.

EP 2 690 102 A1 describes also bicyclic aza-amides derivatives and stereoisomeric forms, solvates, hydrates and/or pharmaceutically acceptable salts of these compounds as well as pharmaceutical compositions containing at least one of these bicyclic aza-amides derivatives together with pharmaceutically acceptable carrier, excipient and/or diluents.

Therefore, it is the object of the present invention to provide compounds and/or pharmaceutically acceptable salts thereof, which inhibit FKBPs or MIPs more effectively based on significantly increased affinity to FKBPs.

Another aspect of the invention is to provide compounds and/or pharmaceutically acceptable salts thereof which can be used as pharmaceutically active agents, especially for the use in the treatment of psychiatric, metabolic, infective, neurological and hematologial disorders as well as pain diseases and cancers, as well as compositions comprising at least one of those compounds and/or pharmaceutically acceptable salts thereof as pharmaceutically active ingredients.

Also described are methods for preparing said compounds.

The object of the present invention is solved by the teaching of the independent claims. Further advantageous features, aspects and details of the invention are evident from the dependent claims, the description, and the examples of the present application.

### Description of the invention

The present invention relates to alpha-methyl substituted diazabicyclo-[4.3.1]-decane derivatives and stereoisomeric forms, solvates, hydrates and/or pharmaceutically acceptable salts of these compounds as well as pharmaceutical compositions containing at least one of these bicyclic aza-amides derivatives together with pharmaceutically acceptable carrier, excipient and/or diluents. Especially, it has been surprisingly found that a methyl group in the 1 position relative to the anchor point in the R^{A} position of the diazabicyclo-[4.3.1]-decalin derivatives leads to at least 2.5-fold, in some embodiments 5-fold, in other embodiments 10-fold, and up to 20-fold increase in the binding affinity for all FKBPs tested to date. This was demonstrated by FP-assays with an excellent accordance of the different data sets.

Thereby is the S-configuration of the methyl-group essential, a methyl group with an R-configuration worsened the affinity (about 10-fold diminished as compared to the non-methyl derivatives and up to 100-fold diminished as compared to the (S)-configuration). A hydrogen bond acceptor in the 3 position relative to the anchor point in the R^{A} position of the diazabicyclo-[4.3.1]-decalin is also essential for the increase in affinity, while the increase in affinity is independent of the residues R^{B} and R^{C}.

The molecular structure of the inventive molecule can be generally depicted as follows:

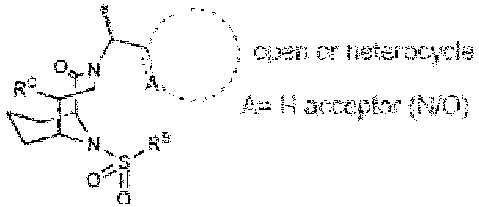

Said alpha-methyl substituted-diazabicyclo-[4.3.1]-decane derivatives have been identified as specific and potent inhibitors of the FK506 binding proteins (FKBP's), especially to FKBP12, FKBP12.6, FKBP51 and FKBP52 as well as bacterial MIP such LpMIP, CtMIP, CpMI, BpMIP, TCMIP, and are useful for the treatment of psychiatric disorders (such as depression or post-traumatic stress disorder), metabolic disorders (such as obesity or diabetes), infective disorders (such as Legionnaire's disease or Chagas diseases), neurological disorders (such as Alzheimer's diseases or Parkinson's diseases) and haematological disorders (such as hereditary haemorrhagic telangiectasia or pulmonary arterial hypertension) as well as pain diseases (such as chronic neuropathic pain or fibromyalgia) and cancers (such as prostate cancer, malignant melanoma or glioblastoma).

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalyzed preferably by bases or acids or other suitable compounds.

In regard to diazabicyclic compounds as FKBP12, FKBP12.6, FKBP51, FKBP52, or MIP ligands which have (S)-alpha-methyl-substituted 3,10-diazabicyclo-[4.3.1]-decan-2- as back bone structure, the compounds of the present invention are characterized by better affinity as compared to the non-methylated analog. These alpha-methyl group is useful for fixing the conformation of FKBP12, FKBP12.6, FKBP51, FKBP52 and MIP ligands, specifically the positioning and orientation of the hydrogen bond acceptor in the 3-position. Thus, these alpha-methylated bicyclic compounds have usually better affinity to FKBP12, FKBP12.6, FKBP51, FKBP52 and MIP proteins than the corresponding compounds lacking the alpha-methyl group.

FKBP inhibitors or FKBP ligands as used herein are defined as compounds that
(i) inhibit the peptidyl-prolyl isomerase activity of FKBPs or MIPs (PPlase inhibitors, also referred to as rotamase inhibitors) or
(ii) displace FK506 or FK506 analogs from the PPlase active site of FKBPs or MIPs
(iii) bind to the FK506-binding domain of FKBPs or MIPs as determined biophysically by isothermal calorimetry, surface plasmon resonance, tryptophan quenching, NMR or x-ray crystallography.

The molecules of the present invention may be represented as:
1. A compound of the general formula (I): 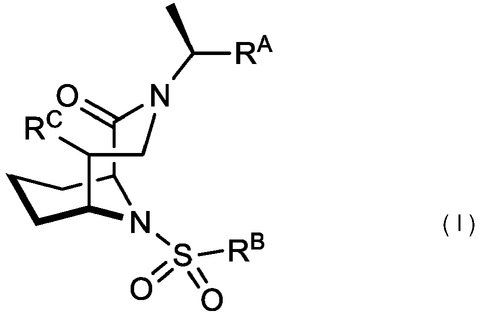
   wherein the methyl group in the 1 position relative to the anchor point in the RA position is in S-configuration;
   wherein **R^{A}** represents: 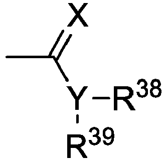
   wherein X, Y represent independently of each other O, N, S;
   or wherein **R^{A}** represents:
      -CH₂OR¹⁶,
      -CH₂NR³⁸R³⁹, 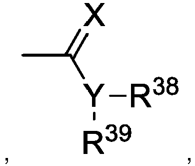 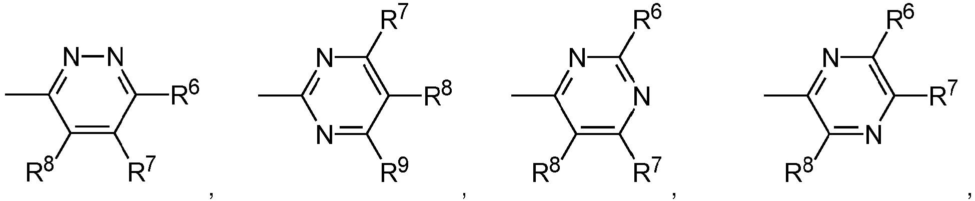 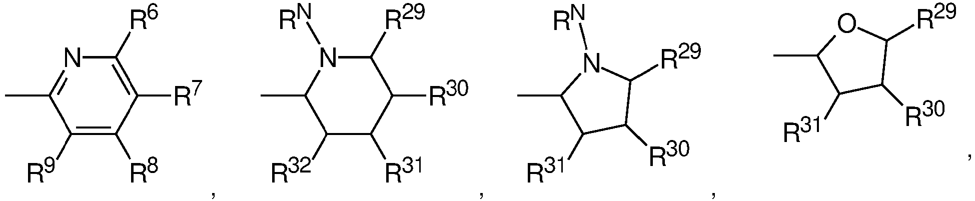 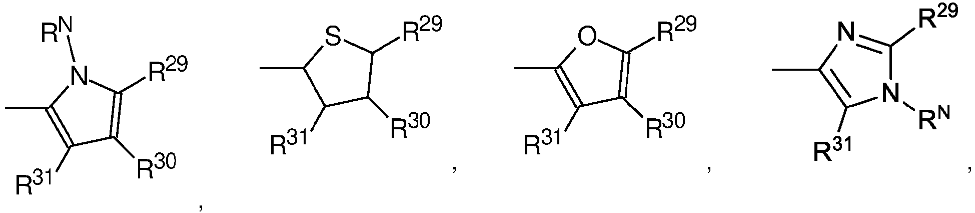 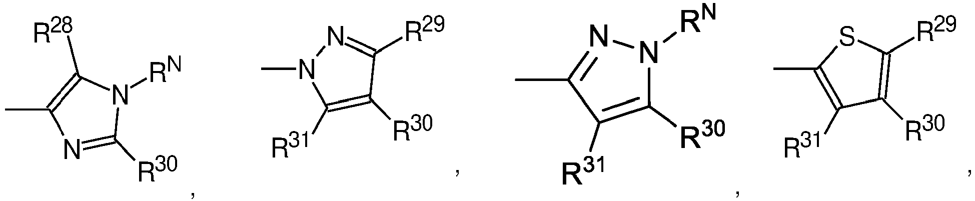 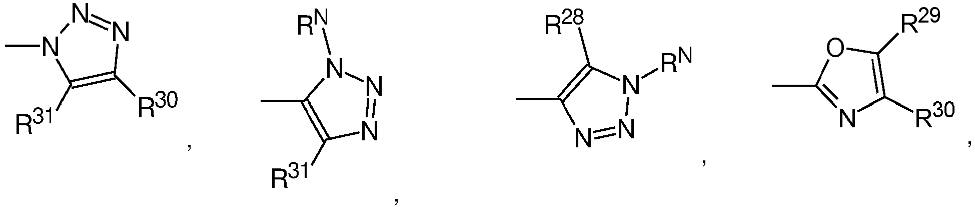 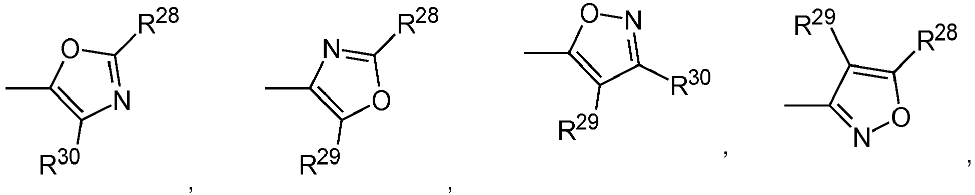 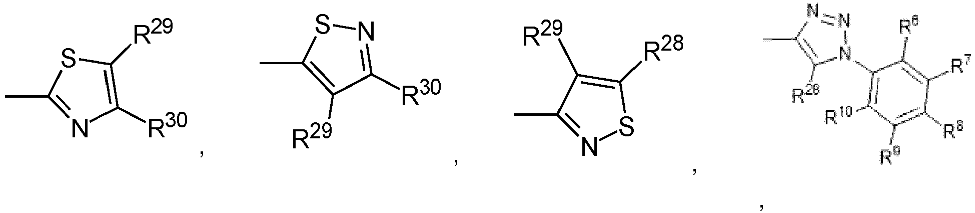 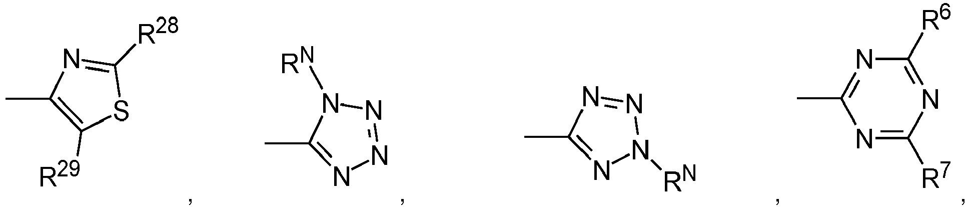 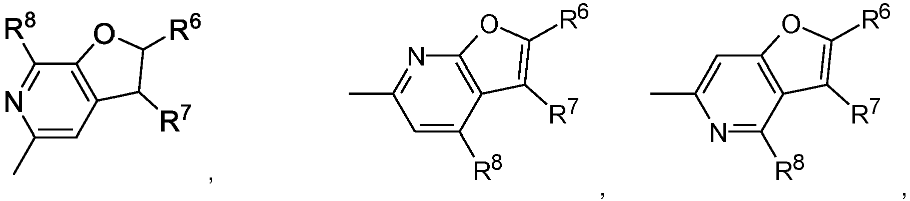 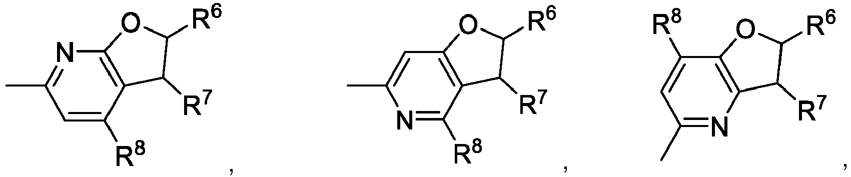 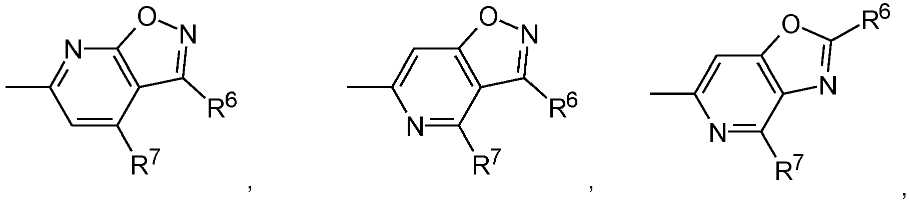 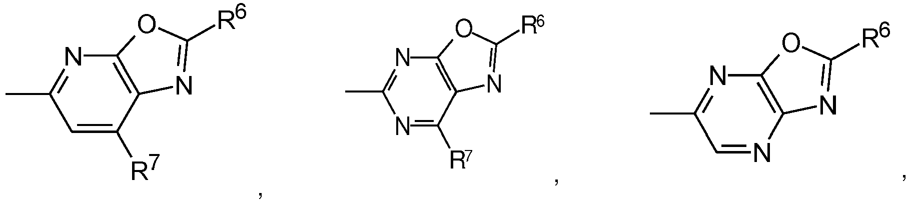 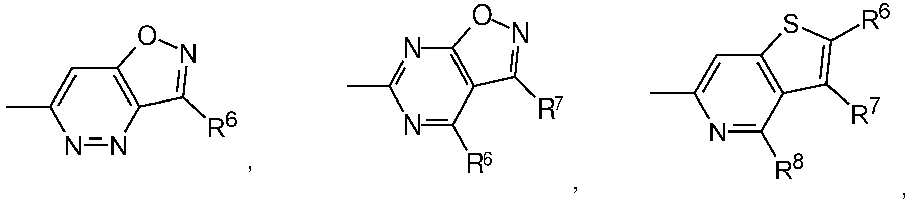 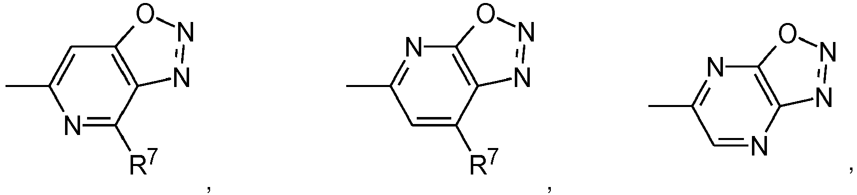 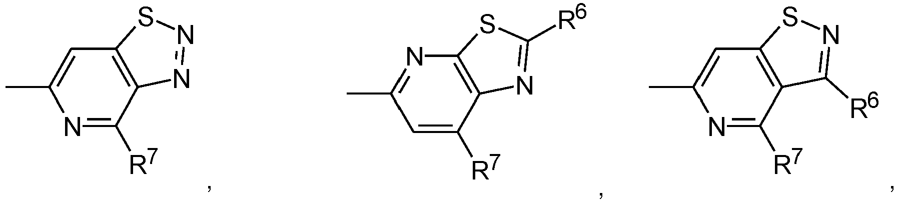 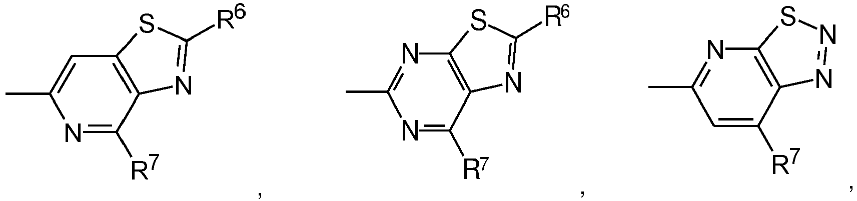 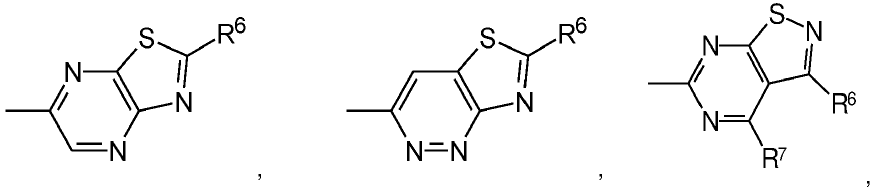 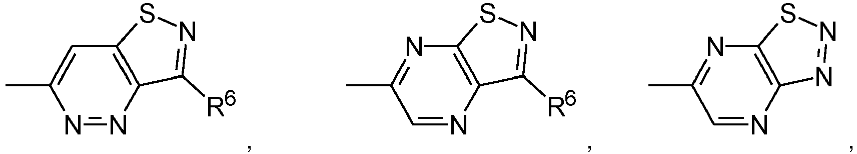 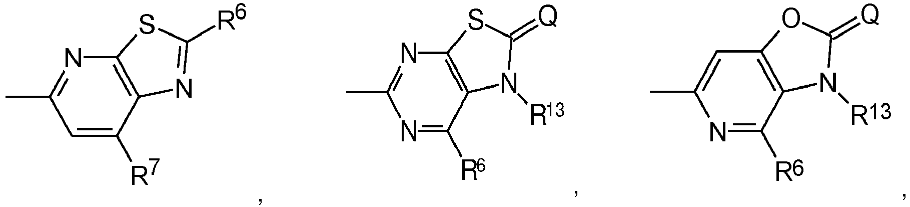 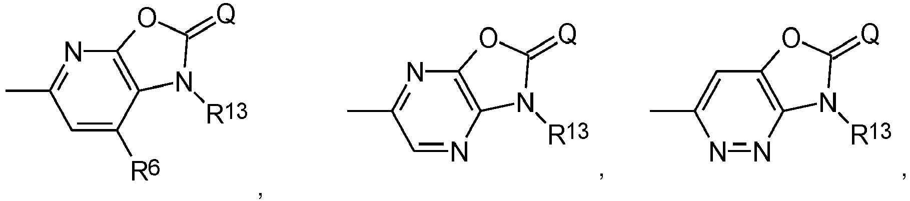 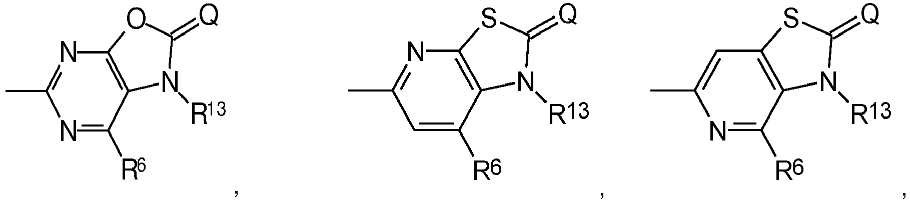 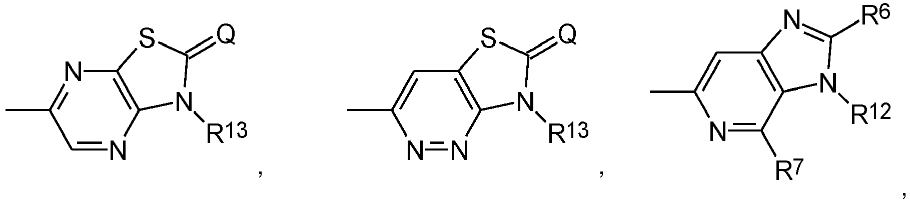 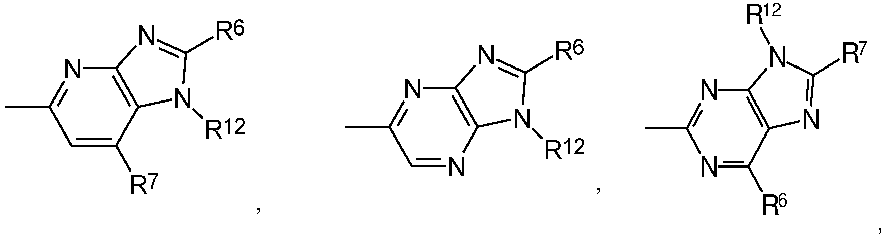 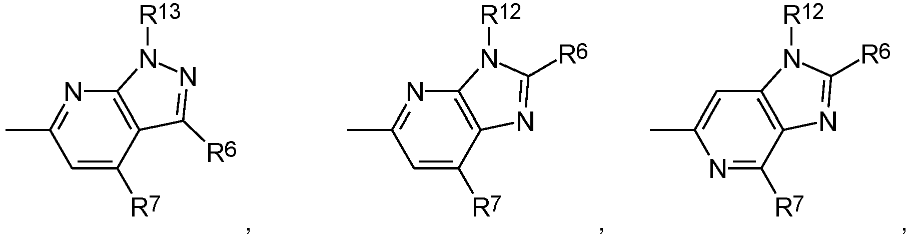 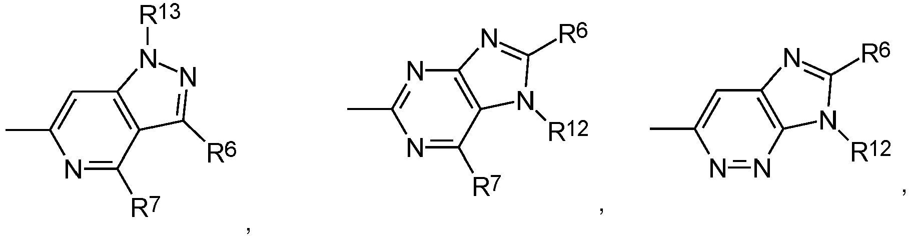 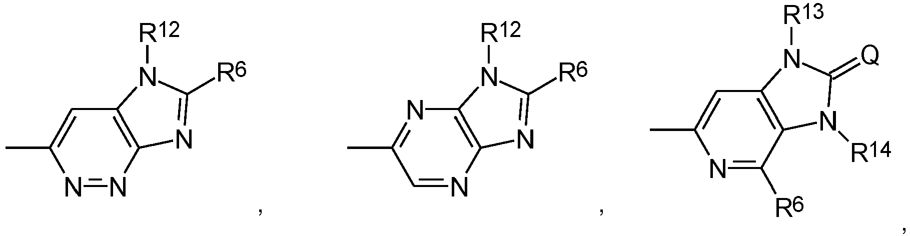 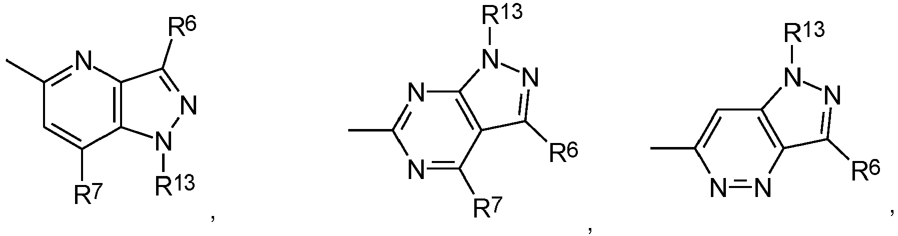 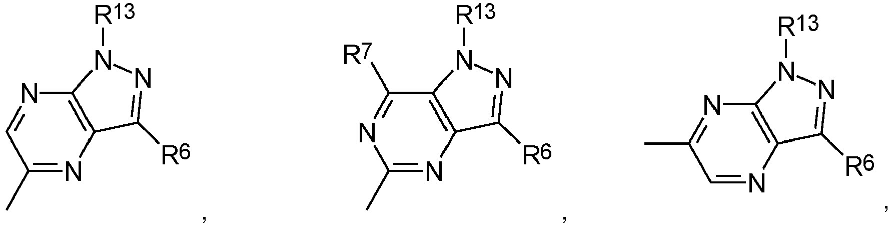 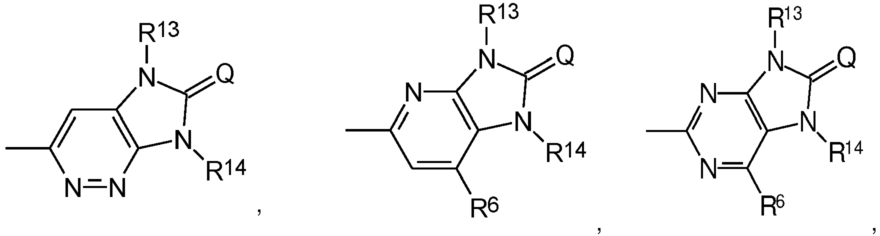 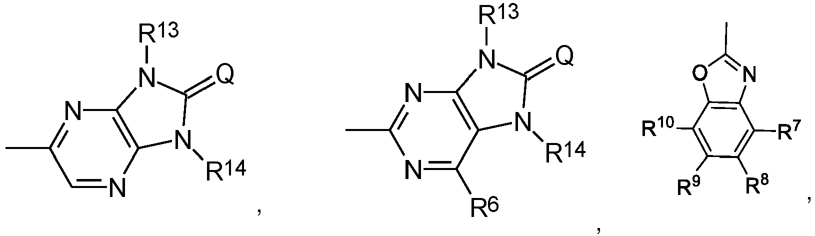 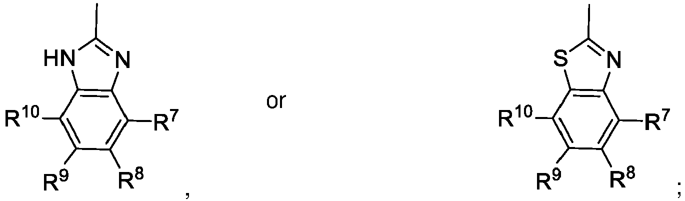
   wherein **X, Y** represent independently of each other O, N, S;
   wherein **Q** represents =O, =S, or =N-R¹²;
   wherein **R^{N}** represents -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COPh, -COCN, -COOCH₃, -COOC₂H₅, -COOC₃H₇, -COO-cyclo-C₃H₅, -COOCH(CH₃)₂, -COOC(CH₃)₃, -COCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SO₂CH₃, -SO₂C₂H₅, -SO₂CH₂Ph, -SO₂C₃H₇, -SO₂-cyclo-C₃H₅, -SO₂CH(CH₃)₂, -SO₂C(CH₃)₃, -SO₂Ph, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH₂-C≡C-C₂H₅, -CH=CH-CH=C(CH₃)₂, -CH₂-CH(CH₃)-CH₂-C≡CH, -C₂H₄-C≡C-CH₃, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, - C≡C-CH(CH₃)-C≡CH, -CH(CH₃)-C≡C-C≡CH, -CH(C≡CH)-CH₂-C≡CH, -CH(C≡CH)-C≡C-CH₃;
   **R^{B}** represents 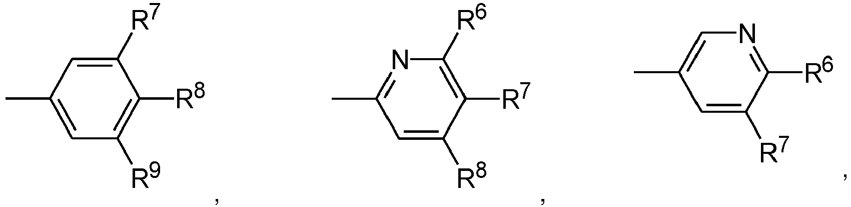 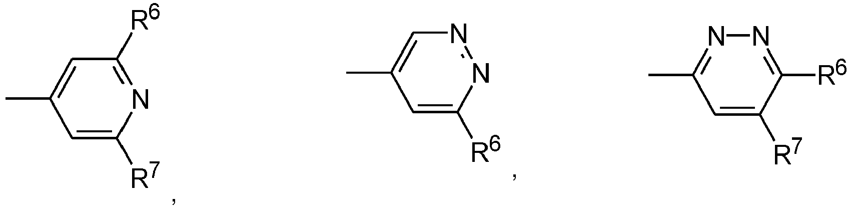 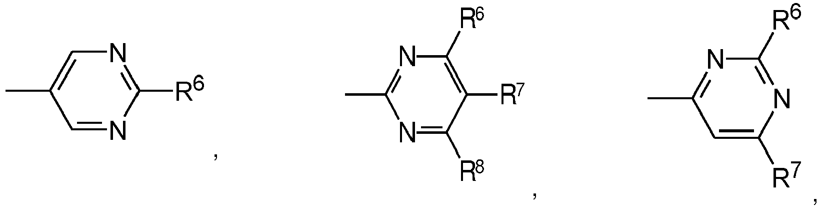 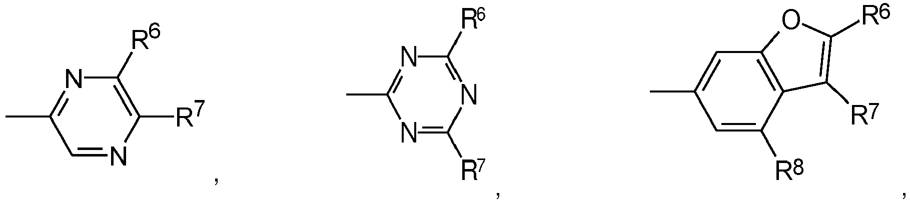 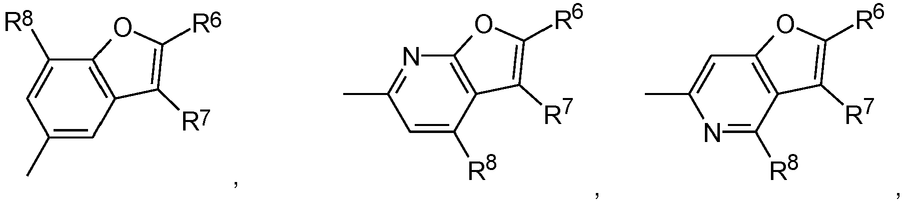 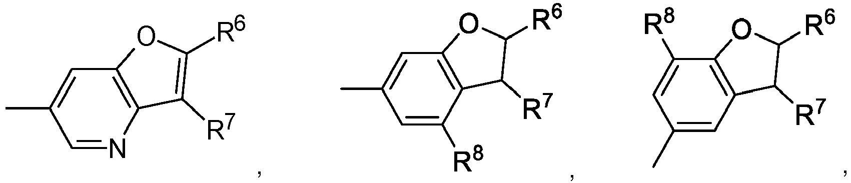 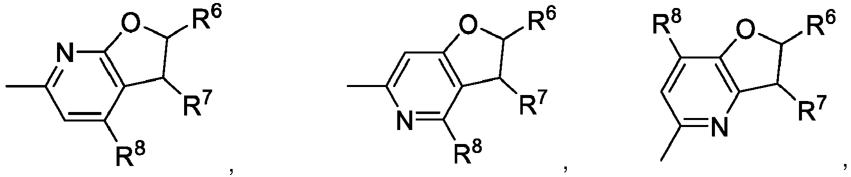 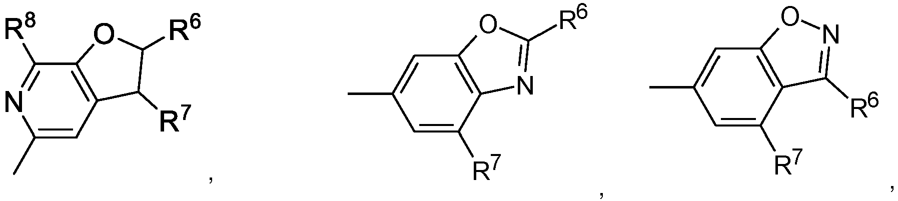 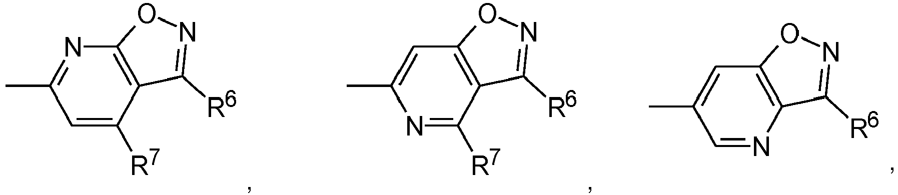 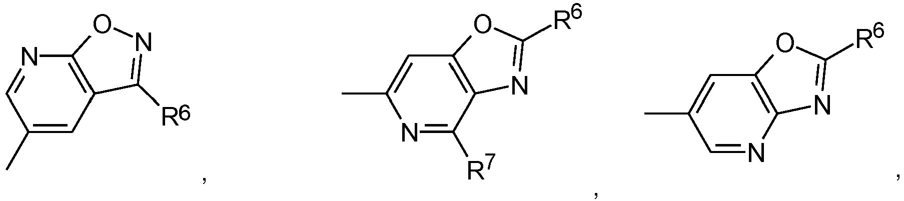 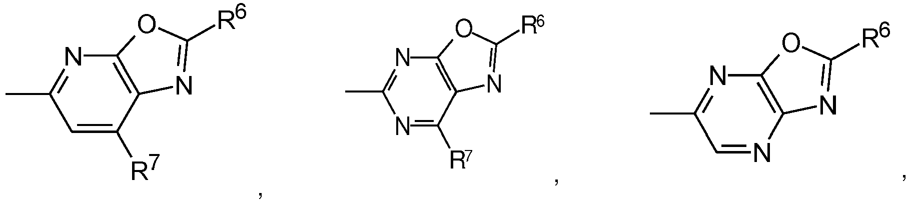 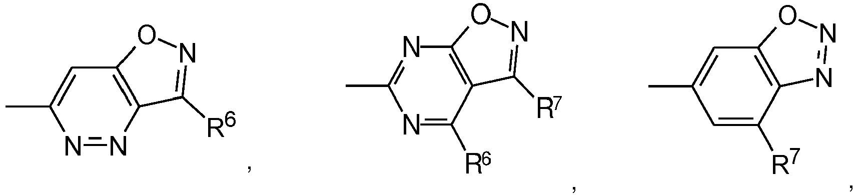 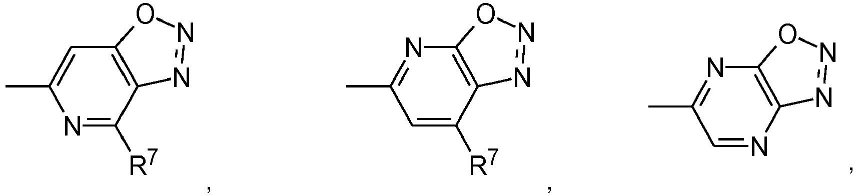 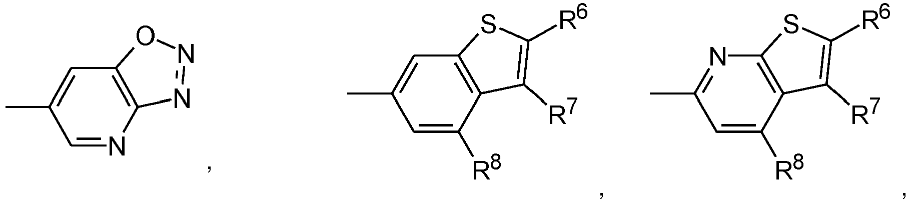 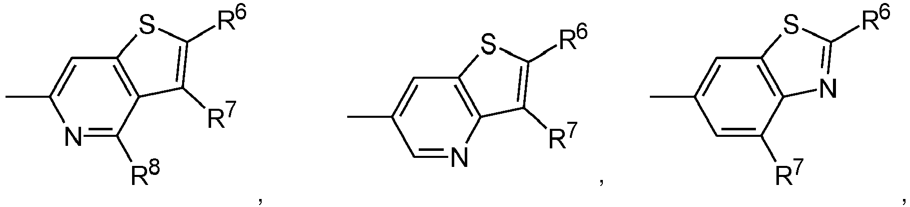 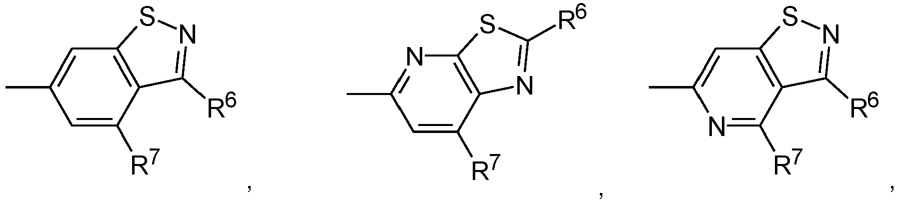 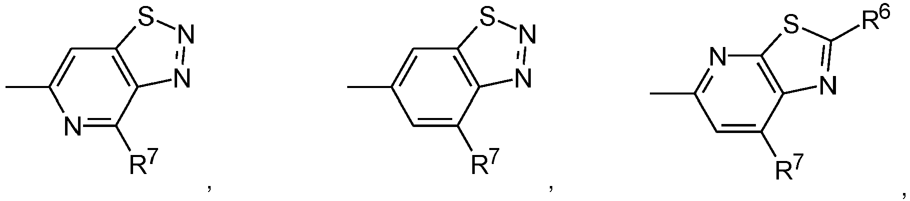 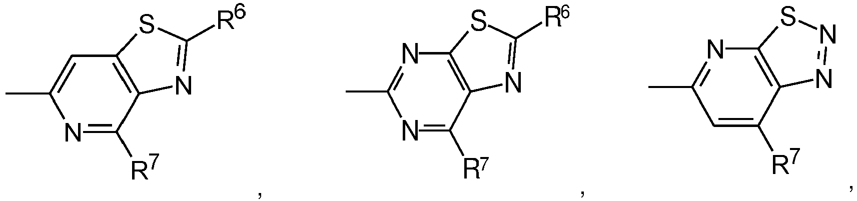 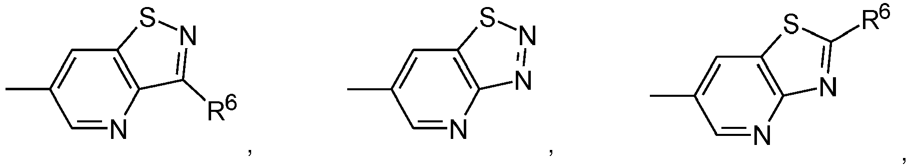 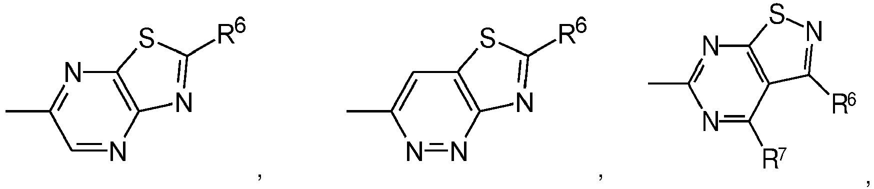 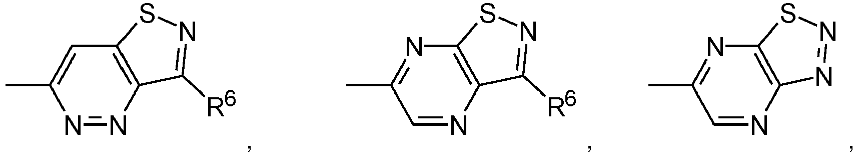 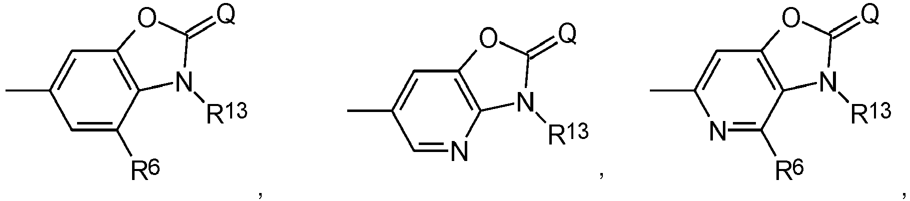 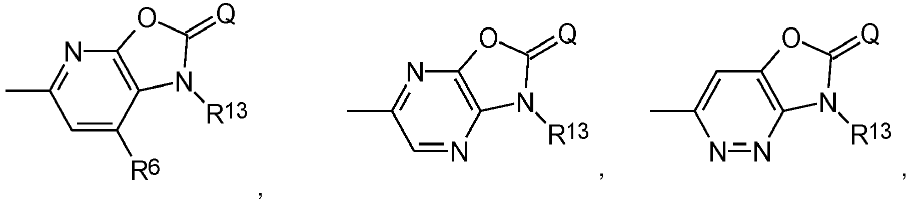 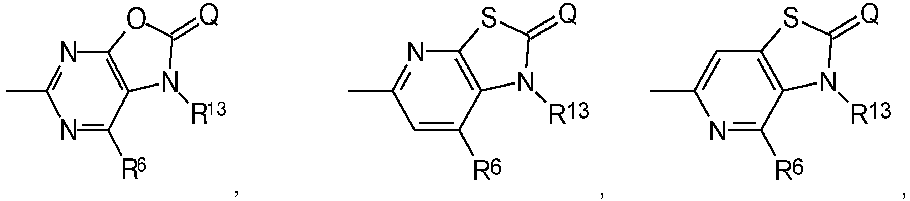 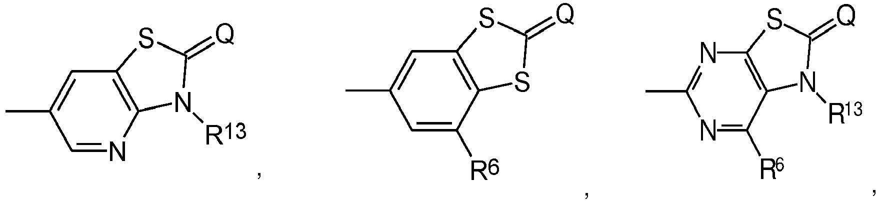 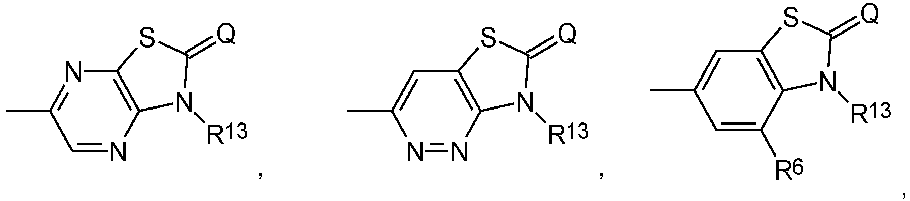 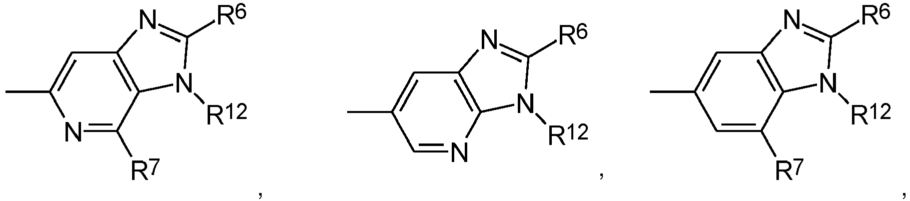 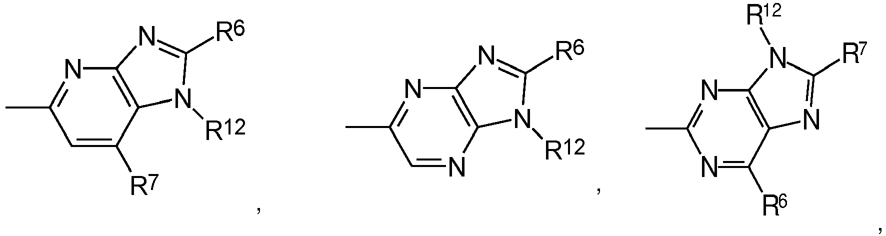 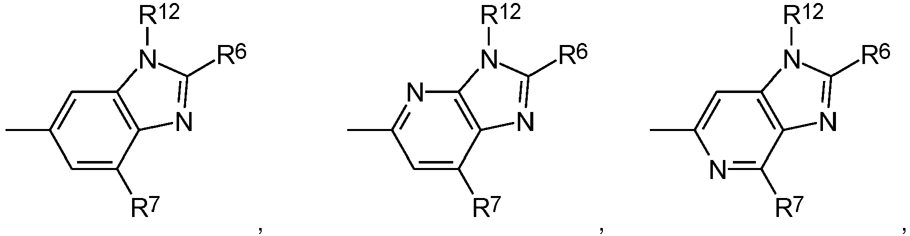 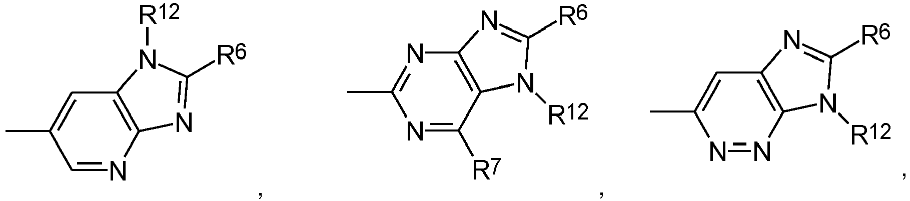 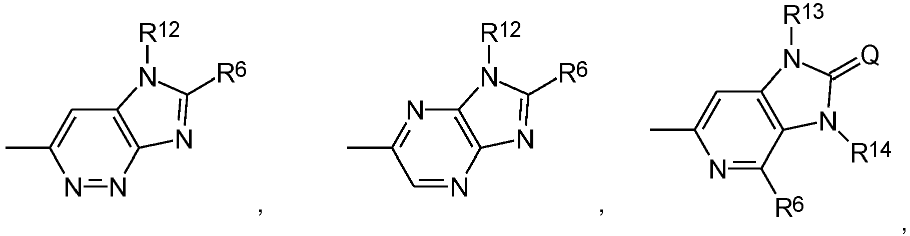 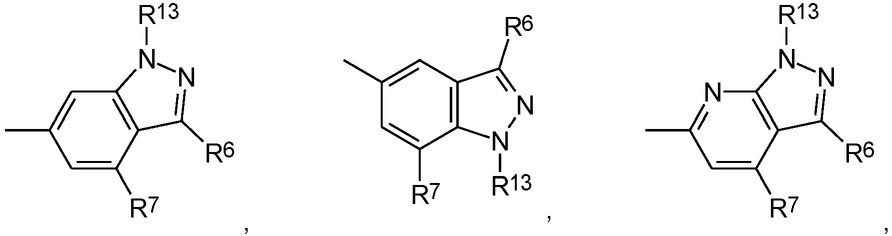 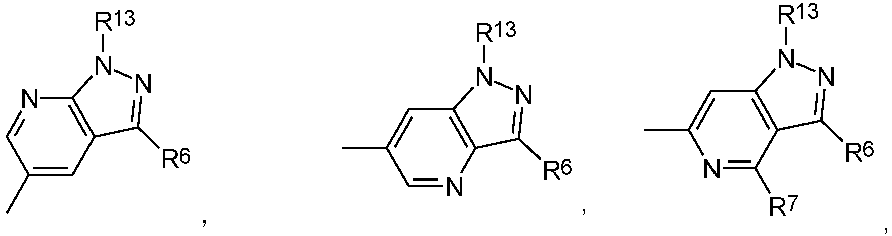 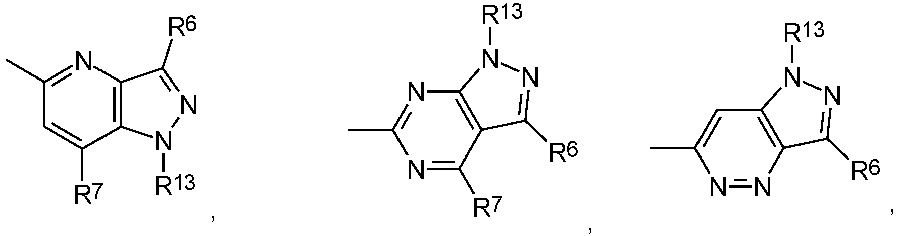 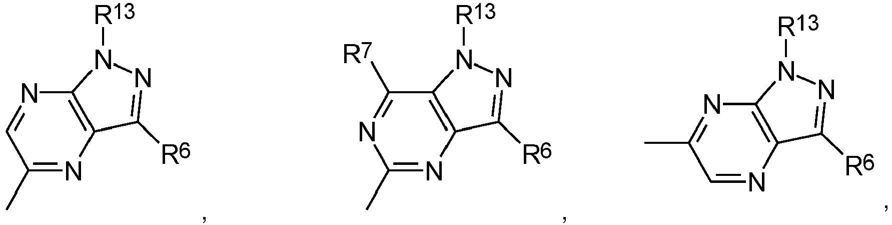 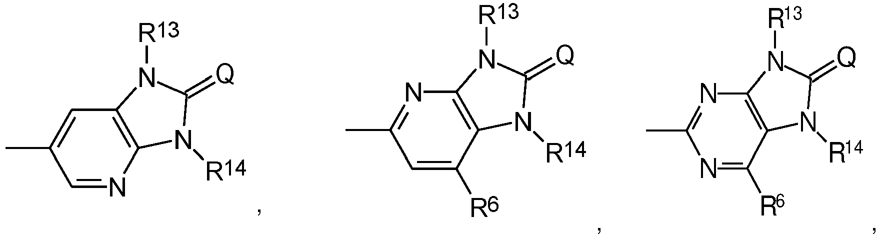 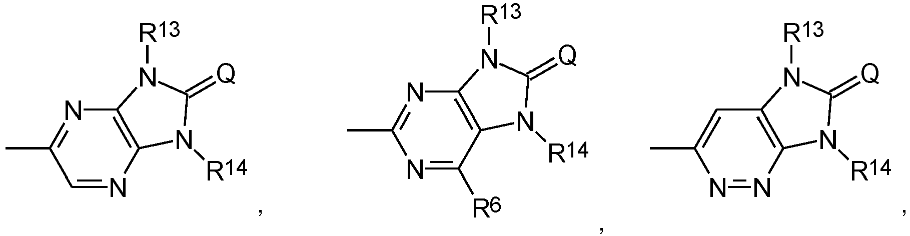 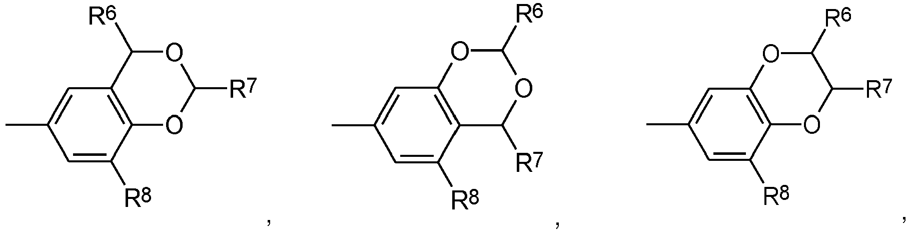 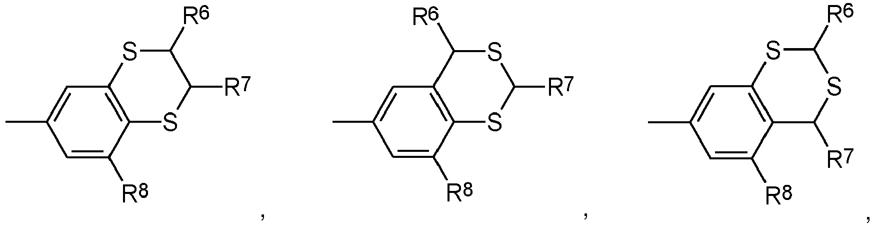 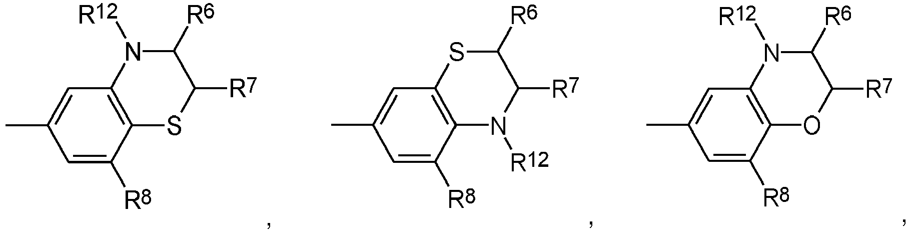 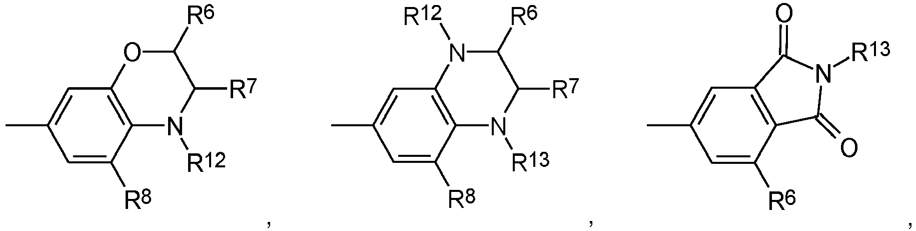 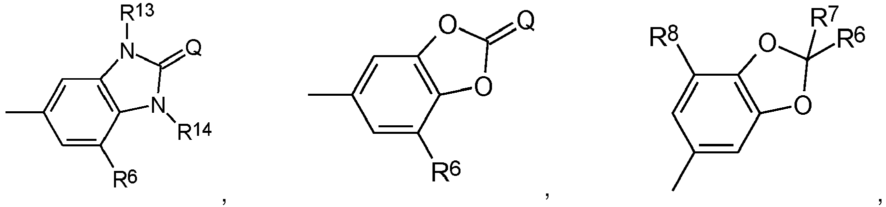 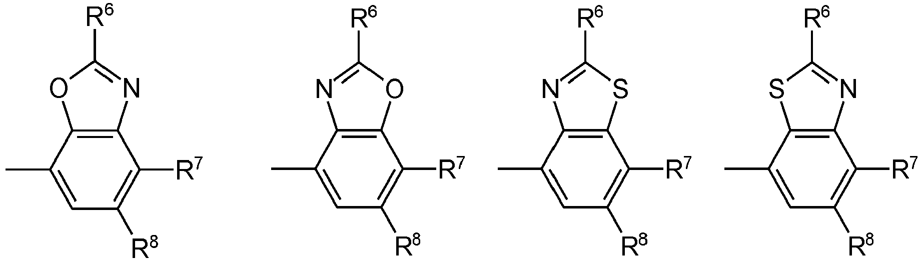 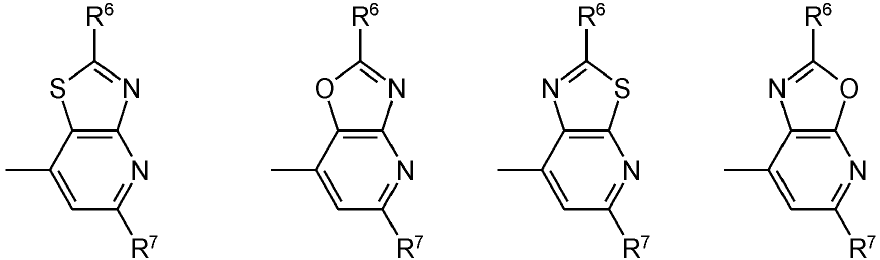 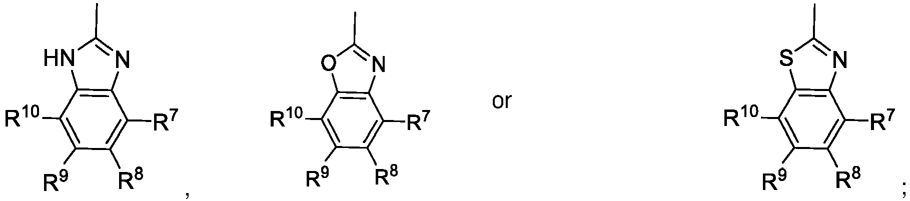
   **Q** represents =O, =S, or =N-R¹²;
   **R^{C}** represents -H, -OH, -CH₂-OH, -CHO, -CH₂CHO, -CH₂CH₂CHO, -C₂H₄-OH, - C₃H₆-OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -CH(OAc)-CH₂OH, -CH(OAc)-CH₂OAc, -CH(OH)-CH₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂H₅)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)CH₂OAc, -CH(NHCH₃)CH₂OAc, -CH(NHC₂H₅)CH₂OAc, -CH(N(CH₃)₂)CH₂OAc, -CH(N(C₂H₅)₂)CH₂OAc, -CH₂-CH(NHAc)CH₂OH, -CH₂-CH(NHAc)CH₂OCH₃, -CH₂-CH(NHA_{C})CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)CH₂OAc, -CH₂-CH(NHCH₃)CH₂OAc, -CH₂-CH(NHC₂H₅)CH₂OAc, -CH₂-CH(N(CH₃)₂)CH₂OAc, -CH₂-CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -NHSO₂CH₃, -NHSO₂CF₃, -NHSO₂CH₂CF₃, -CH₂-NHSO₂CH₃, -CH₂-NHSO₂CF₃, -CH₂-NHSO₂CH₂CF₃, -C₂H₄-NHSO₂CH₃, -C₂H₄-NHSO₂CF₃, -C₂H₄-NHSO₂CH₂CF₃, -CONH₂, -CONHCH₃, -CONHC₂H₅, -CONHC₃H₇, -NH(C₂H₅), -N(C₂H₅)₂, -CH₂-NH(C₂H₅), - CH₂-N(C₂H₅)₂, -C₂H₄-NH(C₂H₅), -C₂H₄-N(C₂H₅)₂, -NO₂, -CH₂-NO₂, -C₂H₄-NO₂, -CH(OH)-NO₂, -CH(NO₂)-OH, -CO₂H, -CH₂-CO₂H, -C₂H₄-CO₂H, -CH=CH-CO₂H, -CO₂CH₃, -CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -O-Si(CH₃)₃, -O-Si(C₂H₅)₃, -CO-CHO, -CO-CO-CH₃, -C(OH)-CO-CH₃, -CO-C(OH)-CH₃, -CO-CH₂-CO-CH₃, -C(OH)-CH₂-CO-CH₃, -CO-CH₂-C(OH)-CH₃, -C(OH)-CH₂-C(OH)-CH₃, -F, -Cl, -Br, -CH₂-F, -CHF₂, -CF₃, -C₂H₄-F, -CH₂-CF₃, -CF₂-CF₃, -O-CHF₂, -O-CF₃, -O-CH₂-CF₃, -O-C₂F₅, -CH₃, -CH₂CH₃, -C₃CH₇, -CH(CH₃)₂, -CH=CH₂, -C≡CH, -CH₂-CH=CH₂, or -CH₂-C≡CH;
   **R¹ - R¹⁰** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-CeH₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, 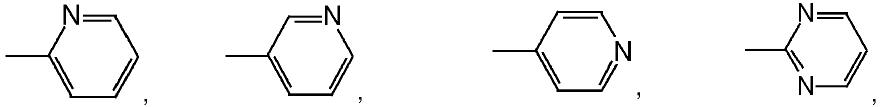 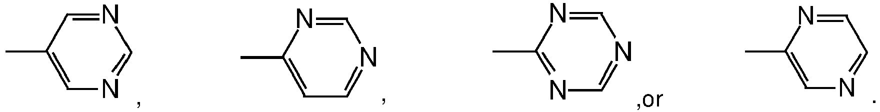
   **R¹⁵** represents **-R²⁰**, -CN, -CH₂-CN, -CH₂-OR¹⁷, -CH₂-CH₂-OR¹⁷, -CH₂-NR¹⁷R¹⁸, -CH₂-NR¹⁷COR¹⁹, -CH₂-CH₂-NR¹⁷R¹⁸, -CH₂-CH₂-NR¹⁷COR¹⁹, -CO₂R¹⁷, -CO-NR¹⁷R¹⁸, -CH₂-CO₂R¹⁷, or -CH₂-CO-NR¹⁷R¹⁸;
   **R¹⁶**, **R³⁸**, **R³⁹** represent independently of each other **-R²¹**, a lone pair, -H, -CH₃, -C₂H₅, - C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH₂OH, -CH₂-SH, -CH(OH)CH₃, -C₂H₄OH, -C₃H₆OH, - C₄H₈OH, -CH(CH₃)-C₂H₄OH, -C₅H₁₀OH, -CH₂-S-CH₃, -CH₂-CH₂-S-CH₃, -C₃H₆-S-CH₃, -CH₂OCH₃, -C₂H₄OCH₃, -C₃H₆OCH₃, -C₄H₈OCH₃, -CH(CH₃)-C₂H₄OCH₃, -C₅H₁₀OCH₃, -CH₂NH₂, -C₂H₄NH₂, -C₃H₆NH₂, -C₄H₈NH₂, -CH(CH₃)-C₂H₄NH₂, -C₅H₁₀NH₂, -CH₂-CH₂-CH₂-NH-C(NH)NH₂, -CH₂-CO₂H, -CH₂-CONH₂, -CH₂-CH₂-CO₂H, -CH₂-CH₂-CONH₂, -CH₂-CO₂CH₃, -CH₂-CONHCH₃, -CH₂-CON(CH₃)₂, -CH₂-CH₂-CO₂CH₃, -CH₂-CH₂-CONHCH₃, -CH₂-CH₂-CONH(CH₃)₂, -CH=CH-CO₂H, -CH=CH-CO₂CH₃, -CH=CH-CONHCH₃, -CH=CH-CONHC₂H₅, -CH=CH-CON(CH₃)₂, -CH=CH-CON(C₂H₅)₂, -CH₂-CH=CH-CO₂H, -CH₂-CH=CH-CO₂CH₃, -CH₂-CH=CH-CONHCH₃, -CH₂-CH=CH-CON(CH₃)₂, -CH₂-CH=CH-CONHC₂H₅, -CH₂-CH=CH-CON(C₂H₅)₂, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, - CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, - C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, - CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-Ph, 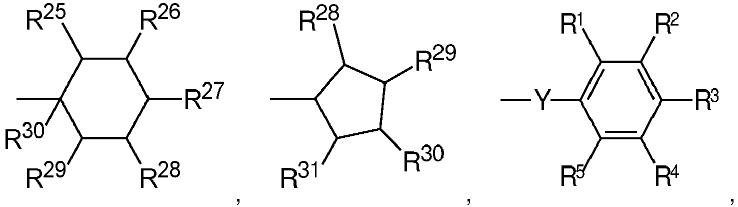 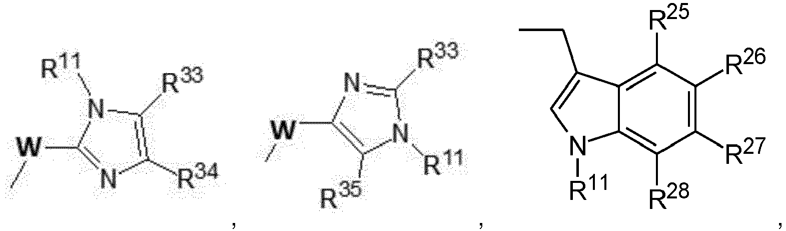 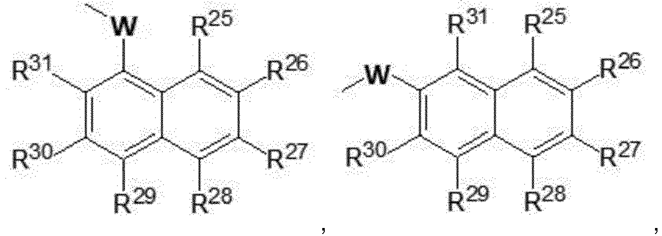 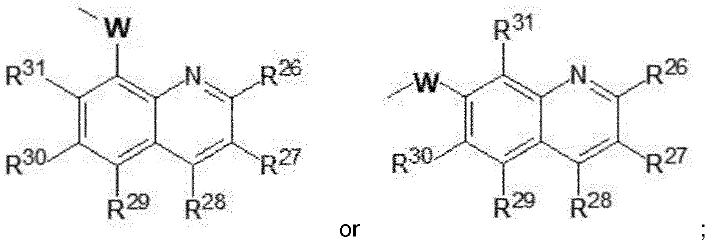
   wherein W represents O, N-R¹², S;
   **R¹¹** - **R¹⁴** and **R¹⁷- R²¹** represent independently of each other -H, -CH₂F, -CHF₂, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂l, -cyclo-C₃H₅, -cyclo-C₄H₇, -cyclo-C₅H₉, -cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H_{5,} -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, or -CH₂-CH(C≡CH)₂;
   **R²²- R³⁷** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇₅ -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 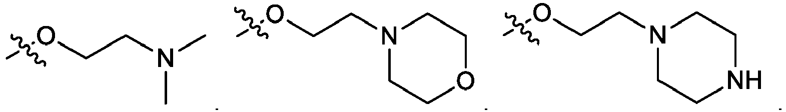 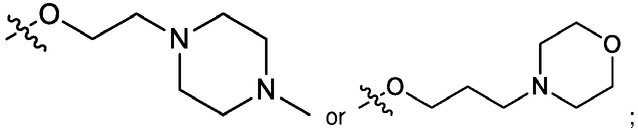
   and enantiomers, stereoisomeric forms, mixtures of enantiomers, anomers, deoxy-forms, diastereomers, mixtures of diastereomers, tautomers, hydrates, solvates and racemates of the above mentioned compounds and pharmaceutically acceptable salts.

The expression "tautomer" is defined as an organic compound that is interconvertible by a chemical reaction called tautomerization. Tautomerization can be catalyzed preferably by bases or acids or other suitable compounds.

In yet another preferred embodiment of the present invention, the compound according to the general formula (I) is selected from the group comprising or consisting of:

Preferred substituents for **R^{A}** are 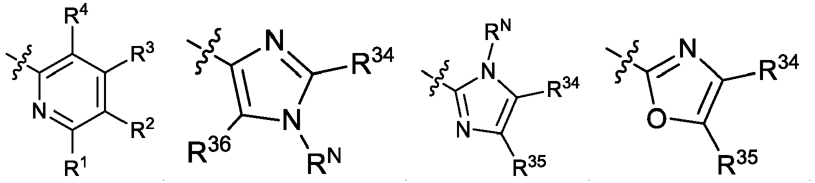 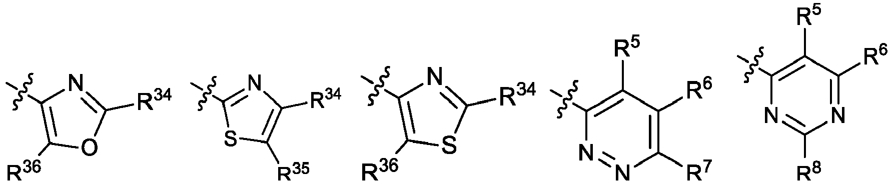 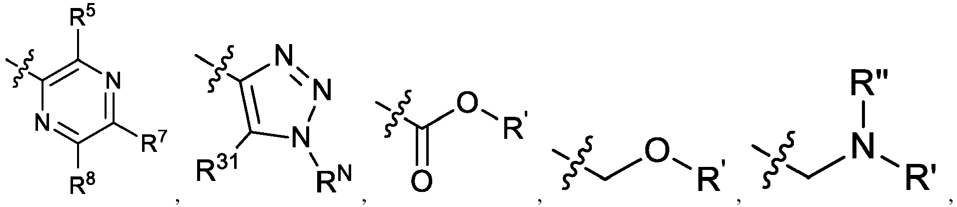 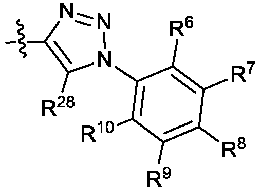 wherein R¹ - R³⁶, R^{N} have the meanings as defined in the general formula (I) and R' and R" represent independently of each other -H, -CH₃, -C₂H₅, -CH(CH₃)₂, -CF₃, -COCH₃.

More preferred are compounds of the formula (I) having one of the following substituents **R^{A}:**

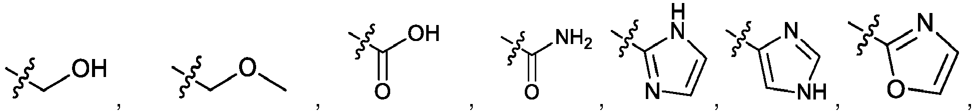

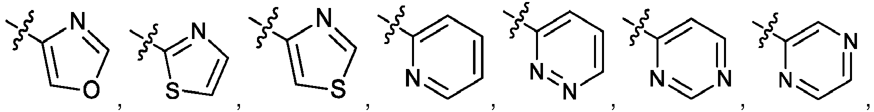

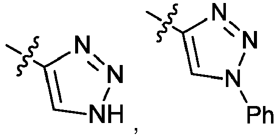

Especially preferred **R^{A}** is selected from the following group:
-COOH, -COOCH₃, -COOC₂H₅, -COOC₃H₇, -CH₂NH₂, -CH₂NHCH₃, -CH₂NHC₂H₅, -CH₂NHC₃H₇, -CH₂N(CH₃)₂, -CH₂N(C₂H₅)₂, -CH₂N(C₃H₇)₂, -CONH₂, -CONHCH₃, - CONHC₂H₅, -CONHC₃H₇, -CON(CH₃)₂, -CON(C₂H₅)₂, -CON(C₃H₇)₂;
and in some embodiments -COOH, -COOC₂H₅, -CH₂NHCH₃, -CONH₂, 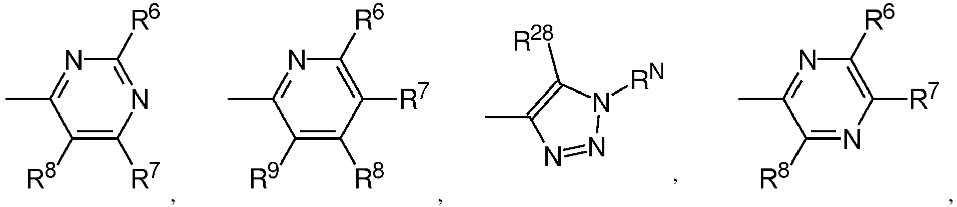 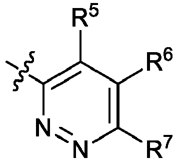 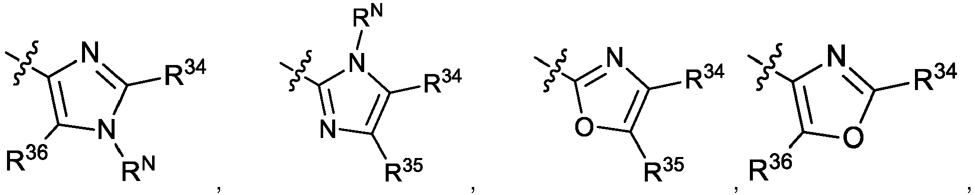 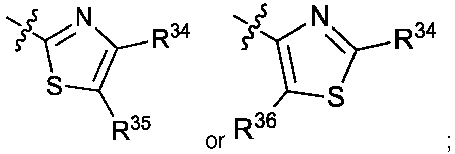
**R⁶** - **R⁹** and **R²⁸** - **R³⁶** represent independently of each other -H or -CH₃ and more preferably - H;
**R^{N}** represents -H, -COCH₃, -COC₂H₅, -COPh, -COCH₂Ph, -SO₂Ph, -SO₂CH₂Ph, -CH₃, -C₂H₅, -C₃H₇, -CH(CH₃)₂, -Ph, or -CH₂-Ph, and more preferably - H, -COPh, -SO₂Ph, -Ph, or -CH₂-Ph.

In an alternative definition preferred are compounds of the formula (I) having a substituent **R^{A}** with a molecular weight of <200 g/mol, more preferable <100 g/mol, and most preferable <50 g/mol.

Preferred substituents for **R^{B}** are: 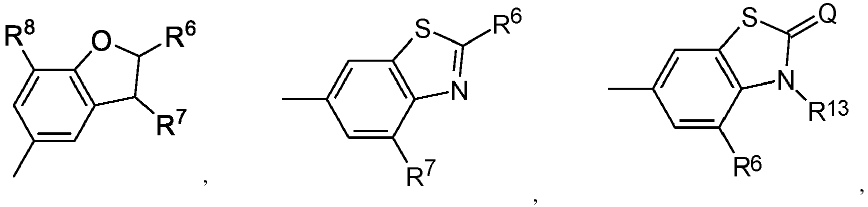 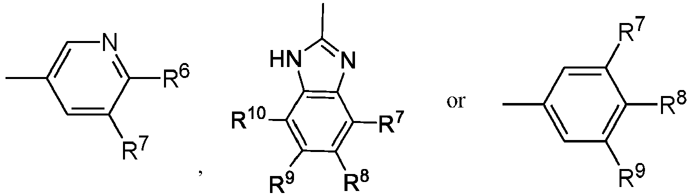 wherein **R⁶ - R¹⁰, R¹³** and **Q** have the meanings as defined in the general formula (I);

In some embodiments **R⁶ - R¹⁰, R¹³** and **Q** represent:
Q represents =O;
**R⁶ - R⁹** represent independently of each other -H, -F, -Cl, -Br, -I, -OH, -OCH₃, -OC₂H₅, -OC₃H₇, -CN, -CONH₂, -NHCOCH₃, -NHCOC₂H₅, -NHCOC₃H₇, -COOH, -COOCH₃, -COOC₂H₅, -COOC₃H₇, -Ph, 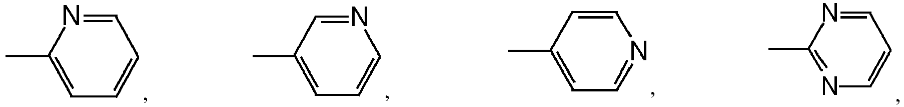 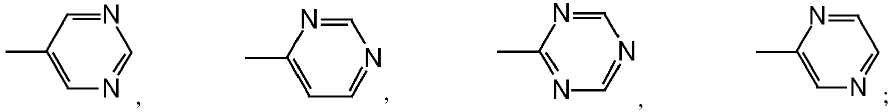
and in some embodiments -F, -Cl, -Br, -OH, -OCH₃, -CN, -CONH₂, -NHCOCH₃, -COOCH₃, -Ph, or 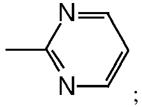
**R¹⁰** represents -H;
**R¹³** represents -H, -CH₃, or -C₂H₅, and more preferably -H;
and in some embodiments one of **R⁷ - R⁹** is different from hydrogen.

In other embodiments the compounds of the formula (I) are having one of the following substituents **R^{B}** : 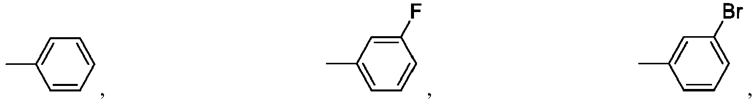 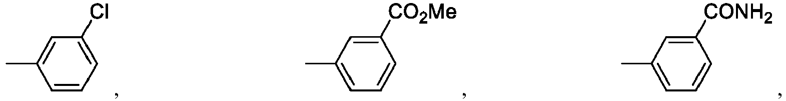 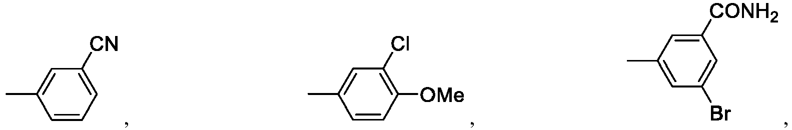 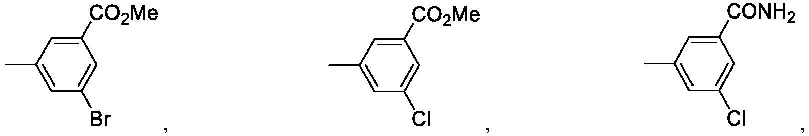 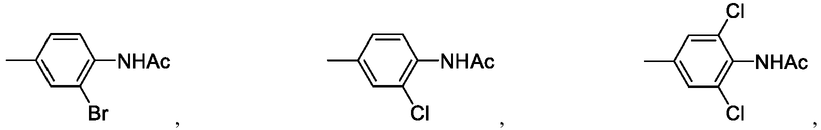 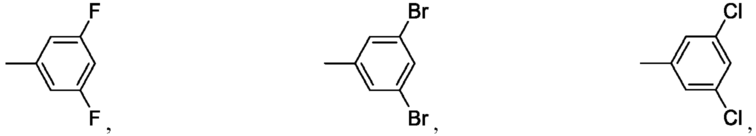 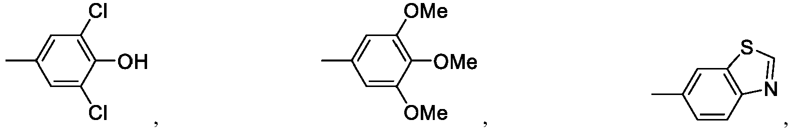 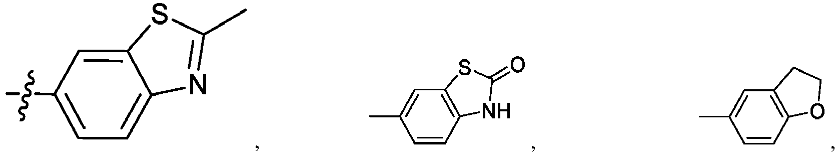 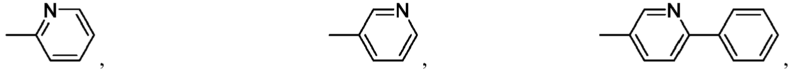 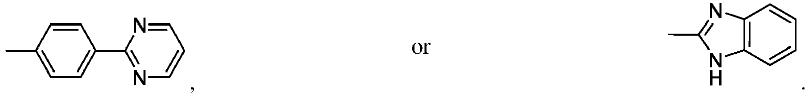 In some embodiments substituents for **R¹** - **R¹⁰** are: -H, -OH, -OCH₃, -OC₂H₅, -OC₃H₇, -OCH(CH₃)₂, -OCPh₃, -CH₂-OCH₃, -CH₂-OH, -OC₃H₇, -OC(CH₃)₃, -OCH₂-COOH, - CO₂Me, -CO₂Et, -CONH₂, -NHCOCH₃, -NHSO₂CH₃, -CH₃, -CH₂-OH, -C₂H₅, -C₃H₇, - CH(CH₃)₂, -CN, -F, -Cl, -Br, -I,

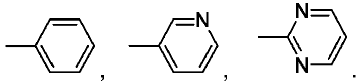

In an alternative definition preferred are compounds of the formula (I) having a substituent **R^{B}** with a molecular weight of <300 g/mol, more preferable <200 g/mol, and most prefereable <130 g/mol.

In some embodiments the compounds of the formula (I) are having one of the following substituents
**R^{C} :** -CH₂-OH, -CHO, -CH₂CHO, -CH₂CH₂CHO, -C₂H₄-OH, -C₃H₆-OH, -OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -CH(OAc)-CH₂OH, -CH(OAc)-CH₂OAc, -CH(OH)-CH₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂H₅)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)CH₂OAc, -CH(NHCH₃)CH₂OAc, -CH(NHC₂H₅)CH₂OAc, -CH(N(CH₃)₂)CH₂OAc, -CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)CH₂OAc, -CH₂-CH(NHCH₃)CH₂OAc, -CH₂-CH(NHC₂H₅)CH₂OAc, -CH₂-CH(N(CH₃)₂)CH₂OAc, -CH₂-CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -CONH₂, -CONHCH₃, -CONHC₂H₅, -CONHC₃H₇, -NH(C₂H₅), -N(C₂H₅)₂, -CH₂-NH(C₂H₅), -CH₂-N(C₂H₅)₂, -C₂H₄-NH(C₂H₅), -C₂H₄-N(C₂H₅)₂, -CO₂H, -CH₂-CO₂H, -C₂H₄-CO₂H, -CH=CH-CO₂H, -CO₂CH₃, -CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -CH₂-F, -CHF₂, -CF₃, -C₂H₄-F, -CH₂-CF₃, -CF₂-CF₃, -O-CHF₂, -O-CF₃, -CH₃, -CH₂CH₃, -C₃CH₇, -CH(CH₃)₂, -CH=CH₂, -C≡CH, -CH₂-CH=CH₂, or -CH₂-C≡CH.

In other embodiments **R^{C}** represents: -OH, -CH₂-OH, -C₂H₄-OH, -C₃H₆-OH, -CHO, -CH₂CHO, -CH₂CH₂CHO, -O-CH₃, -O-C₂H₅, -O-C₃H₇, -O-CH(CH₃)₂, -CONH₂, -CONHCH₃, -CONHC₂H₅, -CONHC₃H₇, -CO-CH₃, -CO-C₂H₅, -CO-C₃H₇, -CO-CH(CH₃)₂, -CO₂H, -CH₂-CO₂H, -C₂H₄-CO₂H, -O-CH₂-OH, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-C₃H₇, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -NH(C₂H₅), -N(C₂H₅)₂, -CH₂-NH(C₂H₅), -CH₂-N(C₂H₅)₂, -C₂H₄-NH(C₂H₅), -C₂H₄-N(C₂H₅)₂, -CH₂-F, -CHF₂, -CF₃, -C₂H₄-F, -CH₂-CF₃, -CF₂-CF₃, -CH₃, -CH₂CH₃, -C₃CH₇, -CH(CH₃)₂, -CH=CH₂, -C≡CH, -CH₂-CH=CH₂, or -CH₂-C≡CH.

In even further embodiments **R^{C}** represents: **R^{C}** represents: -OH, -NH₂, -CH₂F, -CHF₂, - CH₂CH₃, -CH=CH₂, -CH₂OH, -CHO, -CO₂H, -CONH₂, -COCH₃, -OCH₃, -OCH₂CH₃, -OCH(CH₃)₂, -OCH₂OCH₃, -OC₂H₄OCH₃, -CH₂OCH₃, -CH₂OCH₂CH₃, -CH₂CH₂OH, -CH₂CHO, -CH₂CH₂CHO, -CH₂NH₂, -CH₂NHCH₃, -CH₂N(CH₃)₂, -CH(OH)CH₃, -C(OH)(CH₃)₂, -CH₂CH(OH)CH₃ or -CH(OH)CH₂OH.

In yet even further embodiments **R^{C}** represents: -OH, -NH₂, -CH₂F, -CHF₂, -CH₂CH₃, -CH₂OH, -CHO, -CO₂H, -CONH₂, -COCH₃, -OCH₃, -OCH₂CH₃, -OCH(CH₃)₂, -OCH₂OCH₃, -OC₂H₄OCH₃, -CH₂OCH₃, -CH₂OCH₂CH₃, -CH₂CH₂OH, -CH₂CHO, -CH₂CH₂CHO, -CH₂NH₂, -CH₂NHCH₃, -CH₂N(CH₃)₂, -CH(OH)CH₃, -C(OH)(CH₃)₂, -CH₂CH(OH)CH₃ or -CH(OH)CH₂OH.

In even further embodiments **R^{C}** residues comprise a hydroxyl group or an alkoxy group such as -CH₂OH, -CH₂CH₂OH, -CH(OH)CH₃, -C(OH)(CH₃)₂, -CH₂CH(OH)CH₃, -CH(OH)CH₂OH and -CH₂OCH₃.

In an alternative definition preferred are compounds of the formula (I) having a substituent **R^{C}** with a molecular weight of <200 g/mol, more preferable <100 g/mol, and most preferable <50 g/mol.

In an alternative definition preferred are compounds of the formula (I) having substituents **R^{A}**, **R^{B} and R^{C}** with a combined molecular weight of <400 g/mol, more preferable <300 g/mol, and most preferable <250 g/mol.

In another embodiment the general formula is formula (V): 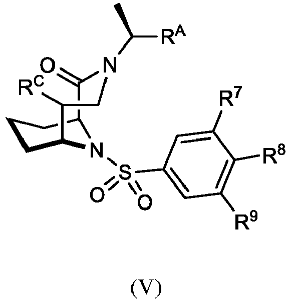 wherein and the substituents **R^{A} R^{B} , R^{C}** and **R⁷** - **R⁹** have the meanings as defined for formula (I) herein.

Yet further embodiments are the general formulas (VI) and (VIa): 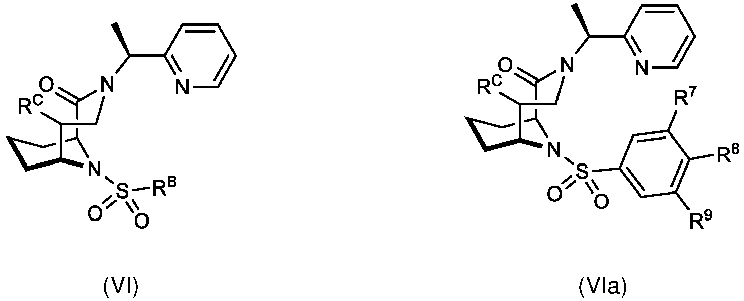 wherein the substituents **R^{B}, R^{C}** and **R⁷** - **R⁹** have the meanings as defined for formula (I) herein.

Yet further embodiments are the general formulas (VII) and (VIIa): 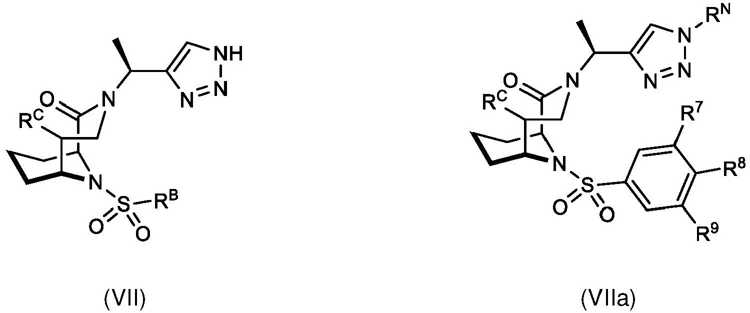 wherein the substituents **R^{B}, R^{C}** , **R^{N}** and **R⁷** - **R⁹** have the meanings as defined for formula (I) herein.

Yet further embodiments are the general formulas (VIII) and (Villa): 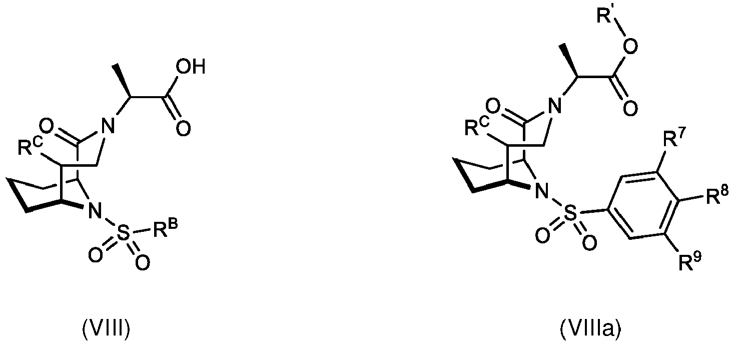 wherein the substituents **R^{B}, R^{C}** , **R^{N}**, R' and **R⁷** - **R⁹** have the meanings as defined for formula (I) herein.

Further embodiments are compounds of general formula (IX) an (X): 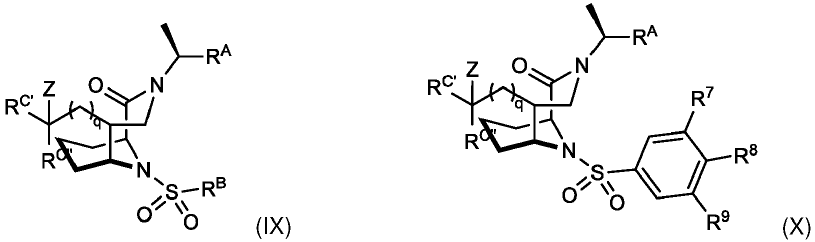 wherein the substituents **R^{A}**, **R^{B}** and **R⁷** - **R⁹** have the meanings as defined for formula (I) herein;
**R^{C'}** and **R^{C"}** represent independently of each other, -H, -CH₃, -C₂H₅, -CH₂-OH;
**Z** represents -OH, -OCH₃, -OC₂H₅, -OCH(CH₃)₂, -NH₂, -NH(CH₃), -NH(C₂H₅), -NHCH(CH₃)₂, -N(CH₃)₂, or -N(C₂H₅)₂; and **q** is 0 or 1.

Further embodiments are compounds of formula (IX) or (X), wherein
**R^{A}** represents -CH₂-OH, -CH₂-OCH₃, -COOCH₃, -COOH or 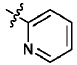
and/or compounds of formula (IX) wherein **R^{B}** represents

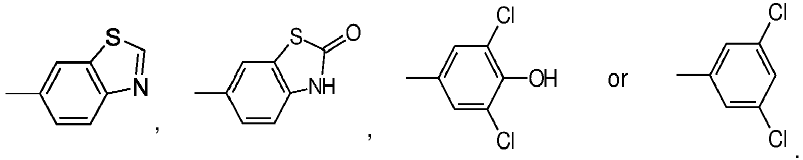

Further embodiments are compounds of formula (V), 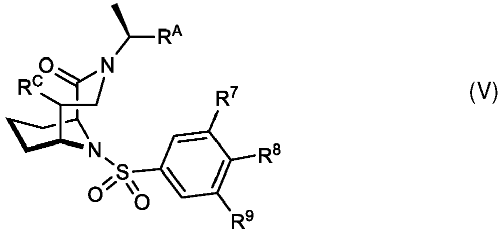
wherein and the substituent **R^{C}** has the meanings as defined for formula (I) herein;
wherein in general formula (V) and (X) **R⁷** - **R⁹** represent independently of each other -H, -F, -Cl, -Br, -I, -OH, -OCH₃, -OC₂H₅, -OC₃H₇, -CN, -CONH₂, -NHCOCH₃, -NHCOC₂H₅, -NHCOC₃H₇, -COOH, -COOCH₃, -COOC₂H₅, -COOC₃H₇, -Ph, 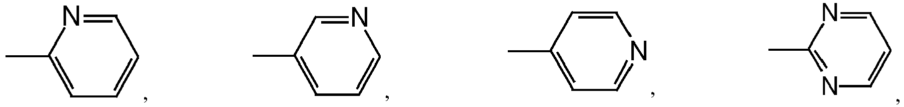 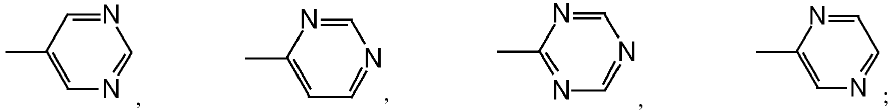
and in further embodiments -F, -Cl, -Br, -OH, -OCH₃, -CN, -CONH₂, -NHCOCH₃, -COOCH₃, -Ph, or

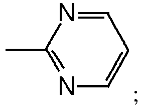

Further embodiments for any general formula disclosed herein and especially for general formulae (I), (V), (IX) and (X) substituents for **R^{A}** are preferably selected from: 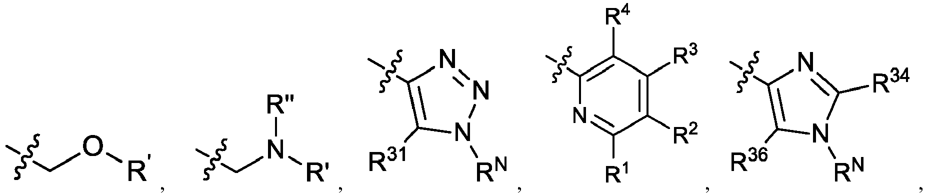 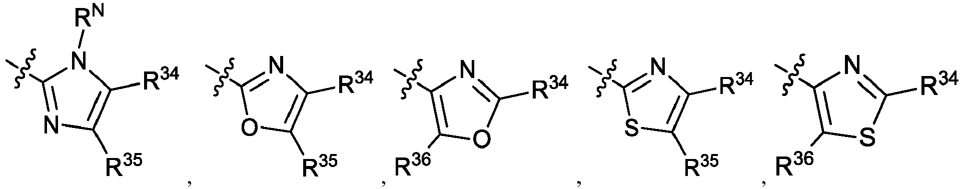 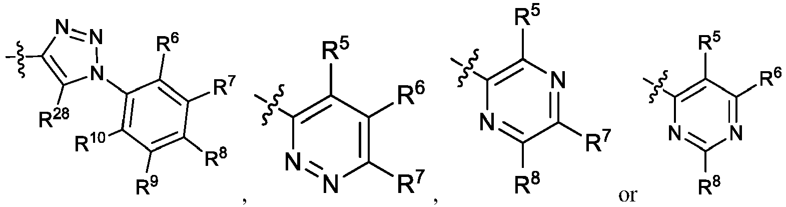 wherein **R¹** - **R⁸**, **R¹⁵** - **R¹⁶**, **R³¹**, **R³⁴** - **R³⁶** and **R^{N}** have the meanings as defined in the general formula (I) and **R'** and **R"** represent independently of each other -H, -CH₃, -C₂H₅, -CH(CH₃)₂, -CF₃, -COCH₃.

In further embodiments the compounds of the formula (I) are having one of the following substituents **R^{A}**:

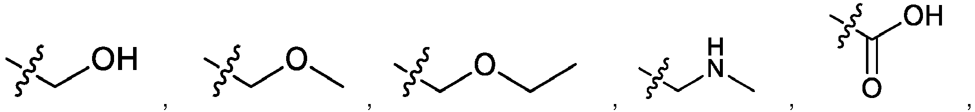

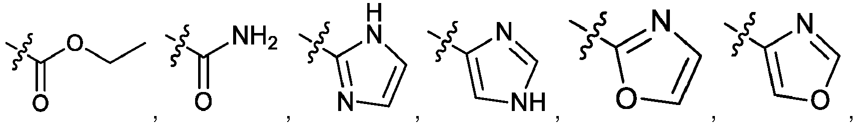

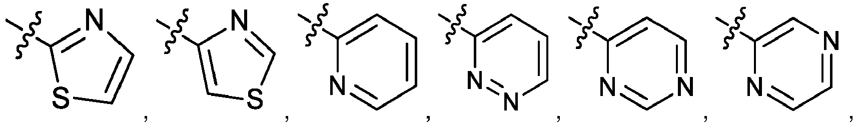

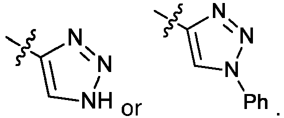

In further embodiments the compounds of the formula (I) are having one of the following substituents **R^{A}**:

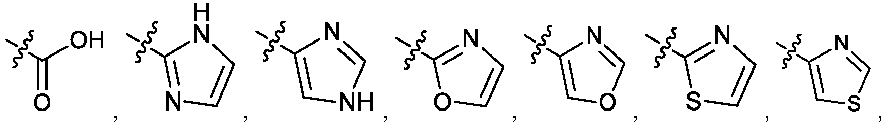

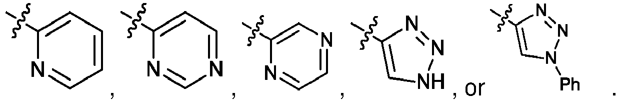

Further embodiments for molecules according to the present invention are:

| | |
|---|---|
| **6d** | (1S,5S,6R)-10-((3,5-dichlorophenyl)sulfonyl)-3-((S)-1-(pyridin-2-yl)ethyl)-5-vinyl-3,10-diazabicyclo[4.3.1]decan-2-one |
| **6f** | (1S,5S,6R)-10-((3,5-dichlorophenyl)sulfonyl)-3-((S)-4-(trimethylsilyl)but-3-yn-2-yl)-5-vinyl-3,10-diazabicyclo[4.3.1]decan-2-one |
| **7d** | (1S,5S,6R)-10-(benzo[d]thiazol-6-ylsulfonyl)-3-((S)-1-(pyridin-2-yl)ethyl)-5-vinyl-3,10-diazabicyclo[4.3.1]decan-2-one |
| **15a** | (1S,5S,6R)-3-((S)-1-(benzyloxy)propan-2-yl)-10-((3,5-dichlorophenyl)sulfonyl)-5-vinyl-3,10-diazabicyclo[4.3.1]decan-2-one |
| **16a** | (1S,5S,6R)-10-(benzo[d]thiazol-6-ylsulfonyl)-3-((S)-1-(benzyloxy)propan-2-yl)-5-vinyl-3,10-diazabicyclo[4.3.1]decan-2-one |
| **17a** | (1S,5S,6R)-10-(benzo[d]thiazol-6-ylsulfonyl)-3-((S)-1-hydroxypropan-2-yl)-5-vinyl-3,10-diazabicyclo[4.3.1]decan-2-one |
| **18b** | (1S,5S,6R)-3-((S)-but-3-yn-2-yl)-10-((3,5-dichlorophenyl)sulfonyl)-5-vinyl-3,10-diazabicyclo[4.3.1]decan-2-one |
| **19b** | (1S,5S,6R)-10-((3,5-dichlorophenyl)sulfonyl)-3-((S)-1-(1-(4-methoxyphenyl)-1H-1,2,3-triazol-4-yl)ethyl)-5-vinyl-3,10-diazabicyclo[4.3.1]decan-2-one |
| **20b** | (1S,5S,6R)-10-((3,5-dichlorophenyl)sulfonyl)-3-((S)-1-hydroxypropan-2-yl)-5-vinyl-3,10-diazabicyclo[4.3.1]decan-2-one |
| **21b** | (2S)-2-((1S,5S,6R)-10-((3,5-dichlorophenyl)sulfonyl)-2-oxo-5-vinyl-3,10-diazabicyclo[4.3.1]decan-3-yl)propanoic acid |
| **22b** | (1S,5S,6R)-10-((3,5-dichlorophenyl)sulfonyl)-3-((S)-1-methoxypropan-2-yl)-5-vinyl-3,10-diazabicyclo[4.3.1]decan-2-one |
| **23b** | (1S,5S,6R)-10-((3,5-dichlorophenyl)sulfonyl)-5-(hydroxymethyl)-3-((S)-1-(pyridin-2-yl)ethyl)-3,10-diazabicyclo[4.3.1]decan-2-one |
| **24b** | (1S,5S,6R)-10-((3,5-dichlorophenyl)sulfonyl)-5-(methoxymethyl)-3-((S)-1-(pyridin-2-yl)ethyl)-3,10-diazabicyclo[4.3.1]decan-2-one |
| **25b** | (1S,5S,6R)-10-(benzo[d]thiazol-6-ylsulfonyl)-5-(hydroxymethyl)-3-((S)-1-(pyridin-2-yl)ethyl)-3,10-diazabicyclo[4.3.1]decan-2-one |
| **26b** | (1S,5S,6R)-10-(benzo[d]thiazol-6-ylsulfonyl)-5-ethyl-3-((S)-1-(pyridin-2-yl)ethyl)-3,10-diazabicyclo[4.3.1]decan-2-one |

### Synthetic Methods

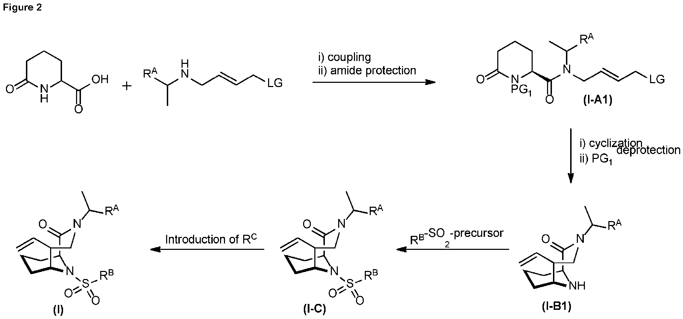

Compounds of the general formula (I) can be prepared according to the following synthetic route depicted in Figure 2. Accordingly, intermediate compound (I-A1) can be prepared by providing 6-carboxy-2-piperidone and a precursor molecule for the moiety R^{A} which has a suitable leaving group (LG) such as trimethylsilyl (TMS) and a carbon-carbon double bond in allyl position to the R^{A}-CH(CH3) amino group. Said R^{A}-CH(CH3) amino group is reacted with the carboxy moiety of 6-carboxy-2-piperidone. Subsequently, this compound undergoes a cyclization reaction upon which the leaving group LG is detached from the starting molecule. After deprotecting PG1 from amine, 5-vinyl-3,10-diazabicyclo[4.3.1]decane-2-one derivative (I-B1) is formed. This intermediate can subsequently be reacted with a suitable precursor for the moiety -SO2-RB. By suitable transformation reactions of vinyl group at C-5 position of the resulting sulphonamide compound (I-C), the compound of the general formula (I) can be obtained. Preferred, the vinyl group could be transformed by oxidation reaction with oxygen gas or epoxidation reaction.

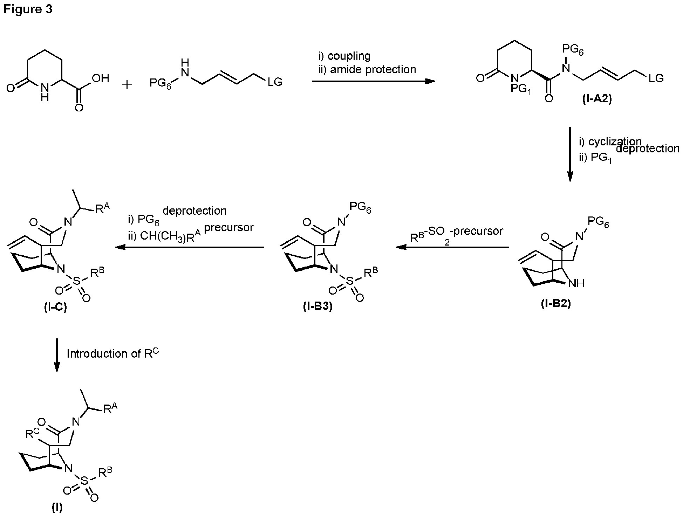

As shown in Figure 3, as synthetic route 2, intermediate compound (I-A2) can be prepared by providing 6-carboxy-2-piperidone and a butenyl amine protected with a protecting group PG6 which has a suitable leaving group (LG) such as trimethylsilyl (TMS) and a carbon-carbon double bond in allyl position to the amino group. Said protected amino group is reacted with the carboxy moiety of 6-carboxy-2-piperidone. Subsequently, this compound undergoes a cyclization reaction upon which the leaving group LG is detached from the starting molecule. After deprotecting PG1 from amide, 5-vinyl-3,10-diazabicyclo[4.3.1]decane-2-one derivative (I-B2) is formed. A sulphonamide intermediate (I-B3) can be obtained by subsequently reacting (I-B2) with a suitable precursor for the moiety -SO2-R^{B}. By deprotecting PG6 from amide group of the intermediate (I-B3) and subsequently reacting with a suitable precursor for the moiety R^{A}, a sulphonamide compound (I-C) can be produced. By suitable transformation reactions of vinyl group at C-5 position of the resulting sulphonamide compound (I-C), the compound of the general formula (I) can be obtained. Preferred, the vinyl group could be transformed by oxidation reaction with oxygen gas or ozone, epoxidation reaction or dihydroxylation catalyzed by osmium (VIII) oxide.

### Pharmaceutical Composition

The present invention also comprises pharmaceutically acceptable salts of the compounds according to the general formula (I), all stereoisomeric forms of the compounds according to the general formula (I) as well as solvates, especially hydrates thereof.

In case, the inventive compounds bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH4OH, tetraalkyl-ammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or re-crystallised from solvents such as aqueous and organic solvents. In such cases solvates may be formed. This invention includes within its scope stoichiometric solvates including hydrates as well as compounds containing variable amounts of water that may be produced by processes such as ly-ophilisation.

Certain compounds of the general formula (I) may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis- or transisomer or a mixture thereof. When an isomeric form of a compound of the invention is provided substantially free of other isomers, it will preferably contain less than 5% w/w, more preferably less than 2% w/w and especially less than 1% w/w of the other isomers.

Therefore, one aspect of the present invention is that the compounds according to the general formula (I) are suitable for use as inhibitor of FK506-binding proteins (FKBP).

In one embodiment these compounds are very potent binders to FK506-binding protein 12 (FKBP12) and FK506-binding protein 12.6 (FKBP12.6) with no immunosuppressive side effects and are therefore a valuable agents for blocking the function of FKBP12 and FKBP12.6.FKBP12 and FKBP12.6 have been implicated in cardiac diseases due to their role as regulators of ryanodine receptors and in haematological diseases due to their role as regulators of receptors of the TGRβ/ALK family. Consequently, FKBP12 and FKBP12.6 inhibitors are useful for the treatment of diseases characterized by an aberrant activity of these receptors.

Another aspect of the present invention relates to the inventive FKBP51/52 ligand derivatives for use as drugs, i.e. as pharmaceutically active agents applicable in medicine.

In one embodiment said compound is suitable for use as inhibitor of the FK506-binding protein 51 (FKBP51) and/or the FK506-binding protein 52 (FKBP52).

FKBP51 has been implicated in numerous in human diseases. Consequently, FKBP51 is a target which is addressed in order to prevent and/or treat the diseases disclosed in the afore-mentioned literature.

Thus, FKBP51 and/or FKBP 52 ligand compounds of the present invention can be for use as pharmaceutically active agent in medicine.

Preferred, the FKBP51/52 ligand compounds of the present invention can be for use in the treatment, or for the preparation of a pharmaceutical formulation for prophylaxis and/or treatment of these FKBP51/52-associated diseases such as depression, obesity or chronic pain.

In one embodiment said compound is suitable for use as inhibitor of bacterial MIP proteins such as LpMIP, TcMIP, BpMIP, CtMIP or CpMIP. These MIPs have been implicated in the infectivity or intracellular replication of the bacterial pathogens. Consequently, MIP inhibitors are useful antiinfective agents, e.g. for the treatment of Legionnaire's disease, Chagas' disease or infections by *Chlamydiae* or *Bukholderiae* species.

The inventive compound of any one of formula (I) and subformulae (II)-(XIII) is used in the manufacture of a medicament or of a pharmaceutical composition for the treatment and/or prevention of FKBP- or MIP-associated diseases.

Another aspect of the present invention relates to the inventive compounds for use in treating FKBP- or MIP-associated diseases comprising administration a therapeutically effective amount of at least one inventive compound or a pharmaceutical composition comprising at least one inventive compound.

These FKBP- or MIP-associated diseases include psychiatric and neurodegenerative diseases, disorders and conditions, for metabolic diseases such as localized adiposity or obesity, for sleep disorders, neuroprotection or neuroregeneration, for the treatment of neurological disorders, for the treatment of diseases relating to neurodegeneration, for the treatment of cancers such as malignant melanoma or acute lymphoblastic leukemia and especially steroid-hormone dependent cancers such as prostate cancer, for the treatment of glucocorticoid hyposensitivity syndromes and for peripheral glucocorticoid resistance, for asthma, especially steroid-resistant asthma, and for the treatment of infectious diseases, for stimulating neurite growth or neuroregeneration, for neuroprotection, for the use as wound healing agents for treating wounds resulting from injury or surgery; for the use in limiting or preventing hemorrhage or neovascularization for treating macular degeneration, and psychiatric disorders (such as depression or post-traumatic stress disorder), metabolic disorders (such as obesity or diabetes), infective disorders (such as Legionnaire's disease or Chagas' diseases), neurological disorders (such as Alzheimer's diseases or Parkinson's diseases) and hematologial disorders (such as hereditary hemorrhagic telangiectasia or pulmonary arterial hypertension) as well as pain diseases (such as chronic neuropathic pain) and cancers (such as prostate cancer, melanoma or glioblastoma).

The FKBP51 and/or FKBP52 ligand compounds of the present invention are preferably suitable for use in the treatment, or for the preparation of a pharmaceutical formulation for use in prophylaxis and treatment of psychiatric diseases. It is especially preferred if these psychiatric diseases are an affective disorder (ICD-10 classification: F30-F39) or an anxiety disorder.

Affective disorder is a mental disorder characterized by dramatic changes or extremes of mood. The affective disorder according to the invention is selected from the group comprising or consisting of depression, bipolar disorder, mania, substance induced mood disorder and seasonal affective disorder (SAD). Among the psychiatric diseases and disorders, the most preferred is depression, the most commonly diagnosed psychiatric disorder.

The anxiety disorder according to the invention is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

Among the hundreds of different neurodegenerative disorders, the attention has been given only to a handful, including Alzheimer's Disease, Parkinson's Disease, and amyotrophic lateral sclerosis.

Among the glucocorticoid hyposensitivity syndromes, the attention has been given to the group of related diseases enclosing resistant asthma, eosinophilic esophagitis, AIDS, rheumatoid arthritis, hypertension and diabetes, metabolic syndrome or obesity.

Among the cancers, the attention has been given to malignant melanoma, acute lymphoblastic leukemia, gliomas, idiopathic myelofibrosis, pancreatic and breast cancers, steroid-hormone dependent cancers or prostate cancer.

Among the hundreds of infectious diseases, the attention has been given to malaria and the Legionnaires' disease and Chlamydia infections.

Among the metabolic disorders, attention has been given to obesity and type 2 diabetes.

Among the neurological disorders, attention has been given to neuropathic pain and fibromyalgia.

Among the haematological disorders, attention has been given to hereditary hemorrhagic telangiectasia or pulmonary arterial hypertension.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral nontoxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and colouring agents may also be incorporated into the tablet or capsule. Powders and tablets may contain about 5 to about 95 weight-% of the benzothiophene-1,1-dioxide derived compound and/or the respective pharmaceutically active salt as active ingredient.

Suitable binders include starch, gelatine, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may comprise an additional pharmaceutically active compound or drug. The pharmaceutically active compound or drug may belong to the group of glucocorticoids. Thus, an embodiment of the current invention comprises the administration of a compound of the current invention in addition to a co-administration of glucocorticoids.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatines or starch for holding or containing compositions comprising the active ingredient(s). Capsules with hard shells are typically made of blended of relatively high gel strength gelatines from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight-% of the composition, more preferably from about 5 to about 10 weight %.

Binders are substances, which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatine and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight-% of the composition, preferably from about 3 to about 10 weight %, and more preferably from about 3 to about 6 weight %.

Lubricants refer to a class of substances, which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight-% of the composition, preferably from about 0.5 to about 2 weight %, and more preferably from about 0.3 to about 1.5 weight-% of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight-% of the final composition, preferably from about 0.5 to about 2 weight %.

Colouring agents are excipients that provide coloration to the composition or the dosage form. Such excipients can include food grade dyes adsorbed onto a suitable adsorbent such as clay or aluminum oxide. The amount of the colouring agent may vary from about 0.1 to about 5 weight-% of the composition, preferably from about 0.1 to about 1 weight %.

Said pharmaceutical compositions may further comprise at least one FKBP ligand of the general formula (I).

The pharmaceutical compositions may further comprise at least one further active agent. It is preferred if this active agent is selected from the group consisting of anti-depressant and other psychotropic drugs. It is further preferred if the anti-depressant is selected from amitriptyline, amioxide clomipramine, doxepine, duloxetine, imipramine trimipramine, mirtazapine, reboxetine, citaloprame, fluoxetine, moclobemide and sertraline.

### Description of the Figures

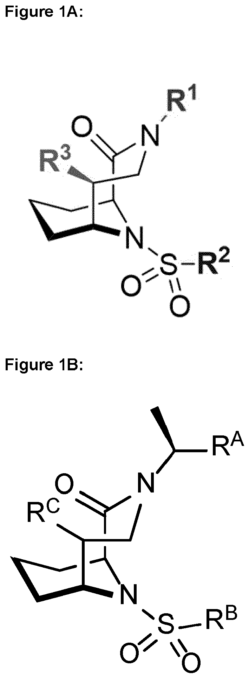

Figure 1 A: Scaffold of bicyclic-[4.3.1]-aza-amides.
Figure 1B: Scaffold of alpha-methyl substituted diazabicyclo-[4.3.1]-decane derivatives.
Figure 2: Synthetic route in order to obtain compounds of general formula (I) as described in the examples.
Figure 3: Synthetic route 2 in order to obtain compounds of general formula (I) as described in the examples.

### Examples

### Experiment 1: Synthesis of sulfonamides 6a-g and 7a, 7d, 7e

The synthesis started with compound **2,** which was generated from precursor 1 (available in two steps from phthalimide) by hydrazinolysis (Scheme 3). Reductive amination with commercially available ketones or aldehydes afforded secondary amines **3a-c** and racemic mixtures of **3d/3e** and **3f/3g,** which were coupled to commercially available (S)-6-oxo-2-piperidinecarboxylic acid followed by Boc-protection. Reduction with DIBAL-H followed by HF-mediated N-acyliminium cyclization yielded the [4.3.1]aza-amide building blocks **5a-g** in good overall yields of 10 - 19 % in 8 steps. At this stage, it was possible to separate the two diastereomers **5d** and **5e** as well as **5f** and **5g** by column chromatography. Reaction with the respective sulfonyl chlorides gave sulfonamides **6a-g** and **7a, 7d, 7e** ready for testing. 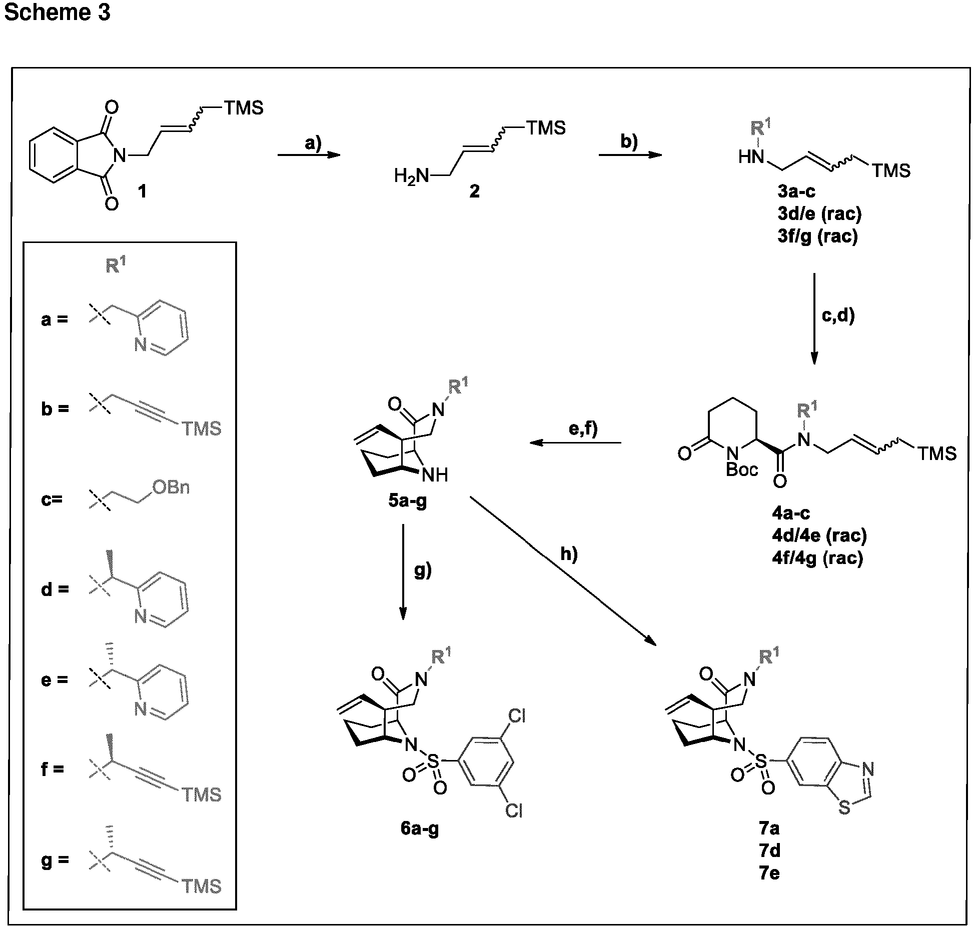 ^{a}Reagent and conditions: (a) Hydrazine, MeOH, 70 °C, 24 h; (b) **3a:** Pyridine-2-carbaldehyde, NaBH₄, EtOH, rt, 4 h, 76%, **3b:** 3-(Trimethylsilyl)-2-propynal, NaBH₄, EtOH, rt, 4 h, 41%, **3c:** benzyloxyacetaldehyde, NaBH₄, EtOH, rt, 4 h, 72%, **3d/3e:** 1-(pyridin-2-yl)ethan-1-one, TTIP, NaBH₄, EtOH, rt, 4 h, 62%, **3f/3g:** 4-(trimethylsilyl)-3-butyn-2-one, TTIP, NaBH₄, EtOH, rt, 4 h, 64% (all yields over 2 steps); (c) (S)-6-Oxopiperidine-2-carboxylic acid, HATU, rt, DMF, 2 h; (d) Boc₂O, DIPEA, DMAP, DCM, 48 h, **4a:** 60%, **4b:** 56%, **4c:** 84%, **4d/4e:** 50%, **4f/4g:** 61% (all yields over 2 steps); (e) DIBAL, THF, -78 °C, 15 min; (f) HF-pyridine, DCM, -78 °C, 1 h, **5a:** 39%, **5b:** 65%, **5c:** 74%, separation of diastereomers: **5d:** 39%, **5e:** 34%, **5f:** 50%, **5g:** 53% (all yields over 2 steps); (g) 3,5-Dichlorobenzene-1-sulfonyl chloride, DIPEA, MeCN, rt, 24 h, **6a:** 66%, **6b:** 53%, **6c:** 62%, **6d:** 42%, **6e:** 42%, **6f:** 52%, **6g:** 50%; (h) Benzo[d]thiazole-6-sulfonyl chloride, DIPEA, MeCN, rt, 24 h, **7a:** 29%, **7d:** 43%, **7e:** 43%

### Experiment 2: Synthesis of bicyclic intermediates 14a and 14b

The two bicyclic intermediates **14a** and **14b** were synthesized via a different route, depicted in **Scheme 4.** The synthesis commenced with commercially available (S)- or (R)-2-amino-1-propanol, which was first benzyl-protected and afterwards nosyl-protected. Allylation with allyl bromide followed by metathesis with Grubbs 1^{st} generation catalyst gave **11a** and **11b,** which were nosyl-deprotected to give the secondary amines **12a** and **12b.** Coupling to (S)-6-oxo-2-piperidinecarboxylic acid and Boc-protection was followed by reduction with DIBAL-H and treatment with HF to give the bicyclic[4.3.1]aza-amides **14a** and **14b.** Reaction with the respective sulfonyl chlorides gave sulfonamides **15a, 15b** and **16a, 16b.** The latter were treated with boron trichloride to give the alcohols **17a** and **17b.** 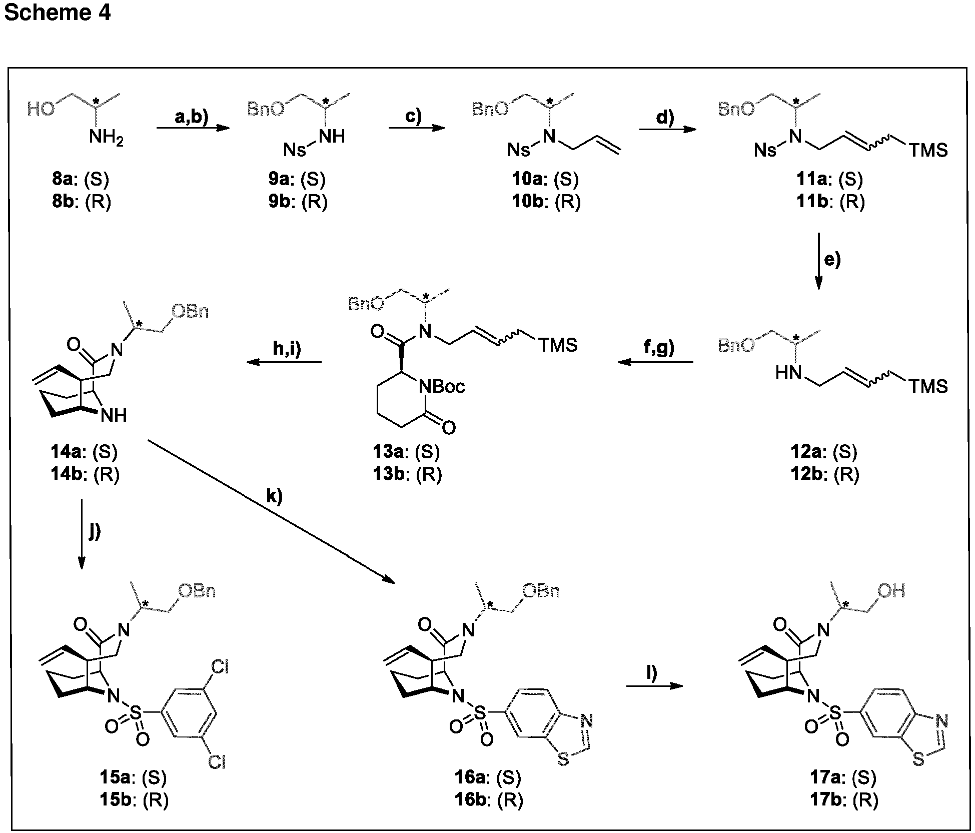 ^{a}Reagent and conditions: (a) NaH, Bn-CI, THF, reflux, 3 h; (b) Ns-Cl, DIPEA, DCM, 1 h, **9a:** 66%, **9b:** 62% (over 2 steps); (c) allyl bromide, K₂CO₃, DMF, 2 h, **10a:** 73%, **10b:** 84%; (d) allyltrimethylsilane, Grubbs II, p-benzoquinone, DCM, reflux, 6h, **11a:** 44%, **11b:** 63%; (e) thiophenol, K₂CO₃, DMF, rt, 16h, **12a:** 84%, **12b:** 90%; (f) (S)-6-Oxopiperidine-2-carboxylic acid, HATU, rt, DMF, 2 h; (g) Boc₂O, DIPEA, DMAP, DCM, 48 h, **13a:** 57%, **13b:** 68% (over 2 steps); (h) DIBAL, THF, -78 °C, 15 min; (i) HF-pyridine, DCM, -78 °C, 1 h, **14a:** 63%, **14b:** 69% (over 2 steps); (j) 3,5-Dichlorobenzene-1-sulfonyl chloride, DIPEA, DMAP, DCM, rt, 16 h, **15a:** 47%, **15b:** 46%; (k) Benzo[d]thiazole-6-sulfonyl chloride, DIPEA, DMAP, DCM, rt, 16 h, **16a:** 33%, **16b:** 42%; (I) BCl₃*SMe₂, DCM, rt, 16 h, **17a:** 58%, **17b:** 70%

### Experiment 3: Functionalization of R¹-residues

Functionalization of R¹-residues, specificallly the TMS-protected alkyne and the benzyl-protected alcohol, are depicted in **Scheme 5.** TMS was removed with potassium carbonate and the free alkyne was subjected to copper(I)-catalyzed alkyne-azide cycloaddition yielding the triazole series **19a-c.** Removal of the benzyl-protective group with boron trichloride gave alcohols **20a-c,** which were either oxidized with Jones-reagent to carboxylic acid derivatives **21a-c** or methylated with methyl iodide to give compounds **22a-c.** 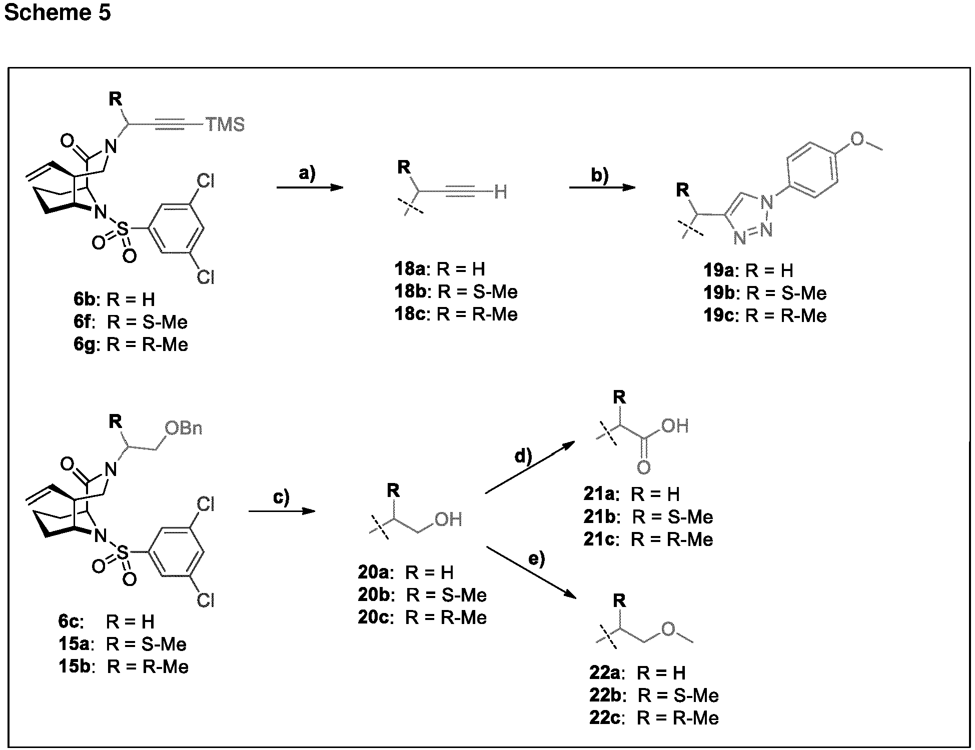 ^{a}Reagent and conditions: (a) THF/2M NaOH, rt, 5 h, **18a:** 80%, **18b:** 87%, **18c:** 84%; (b) 1-azido-4-methoxybenzene, CuSO₄, sodium ascorbate, tert-butanol/water, rt, 16 h, **19a:** 85%, **19b:** 33%, **19c:** 50%; (c) BCl₃*SMe₂, DCM, rt, 16 h, **20a:** 84%, **20b:** 47%, **20c:** 76%; (d) Jones-reagent, acetone, rt, 5 h, **21a:** 40%, **21b:** 56%, **21c:** 35%; (e) methyl iodide, NaH, DMF, 0 °C, 2h, **22a:** 77%, **22b:** 20%, **22c:** 65%

### Experiment 4: Functionalization of the vinyl group at C5 (R³)

Functionalization of the vinyl group at C5 (R³) is depicted in **Scheme 6.** The vinyl group was di-hydroxylated with osmium tetroxide and cleaved to the aldehyde by (diacetoxyiodo)benzene or sodium periodate. Reduction with sodium borohydride furnished alcohols **23a** and **23b,** and **25a** and **25b.** Methylation of **23a** and **23b** gave methyl ethers **24a** and **24b.** Reduction of the vinyl group with palladium-catalyzed hydrogenation gave compounds **26a** and **26b.** 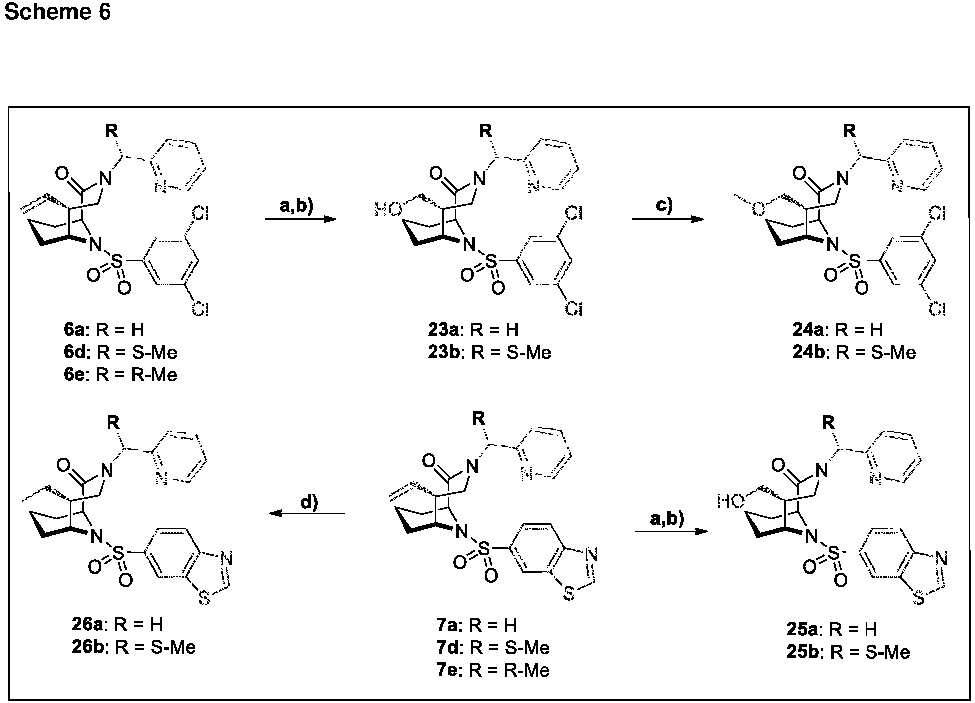 ^{a}Reagent and conditions: (a) OsO₄, NaIO₄, 2,6-lutidine, dioxane/H₂O, rt, 20 h; (b) NaBH₄, EtOH, 1 h, rt, **23a:** 47%, **23b:** 58%, **25a:** 27%, **25b:** 70% (all yields over 2 steps); (c) Mel, DMF, 1h, **24a:** 91%, **24b:** 82%; (d) H₂, Pd/C, 1 h, **26a:** quant., **26b:** quant.

### Experiment 5: FP Assay

Affinity determination by fluorescence polarization and isothermal titration calorimetry. The 33 synthesized sulfonamides, which can be divided in 13 series consisting of the respective parent compound and its methylated analog(s), were tested for their binding affinities to four human FKBPs (FKBP12, FKBP12.6, FKBP51, FKBP52) by a fluorescence polarization assay (Table 1). Intriguingly, we found that binding affinities were consistently higher for the (S)-Me diastereomers compared to the parent analogs (R= H, Table 1). Conversely, the (R)-Me diastereomers displayed reduced affinities in most cases. This trend was consistent for all tested FKBPs.

**Table 1: FP Assay data for binding to human FKBPs**

| (Py= Pyridine; Bn= Benzyl; n.d.= not determined; n/a= not available). | | | | | | | |
|---|---|---|---|---|---|---|---|
| **#** | **Ki nM FKBP12** | **Ki nM FKBP12.6** | **Ki nM FKBP51** | **Ki nM FKBP52** | **R¹** | **R²** | **R³** |
| **6e** | 130 | 812 | >1000 | >1000 | (R)-Me-Py | dichlorobenzene | vinyl |
| **6a** | 1.3 | 17 | 119 | 54 | CH₂-Py | | |
| **6d** | 0.15 | 0.41 | 2.6 | 2.2 | (S)-Me-Py | | |
| **19c** | 233 | 390 | n.d. | 1033 | (R)-Me-triazole-Ph(-4-OMe) | dichlorobenzene | vinyl |
| **19a** | 15 | 16 | n.d. | 361 | CH₂-triazole-Ph-4-OMe) | | |
| **19b** | 4.3 | 16 | n.d. | 91 | (S)-Me-triazole-Ph(4-OMe) | | |
| **n/a** | n/a | n/a | n/a | n/a | (R)-Me-Py | dichlorobenzene | CH₂OH |
| **23a** | 0.35 | 3.4 | 33 | 19 | CH₂-Py | | |
| **23b** | 0.06 | 0.31 | 1.9 | 1.2 | (S)-Me-Py | | |
| **n/a** | n/a | n/a | n/a | n/a | (R)-Me-Py | dichlorobenzene | CH₂OMe |
| **24a** | 0.65 | 3.4 | 12 | 11 | CH₂-Py | | |
| **24b** | 0.04 | 0.41 | 0.83 | 0.35 | (S)-Me-Py | | |
| **7e** | 240 | 157 | 4546 | 3451 | (R)-Me-Py | benzothiazol | vinyl |
| **7a** | 7.5 | 6 | 294 | 276 | CH₂-Py | | |
| **7d** | 2.2 | 0.9 | 33 | 29 | (S)-Me-Py | | |
| **n/a** | n/a | n/a | n/a | n/a | (R)-Me-Py | benzothiazol | CH₂OH |
| **25a** | 5.5 | 3.8 | 283 | 190 | CH₂-Py | | |
| **25b** | 1.5 | 0.7 | n.d. | n.d. | (S)-Me-Py | | |
| **n/a** | n/a | n/a | n/a | n/a | (R)-Me-Py | benzothiazol | Ethyl |
| **26a** | 6.5 | 5.2 | 410 | 285 | CH₂-Py | | |
| **26b** | 1.9 | 0.6 | 27 | 22 | (S)-Me-Py | | |
| **15b** | >5000 | n.d. | >5000 | >5000 | CH-((R)-Me)-CH₂-OBn | dichlorobenzene | vinyl |
| **6c** | n.d. | n.d. | n.d. | n.d. | CH₂-CH₂-OBn | | |
| **15a** | 58 | n.d. | 1076 | 1028 | CH-((S)-Me)-CH₂-OBn | | |
| **20c** | >5000 | n.d. | >5000 | >5000 | CH-((R)-Me)-CH₂-OH | dichlorobenzene | vinyl |
| **20a** | 20 | n.d. | 403 | 387 | CH₂-CH₂-OH | | |
| **20b** | 3.5 | n.d. | 107 | 116 | CH-((S)-Me)-CH₂-OH | | |
| **22c** | >5000 | n.d. | >5000 | >5000 | CH-((R)-Me)-CH₂-OMe | dichlorobenzene | vinyl |
| **22a** | 59 | n.d. | 529 | 377 | CH₂-CH₂-OMe | | |
| **22b** | 14 | n.d. | 204 | 205 | CH-((S)-Me)-CH₂-OMe | | |
| **16b** | >5000 | n.d. | >5000 | >5000 | CH-((R)-Me)-CH₂-OBn | benzothiazol | vinyl |
| **n/a** | n/a | n/a | n/a | n/a | CH₂-CH₂-OBn | | |
| **16a** | 28 | n.d. | 263 | 316 | CH-((S)-Me)-CH₂-OBn | | |
| **17b** | >5000 | n.d. | >5000 | >5000 | CH-((R)-Me)-CH₂-OH | benzothiazol | vinyl |
| **n/a** | n/a | n/a | n/a | n/a | CH₂-CH₂-OH | | |
| **17a** | 21 | n.d. | 110 | 102 | CH-((S)-Me)-CH₂-OH | | |
| **21c** | 48 | n.d. | 123 | 296 | CH-((R)-Me)-COOH | dichlorobenzene | vinyl |
| **21a** | 33.4 | n.d. | 172 | 320 | CH₂-COOH | | |
| **21b** | 13 | n.d. | 22 | 80 | CH-((S)-Me)-COOH | | |

**Table 2: FP Assay data for binding to MIP from Legionella pneumophila**

| (Py= Pyridine; n.d.= not determined; n/a= not available). | | | | |
|---|---|---|---|---|
| **#** | **Ki nM LpMip** | **R¹** | **R²** | **R³** |
| **6e** | n.d. | (R)-Me-Py | dichlorobenzene | vinyl |
| **6a** | 1347 | CH₂-Py | | |
| **6d** | 171 | (S)-Me-Py | | |
| **n/a** | n/a | (R)-Me-Py | dichlorobenzene | CH₂OH |
| **23a** | 467 | CH₂-Py | | |
| **23b** | 36 | (S)-Me-Py | | |
| **n/a** | n/a | (R)-Me-Py | dichlorobenzene | CH₂OMe |
| **24a** | 497 | CH₂-Py | | |
| **24b** | 36 | (S)-Me-Py | | |
| **7e** | n.d. | (R)-Me-Py | benzothiazol | vinyl |
| **7a** | 5843 | CH₂-Py | | |
| **7d** | 366 | (S)-Me-Py | | |
| **n/a** | n/a | (R)-Me-Py | benzothiazol | CH₂OH |
| **25a** | 3983 | CH₂-Py | | |
| **25b** | 237 | (S)-Me-Py | | |
| **n/a** | n/a | (R)-Me-Py | benzothiazol | Ethyl |
| **26a** | 5148 | CH₂-Py | | |
| **26b** | 284 | (S)-Me-Py | | |
| **21c** | n.d. | CH-((R)-Me)-COOH | dichlorobenzene | vinyl |
| **21a** | 5148 | CH₂-COOH | | |
| **21b** | 284 | CH-((S)-Me)-COOH | | |

## Claims

1. A compound of the general formula (I): 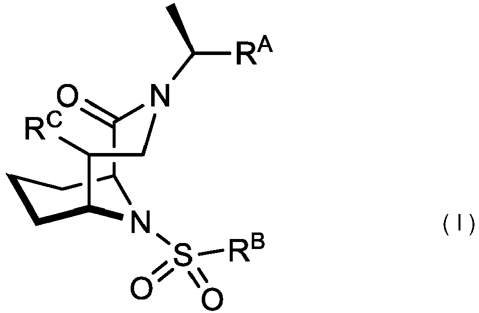
wherein the methyl group in the 1 position relative to the anchor point in the RA position is in S-configuration;
wherein **R^{A}** represents: 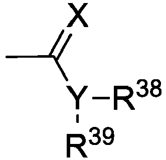
wherein **X, Y** represent independently of each other O, N, S;
or wherein **R^{A}** represents: -CH₂OR¹⁶, -CH₂NR³⁸R³⁹, 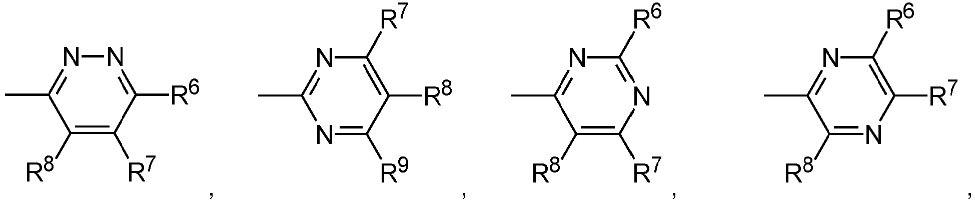 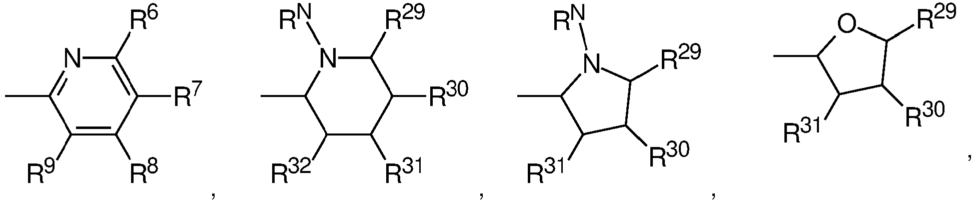 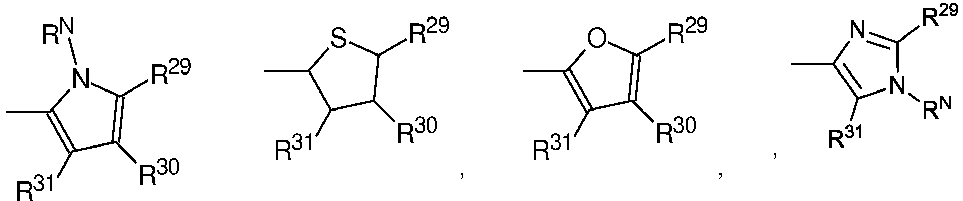 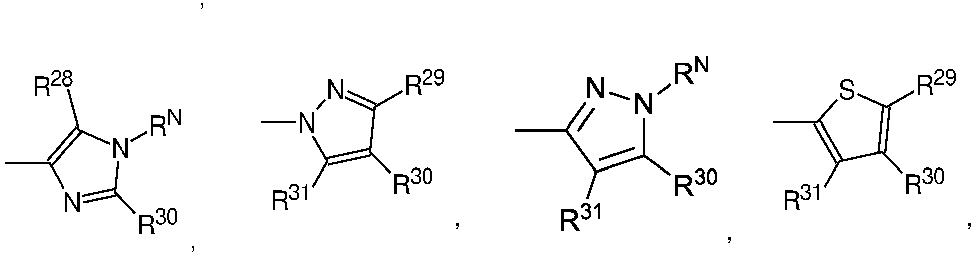 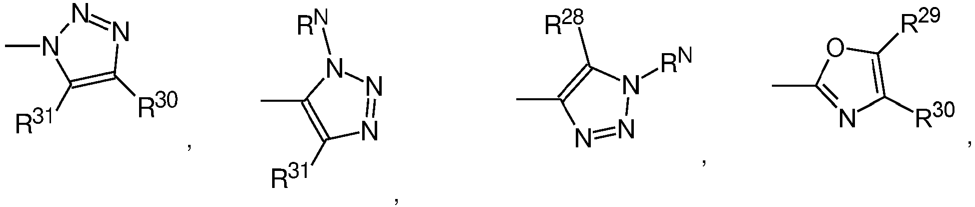 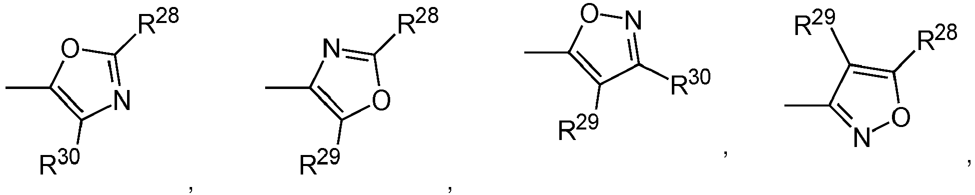 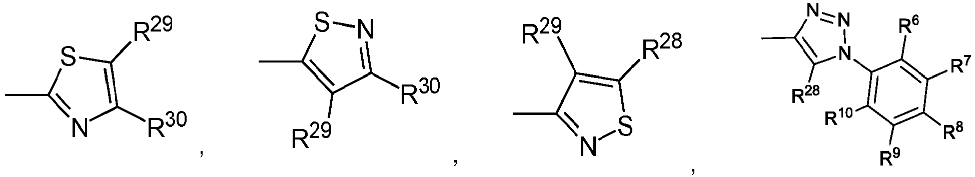 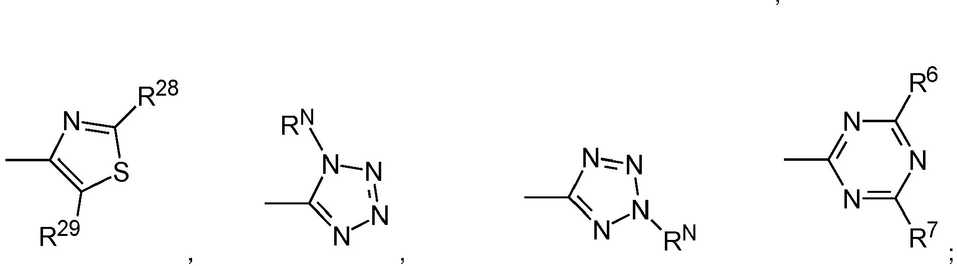 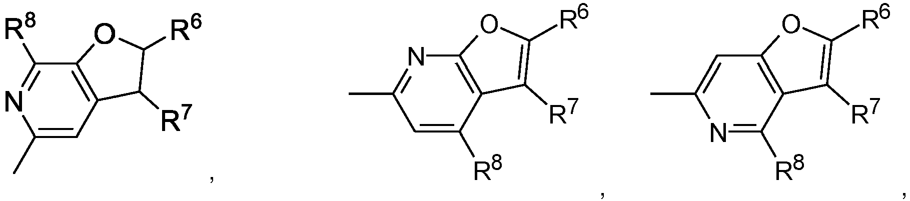 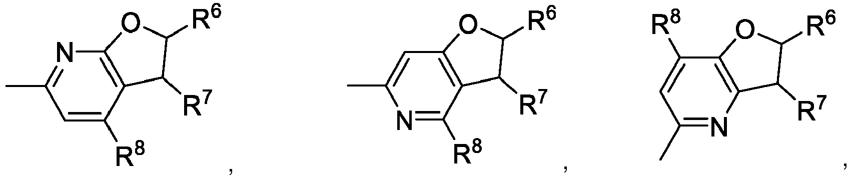 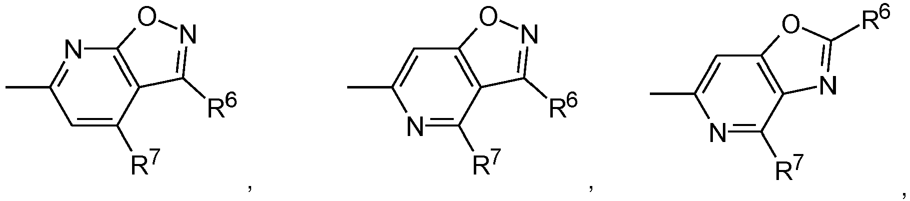 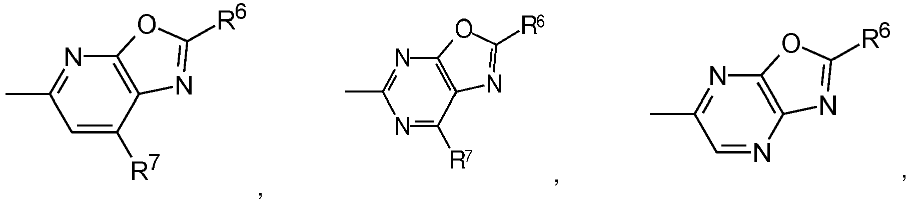 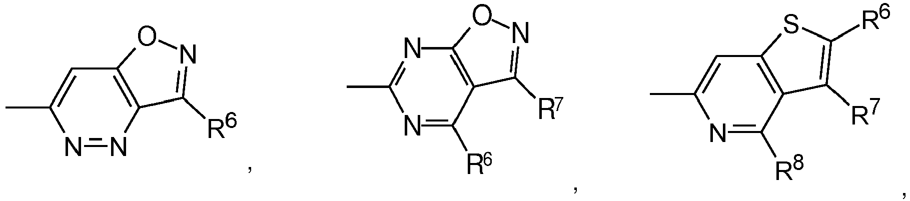 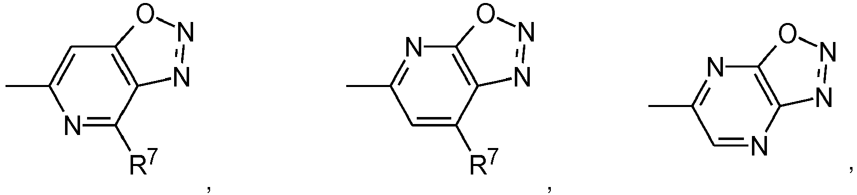 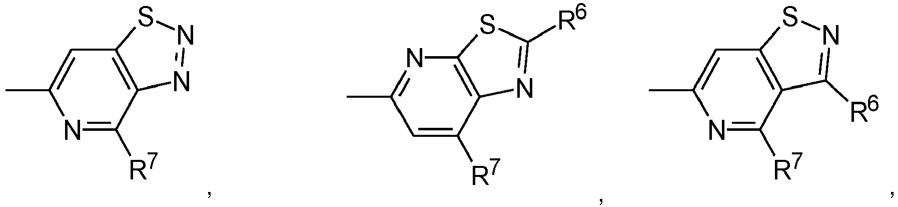 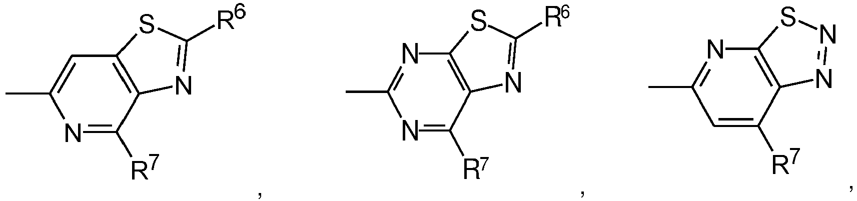 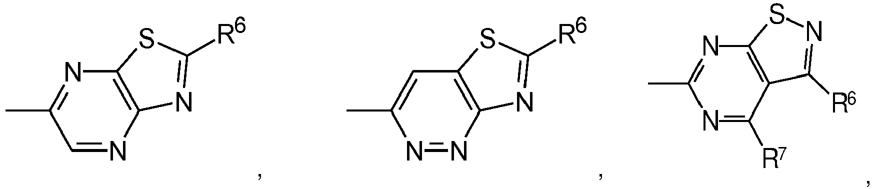 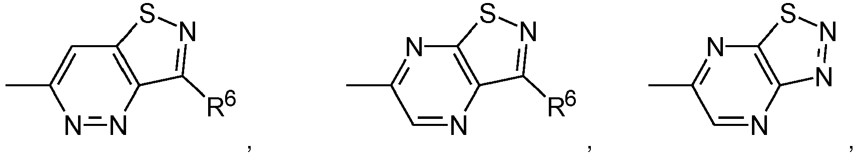 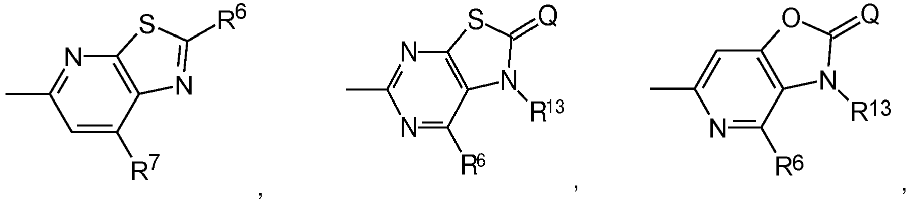 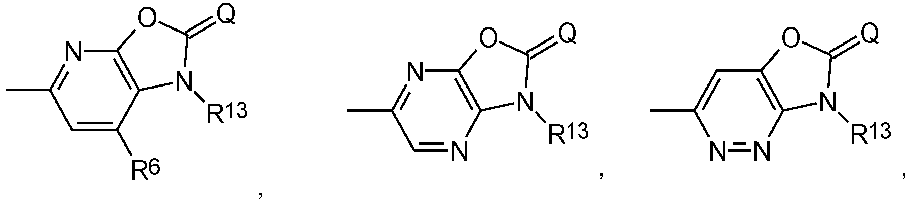 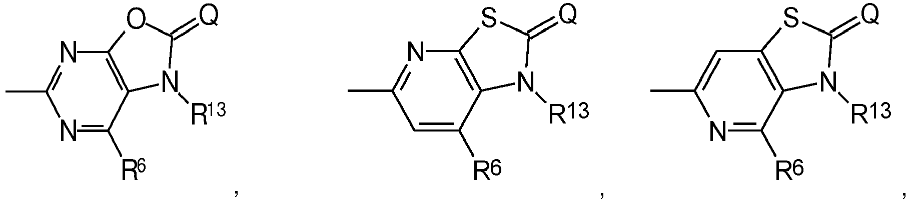 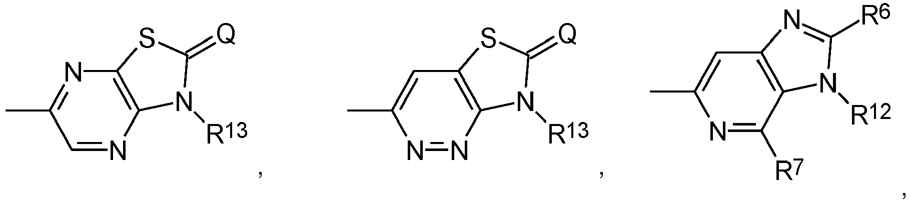 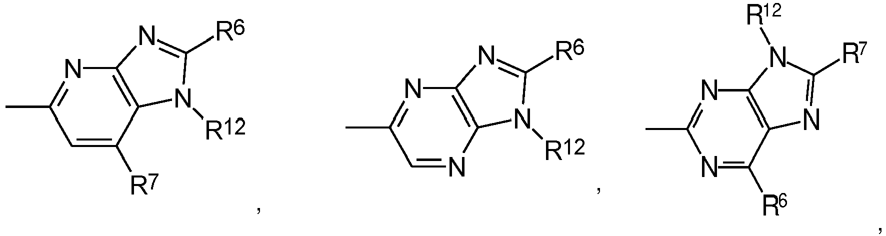 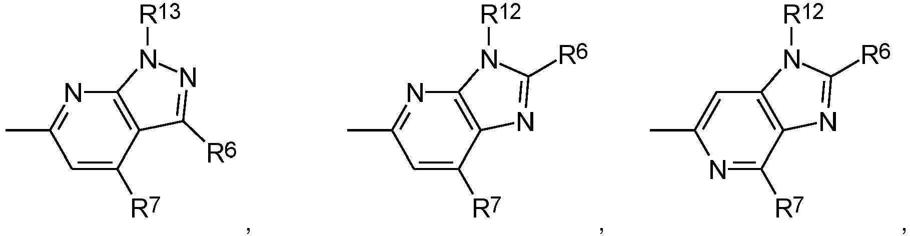 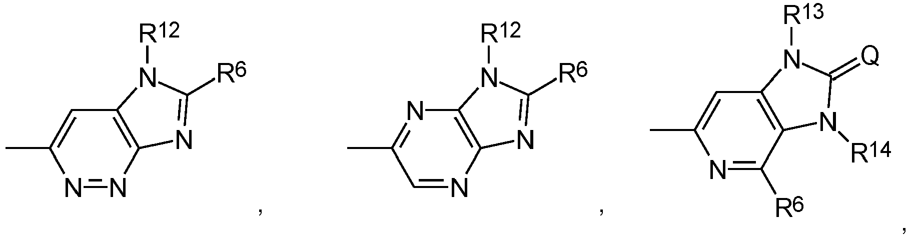 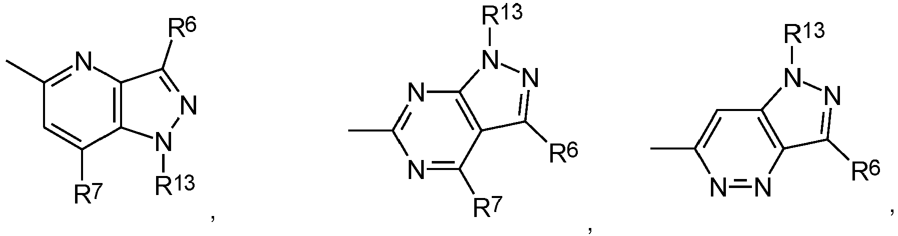 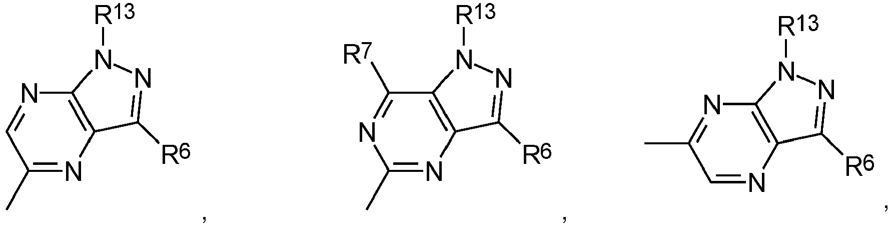 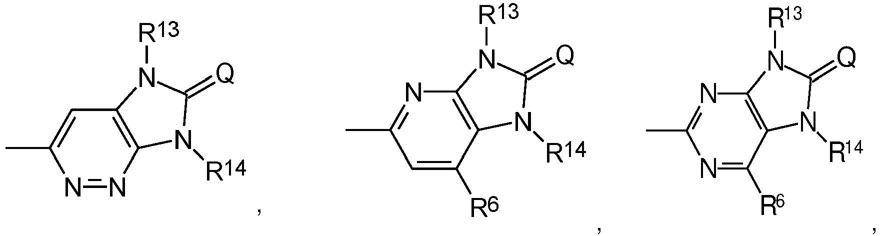 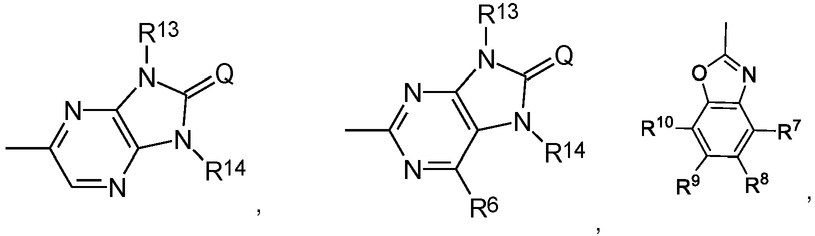 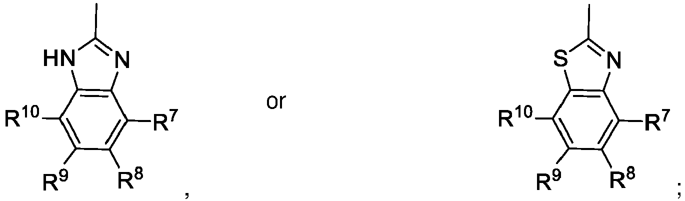
wherein **Q** represents =O, =S, or =N-R¹²;
**R^{N}** represents -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph,-CHO, -COCH₃, -COC₂H₅, -COC₃H₇, -CO-cyclo-C₃H₅, -COCH(CH₃)₂, -COC(CH₃)₃, -COPh, -COCN, -COOCH₃, -COOC₂H₅, -COOC₃H₇, -COO-cyclo-C₃H₅, -COOCH(CH₃)₂, -COOC(CH₃)₃, -COCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SO₂CH₃, -SO₂C₂H₅, -SO₂CH₂Ph, -SO₂C₃H₇, -SO₂-cyclo-C₃H₅, -SO₂CH(CH₃)₂, -SO₂C(CH₃)₃, -SO₂Ph, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH₂-C≡C-C₂H₅, -CH=CH-CH=C(CH₃)₂, -CH₂-CH(CH₃)-CH₂-C≡CH, -C₂H₄-C≡C-CH₃, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, - C≡C-CH(CH₃)-C≡CH, -CH(CH₃)-C≡C-C≡CH, -CH(C≡CH)-CH₂-C≡CH, -CH(C≡CH)-C≡C-CH₃;
**R^{B}** represents 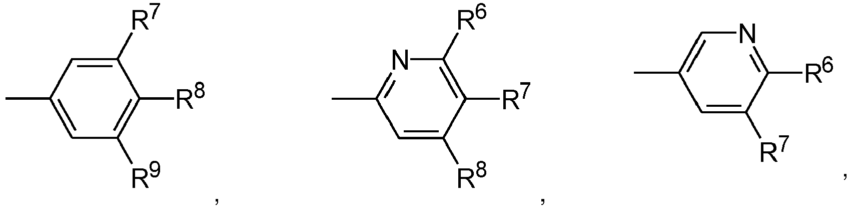 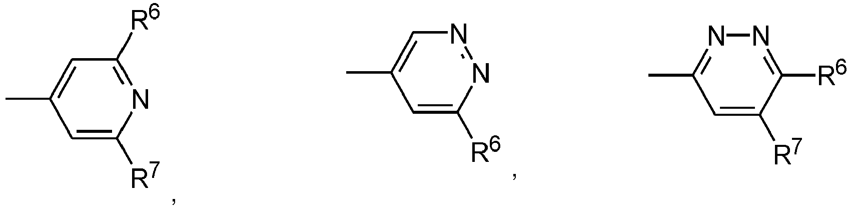 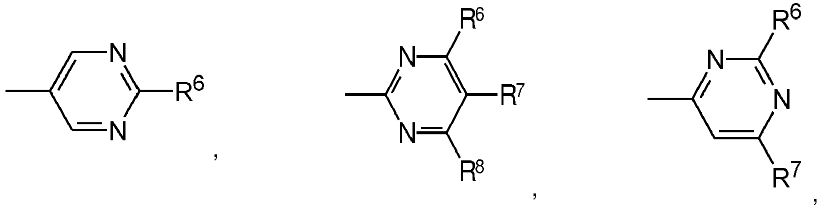 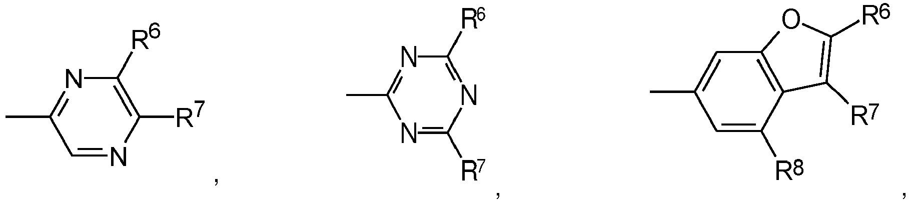 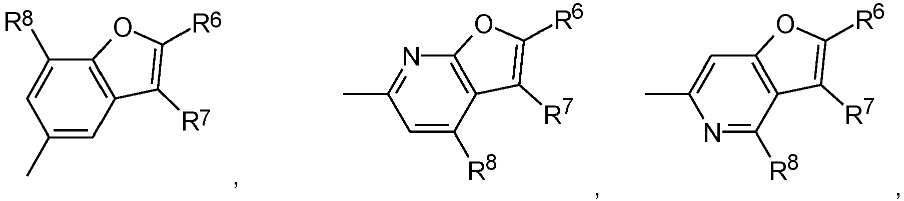 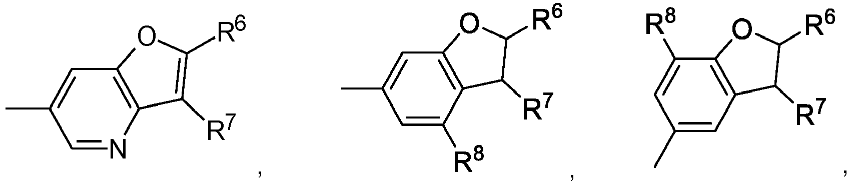 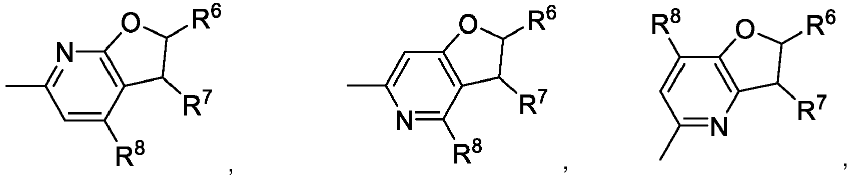 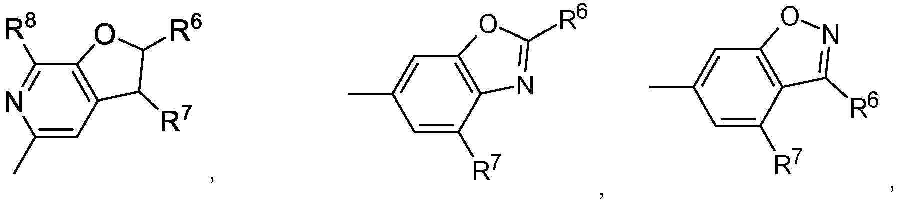 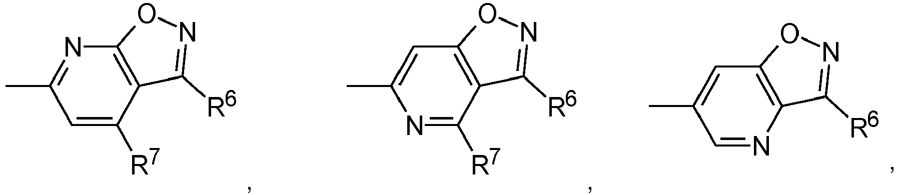 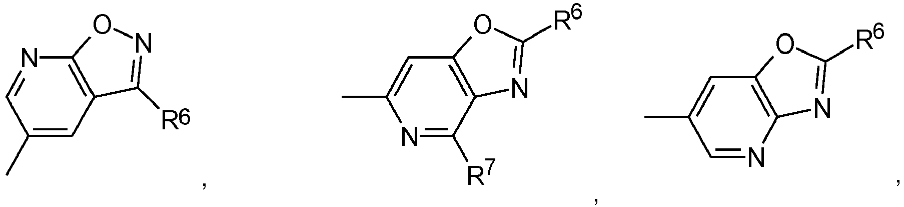 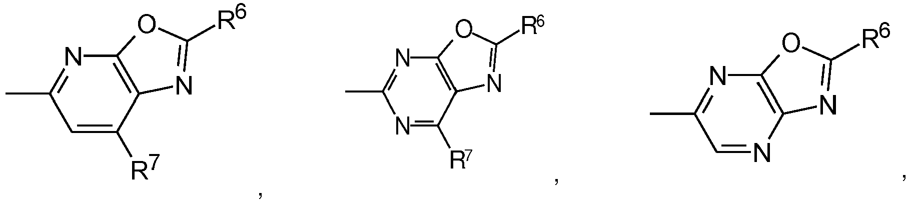 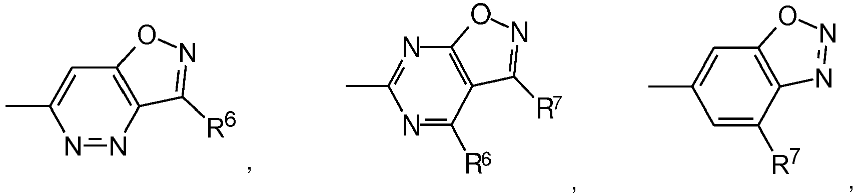 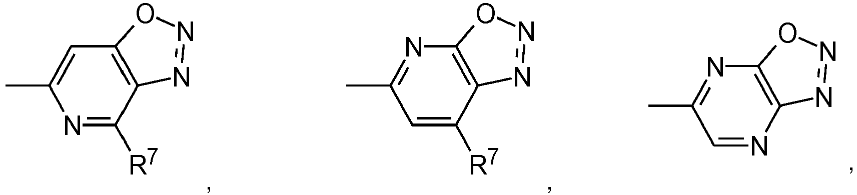 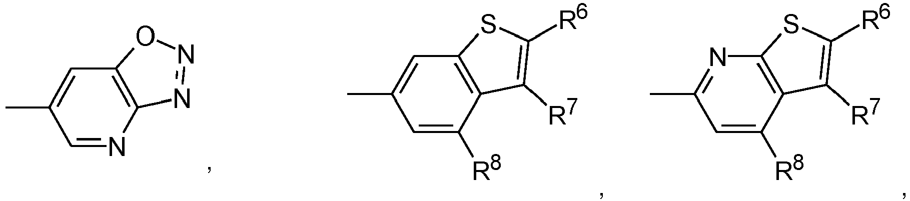 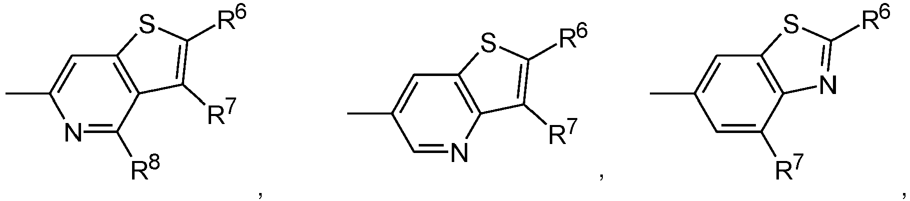 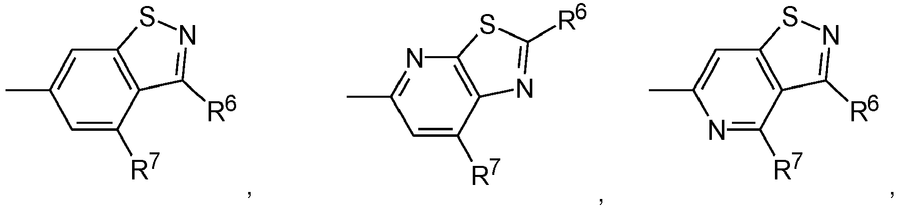 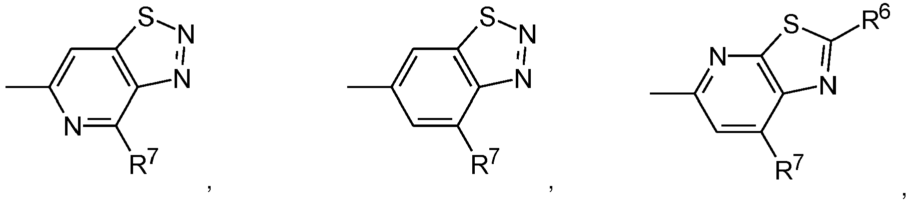 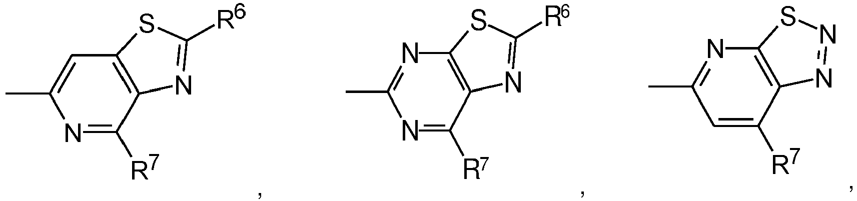 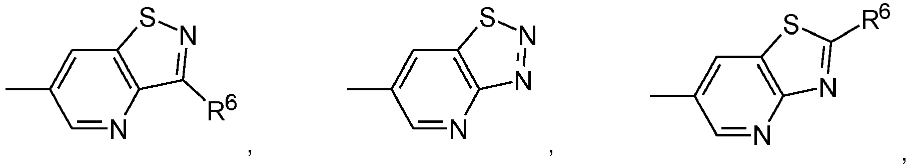 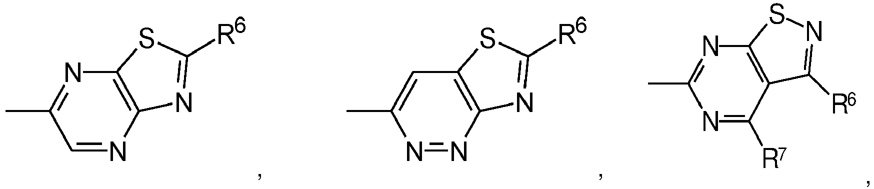 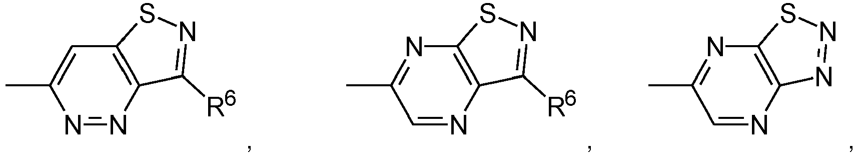 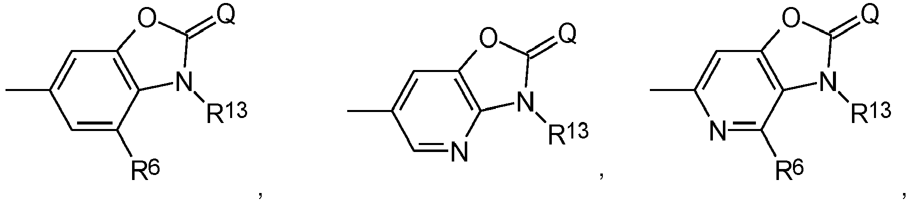 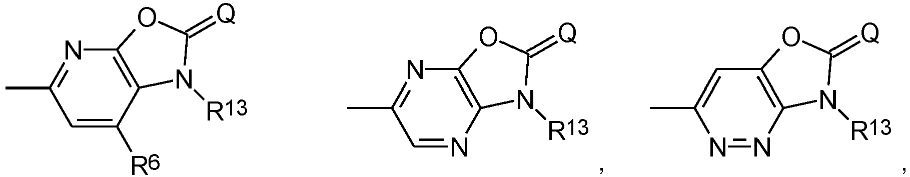 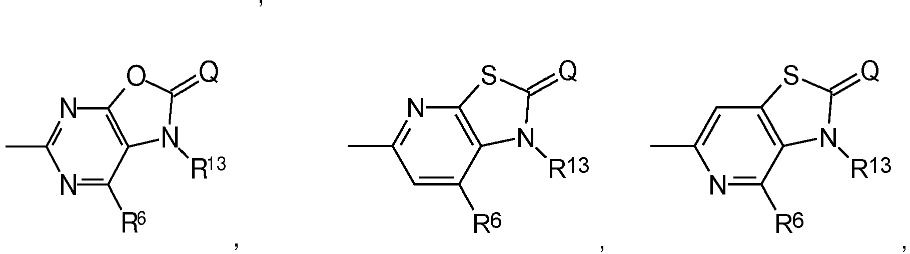 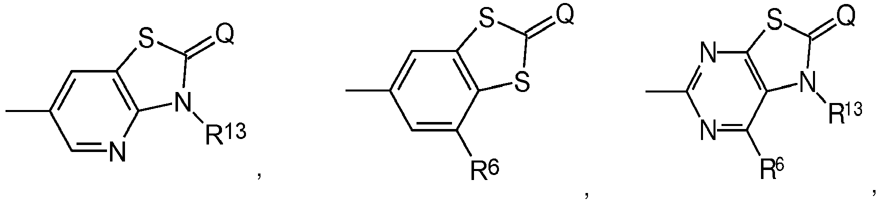 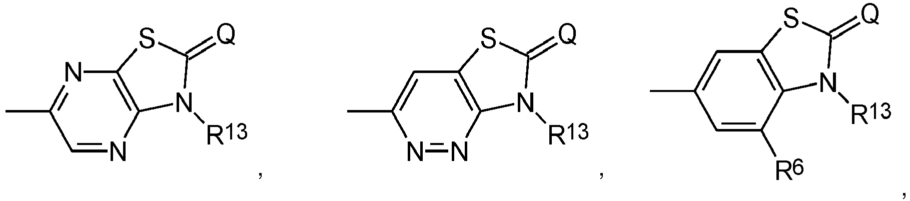 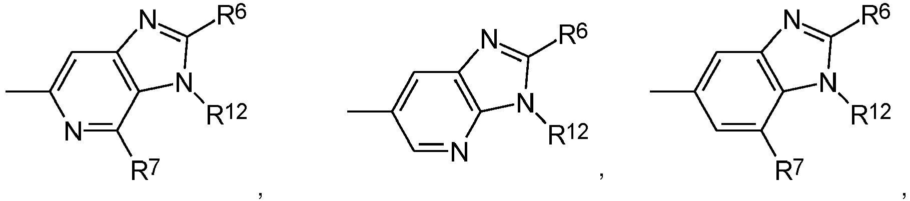 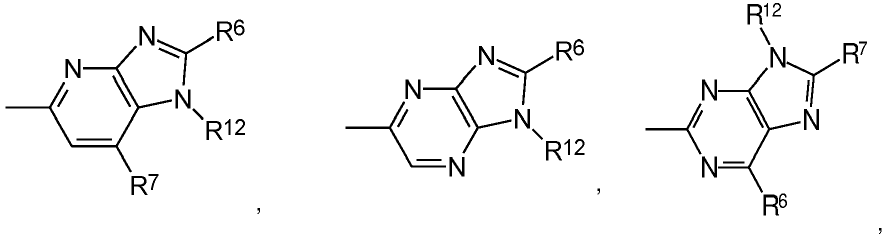 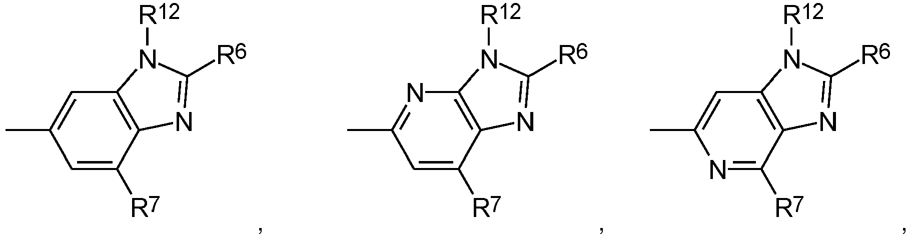 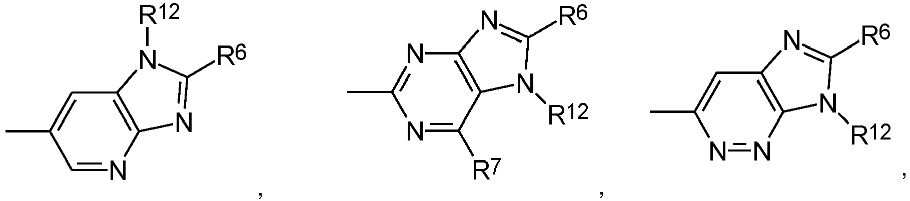 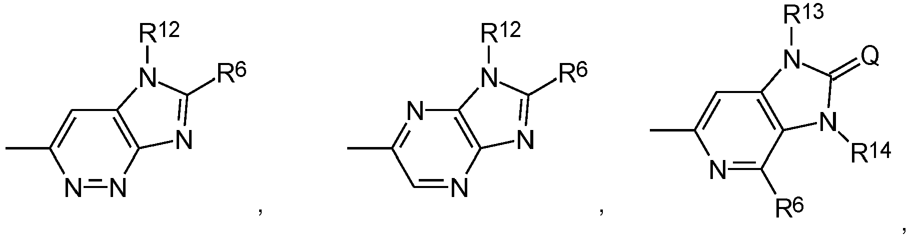 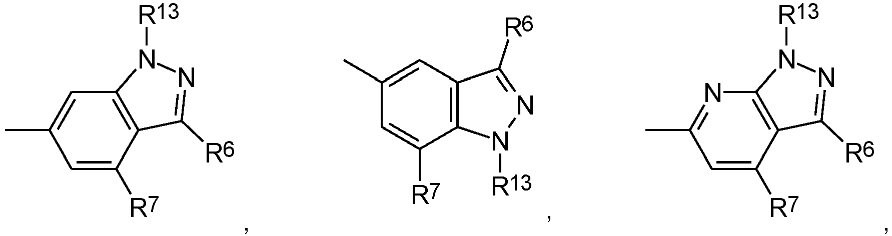 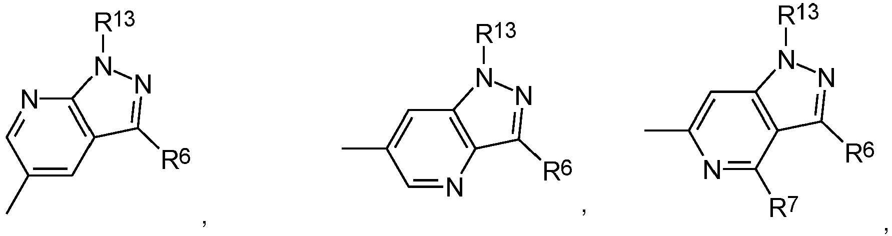 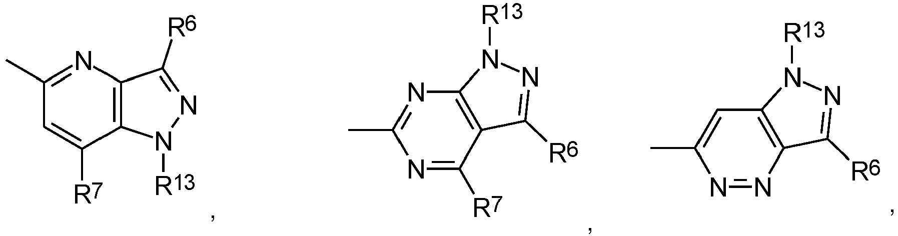 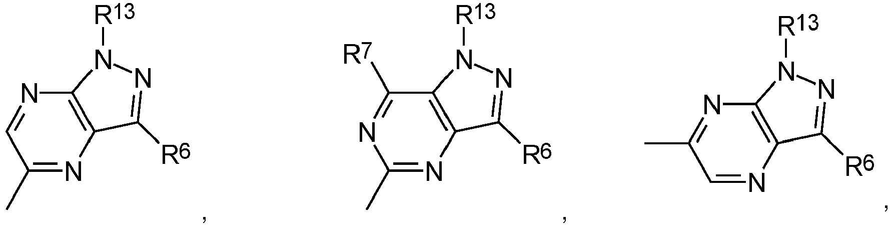 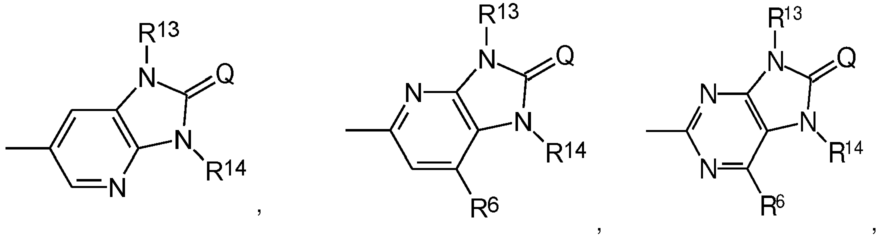 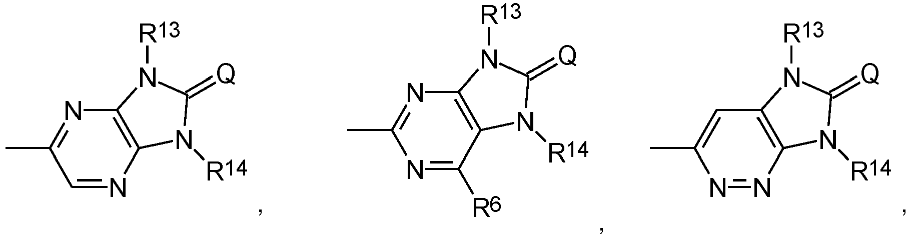 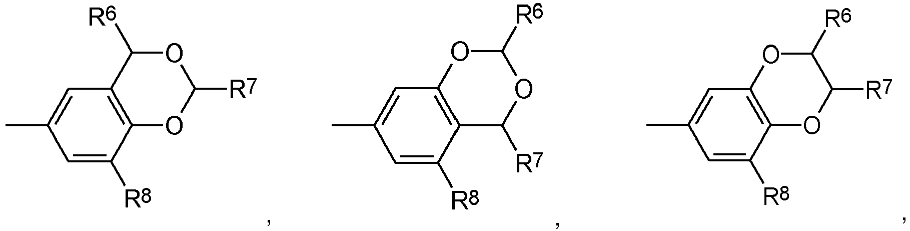 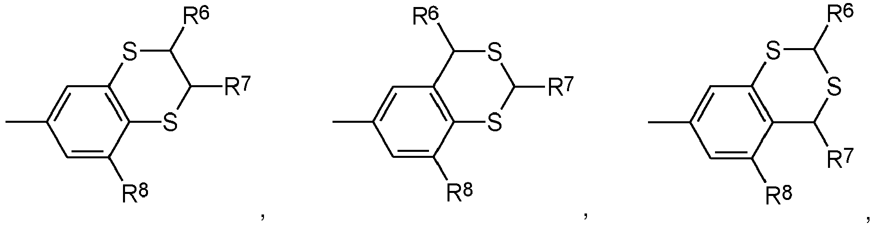 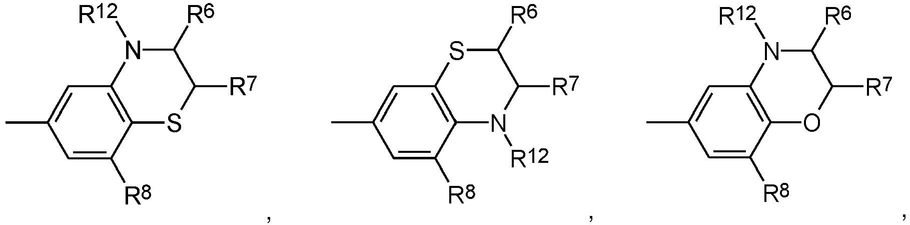 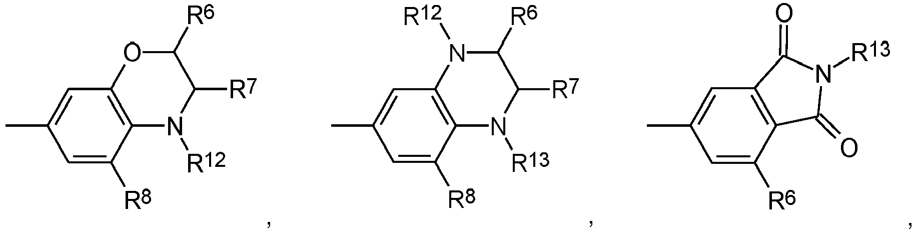 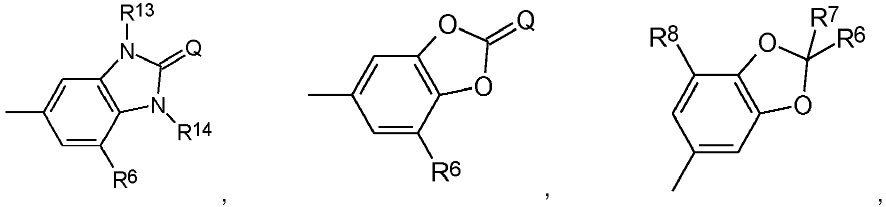 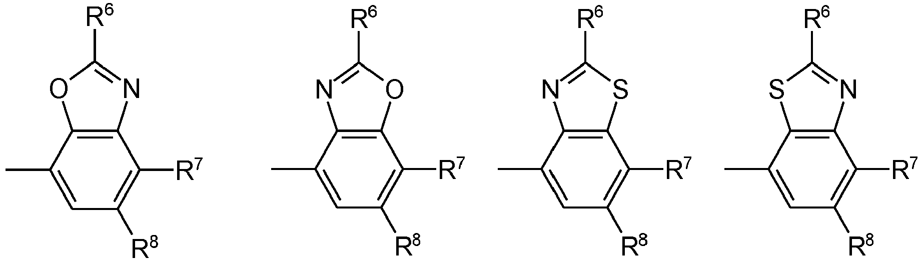 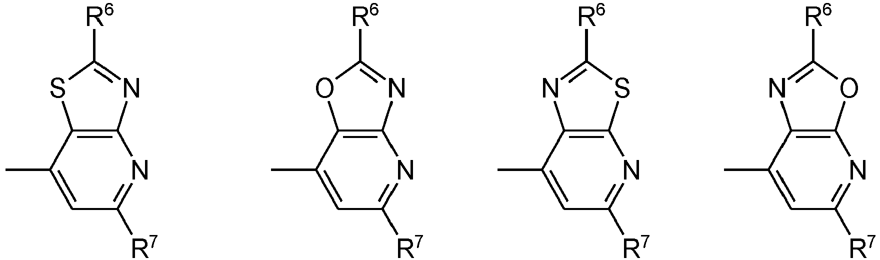 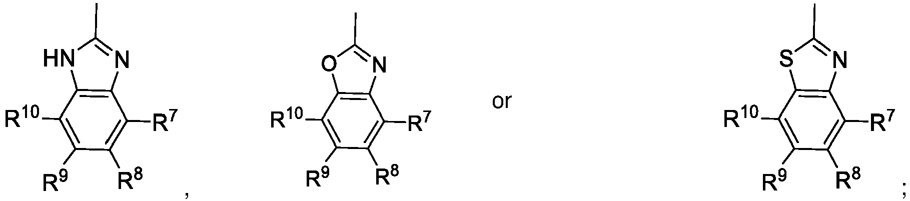
wherein **Q** represents =O, =S, or =N-R¹²;
**R^{C}** represents -H,, -OH, -CH₂-OH, -CHO, -CH₂CHO, -CH₂CH₂CHO, -C₂H₄-OH, - C₃H₆-OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -CH(OAc)-CH₂OH, -CH(OAc)-CH₂OAc, -CH(OH)-CH₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂H₅)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)CH₂OAc, -CH(NHCH₃)CH₂OAc, -CH(NHC₂H₅)CH₂OAc, -CH(N(CH₃)₂)CH₂OAc, -CH(N(C₂H₅)₂)CH₂OA_{C}, -CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)CH₂OAc, -CH₂-CH(NHCH₃)CH₂OAc, -CH₂-CH(NHC₂H₅)CH₂OAc, -CH₂-CH(N(CH₃)₂)CH₂OAc, -CH₂-CH(N(C₂H₅)₂)CH₂OA_{C}, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -NHSO₂CH₃, -NHSO₂CF₃, -NHSO₂CH₂CF₃, -CH₂-NHSO₂CH₃, -CH₂-NHSO₂CF₃, -CH₂-NHSO₂CH₂CF₃, -C₂H₄-NHSO₂CH₃, -C₂H₄-NHSO₂CF₃, -C₂H₄-NHSO₂CH₂CF₃, -CONH₂, -CONHCH₃, -CONHC₂H₅, -CONHC₃H₇, -NH(C₂H₅), -N(C₂H₅)₂, -CH₂-NH(C₂H₅), - CH₂-N(C₂H₅)₂, -C₂H₄-NH(C₂H₅), -C₂H₄-N(C₂H₅)₂, -NO₂, -CH₂-NO₂, -C₂H₄-NO₂, -CH(OH)-NO₂, -CH(NO₂)-OH, -CO₂H, -CH₂-CO₂H, -C₂H₄-CO₂H, -CH=CH-CO₂H, -CO₂CH₃, -CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -O-Si(CH₃)₃, -O-Si(C₂H₅)₃, -CO-CHO, -CO-CO-CH₃, -C(OH)-CO-CH₃, -CO-C(OH)-CH₃, -CO-CH₂-CO-CH₃, -C(OH)-CH₂-CO-CH₃, -CO-CH₂-C(OH)-CH₃, -C(OH)-CH₂-C(OH)-CH₃, -F, -Cl, -Br, -CH₂-F, -CHF₂, -CF₃, -C₂H₄-F, -CH₂-CF₃, -CF₂-CF₃, -O-CHF₂, -O-CF₃, -O-CH₂-CF₃, -O-C₂F₅, -CH₃, -CH₂CH₃, -C₃CH₇, -CH(CH₃)₂, -CH=CH₂, -C≡CH, -CH₂-CH=CH₂, or -CH₂-C≡CH_{;}
**R¹ - R¹⁰** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-0-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, 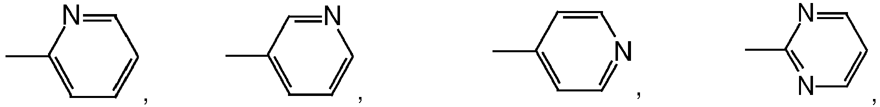 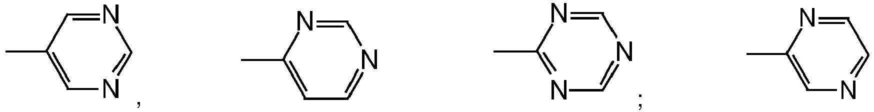
**R¹⁵** represents -**R²⁰**, -CN, -CH₂-CN, -CH₂-OR¹⁷, -CH₂-CH₂-OR¹⁷, -CH₂-NR¹⁷R¹⁸, -CH₂-NR¹⁷COR¹⁹, -CH₂-CH₂-NR¹⁷R¹⁸, -CH₂-CH₂-NR¹⁷COR¹⁹, -CO₂R¹⁷, -CO-NR¹⁷R¹⁸, -CH₂-CO₂R¹⁷, or -CH₂-CO-NR¹⁷R¹⁸;
**R¹⁶, R³⁸, R³⁹** represent independently of each other **-R²¹,** -H, -CH₃, -C₂H₅, -C₃H₇, - CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH₂OH, -CH₂-SH, -CH(OH)CH₃, -C₂H₄OH, -C₃H₆OH, -C₄H₈OH, -CH(CH₃)-C₂H₄OH, -C₅H₁₀OH, -CH₂-S-CH₃, -CH₂-CH₂-S-CH₃, -C₃H₆-S-CH₃, -CH₂OCH₃, -C₂H₄OCH₃, -C₃H₆OCH₃, -C₄H₈OCH₃, -CH(CH₃)-C₂H₄OCH₃, -C₅H₁₀OCH₃, -CH₂NH₂, -C₂H₄NH₂, -C₃H₆NH₂, -C₄H₈NH₂, -CH(CH₃)-C₂H₄NH₂, -C₅H₁₀NH₂, -CH₂-CH₂-CH₂-NH-C(NH)NH₂, -CH₂-CO₂H, -CH₂-CONH₂, -CH₂-CH₂-CO₂H, -CH₂-CH₂-CONH₂, -CH₂-CO₂CH₃, -CH₂-CONHCH₃, -CH₂-CON(CH₃)₂, -CH₂-CH₂-CO₂CH₃, -CH₂-CH₂-CONHCH₃, -CH₂-CH₂-CONH(CH₃)₂, -CH=CH-CO₂H, -CH=CH-CO₂CH₃, -CH=CH-CONHCH₃, -CH=CH-CONHC₂H₅, -CH=CH-CON(CH₃)₂, -CH=CH-CON(C₂H₅)₂, -CH₂-CH=CH-CO₂H, -CH₂-CH=CH-CO₂CH₃, -CH₂-CH=CH-CONHCH₃, -CH₂-CH=CH-CON(CH₃)₂, -CH₂-CH=CH-CONHC₂H₅, -CH₂-CH=CH-CON(C₂H₅)₂, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, - CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, - C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, - CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-Ph, 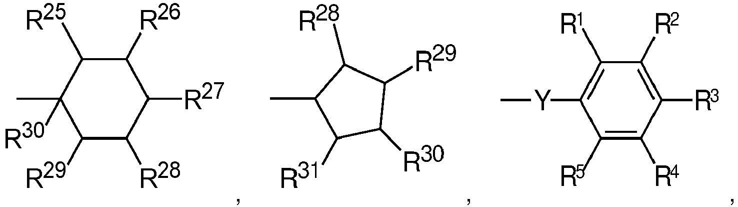 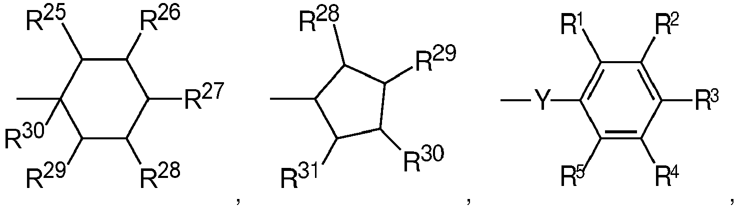 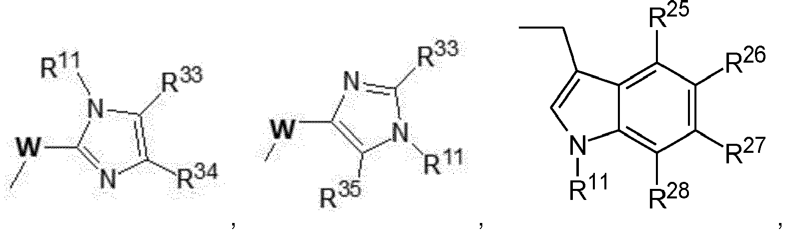 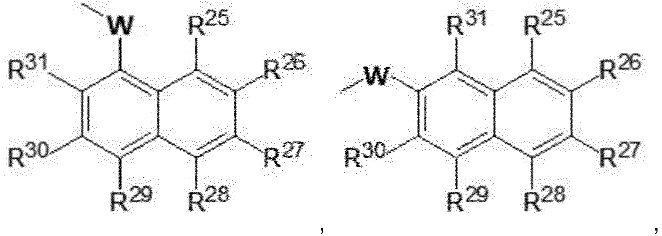 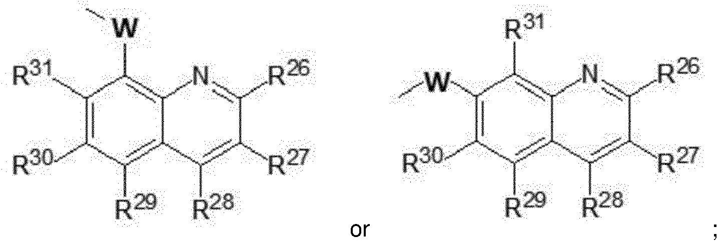
wherein **W** represents O, N-R¹², S;
**R¹¹** - **R¹⁴** and **R¹⁷- R²¹** represent independently of each other -H, -CH₂F, -CHF₂, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, or -CH₂-CH(C≡CH)₂;
**R²²- R³⁷** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇₅ -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 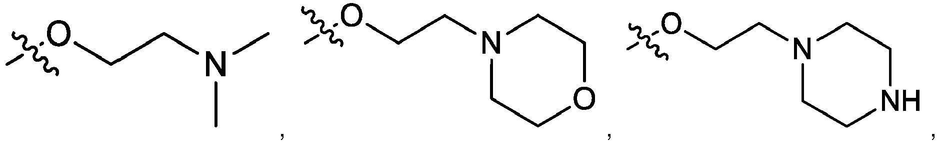 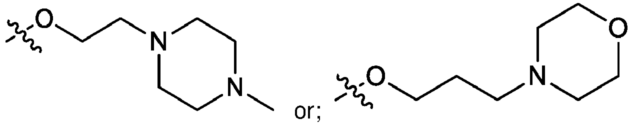
and anomers, deoxy-forms, , tautomers, hydrates, and solvates of the above mentioned compounds and pharmaceutically acceptable salts.

2. Compound according to claim 1, wherein the compound is represented by the following formula (V): 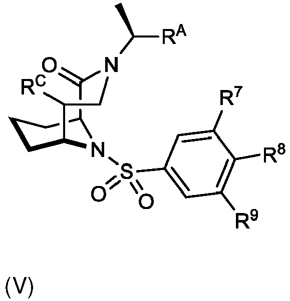
and wherein and the substituents **R^{A} R^{B}** , **R^{C}** and **R⁷** - **R⁹** have the meanings as defined for formula (I) herein;
or wherein the compound is represented by the following formulae (VI) or (Vla): 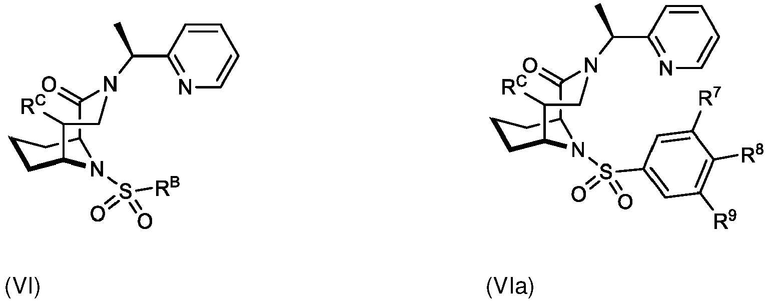
and wherein the substituents **R^{B}, R^{C}** and **R⁷** - **R⁹** have the meanings as defined for formula (I) herein;
or wherein the compound is represented by the following formulae (VII) or (VIIa): 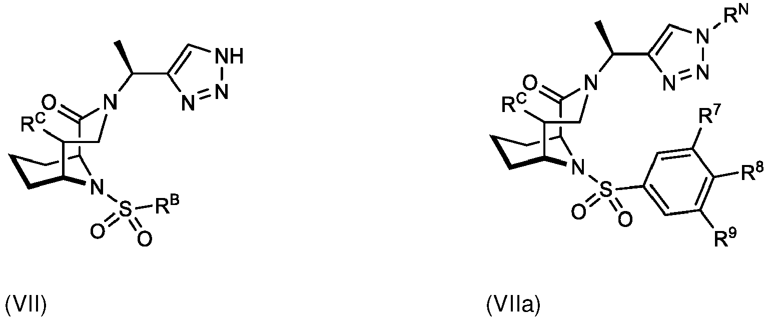
and wherein the substituents **R^{B}, R^{C}, R^{N}** and **R⁷** - **R⁹** have the meanings as defined for formula (I) herein;
or wherein the compound is represented by the following formula (VIII) or (Villa) 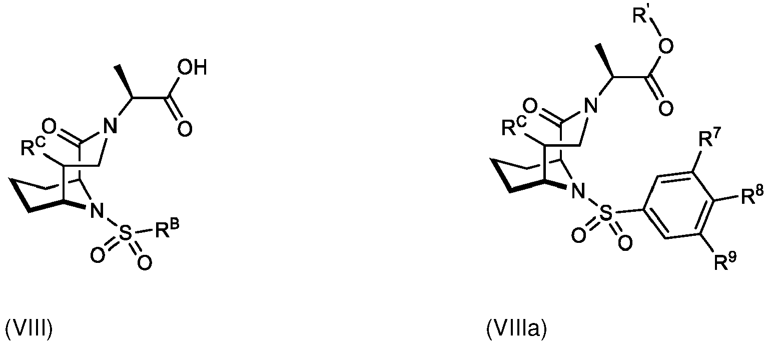
and wherein the substituents **R^{B}, R^{C} , R^{N}, R'** and **R⁷** - **R⁹** have the meanings as defined for formula (I) herein;
or wherein the compound is represented by the following formula (IX) or (X): 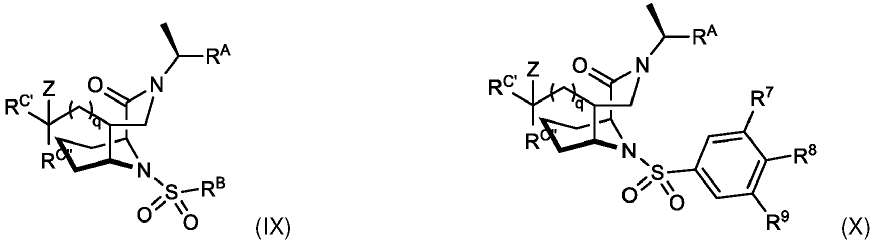
and wherein the substituents **R^{A}, R^{B}** and **R⁷ - R⁹** have the meanings as defined for formula (I) herein;
R^{C'} and R^{C"} represent independently of each other, -H, -CH₃, -C₂H₅, -CH₂-OH;
Z represents -OH, -OCH₃, -OC₂H₅, -OCH(CH₃)₂, -NH₂, -NH(CH₃), -NH(C₂H₅), -NHCH(CH₃)₂, -N(CH₃)₂, or -N(C₂H₅)₂; and q is 0 or 1;
and anomers, deoxy-forms, tautomers, hydrates and solvates of the above mentioned compounds and pharmaceutically acceptable salts.

3. Compound according to any one of the previous claims, wherein the compound is represented by the following formula (V): 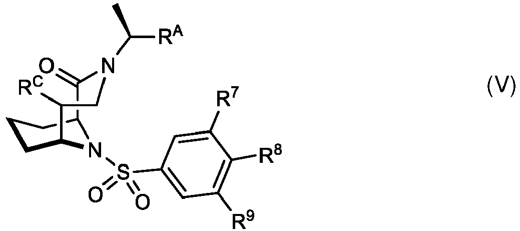
wherein **R^{A}** is selected from: 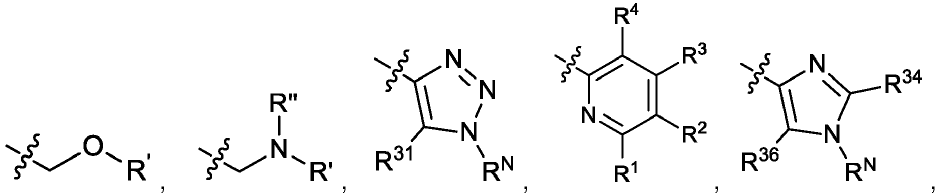 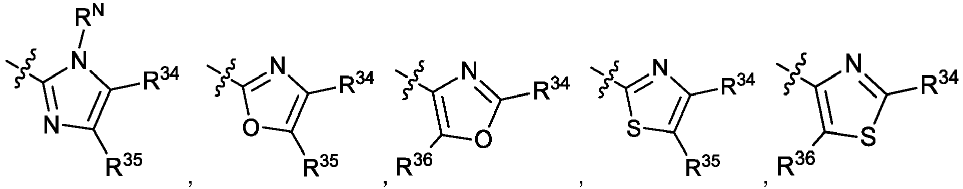 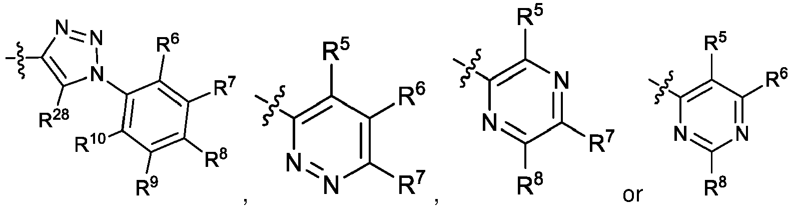
**R^{C}** has the meaning as defined for formula (I) herein;
**R⁷** - **R⁹** are independently selected from each other from: -H, -F, -Cl, -Br, -I, -OH, -OCH₃, -OC₂H₅, -OC₃H₇, -CN, -CONH₂, -NHCOCH₃, -NHCOC₂H₅, -NHCOC₃H₇, -COOH, -COOCH₃, -COOC₂H₅, -COOC₃H₇, -Ph, 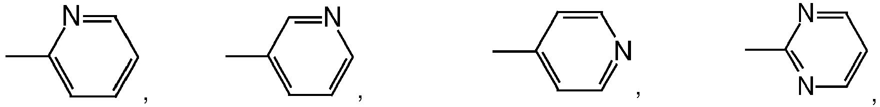 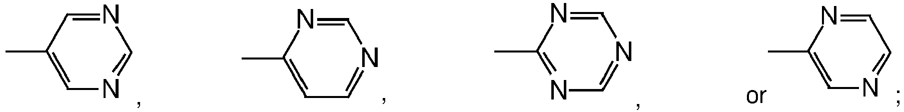
and anomers, deoxy-forms, tautomers, hydrates, and solvates of the above mentioned compounds and pharmaceutically acceptable salts.

4. Use of the compound according to any one of the previous claims as inhibitor of FK506-binding proteins, wherein the inhibition takes place outside the human or animal body.

5. Use of the compound according to claims 1-3 as inhibitor of FKBP12, FKBP12.6, FKBP51, FKBP52, LpMIP, CpMIP, CtMIP BpMIP, and TcMIP, wherein the inhibition takes place outside the human or animal body.

6. Compound according to claims 1-3 for use as pharmaceutically active agent in medicine.

7. Compound according to claims 1-3 for use in the treatment or prophylaxis of psychiatric disorders, neurological disorders, metabolic diseases, cancers, glucocorticoid hyposensitivity syndromes, peripheral glucocorticoid resistance, infectious diseases, alopecia, abnormally elevated intraocular pressure, macular degeneration, oxidative damage to eye tissues, vision disorder, sleeping disorders, asthma, diabetes, traumatic brain injury, nerve injury, Alzheimer's disease, Huntington's disease, Parkinson's disease, ischemia, memory impairment and for neuroprotection, neuroregeneration, promoting hair growth, stimulating neurite growth, wound healing, antiglaucomatous medications, improving vision, enhancing memory performance, for the use in treatment or prevention of multi-drug resistance, for the use in induced abortion and the inhibition of embryo implantation, for the use in limiting or preventing hemorrhage or neovascularization and for the use in treatment of diseases relating to neuro-degeneration.

8. Compound for use according to claim 7, wherein the psychiatric diseases is an affective disorder or an anxiety disorder and/or the metabolic diseases is a localized adiposity, metabolic syndrome or obesity and/or the cancers is prostate cancer, acute lymphoblastic leukaemia or melanoma and/or wherein the affective disorder is selected from the group consisting of: depression, bipolar disorder, mania, substance induced mood disorder and seasonal affective disorder (SAD) and/or wherein the depression is selected from major depression or major depressive disorder and/or wherein the anxiety disorder is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders and/or wherein the infectious disease is selected from the group consisting of Chagas disease, Chlamydia infections, Burkholderia infections, Legionnaires' disease, obesity, diabetes, chronic pain, neuropathic pain, fibromyalgia, hereditary hemorrhagic telangiectasia, pulmonary arterial hypertension, prostate cancer, melanoma, or glioblastoma.

9. Pharmaceutical composition comprising at least one compound according to claims 1-3 together with at least one pharmaceutically acceptable carrier, solvent or excipient or together with at least one pharmaceutically acceptable carrier, solvent or excipient and at least one active agent selected from the group consisting of an anti-depressant and other psychotropic drugs, as well as diabetes drugs, pain drugs and/or antibiotics.

10. Pharmaceutical composition according to claim 9, wherein the anti-depressant is selected from amitriptyline, amioxide clomipramine, doxepine, duloxetine, imipramine trimipramine, mirtazapine, reboxetine, citaloprame, fluoxetine, moclobemide and sertraline.

## Patentansprüche

1. Verbindung der allgemeinen Formel (I): 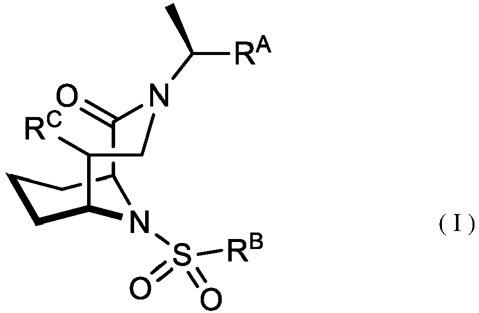
wobei die Methylgruppe in der Position 1 relativ zum Ankerpunkt in der RA-Position in S-Konfiguration vorliegt;
wobei **R^{A}** darstellt: 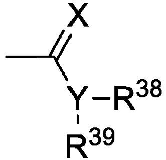
wobei X, Y unabhängig voneinander O, N, S darstellen;
oder wobei **R^{A}** darstellt: -CH₂OR¹⁶, -CH₂NR³⁸R³⁹, 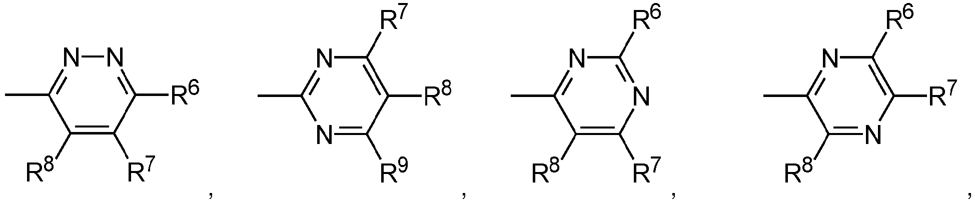 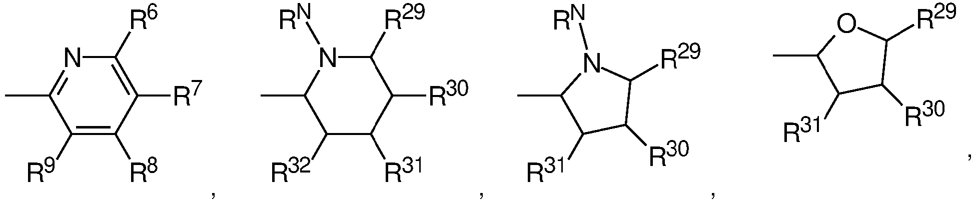 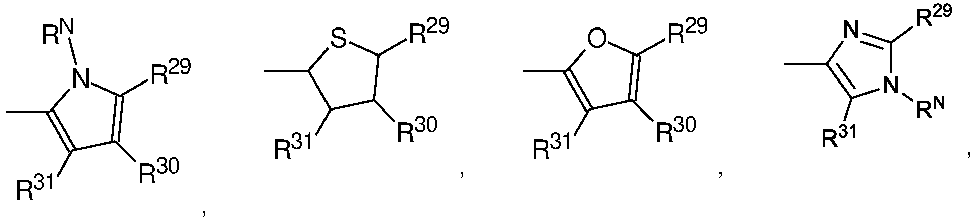 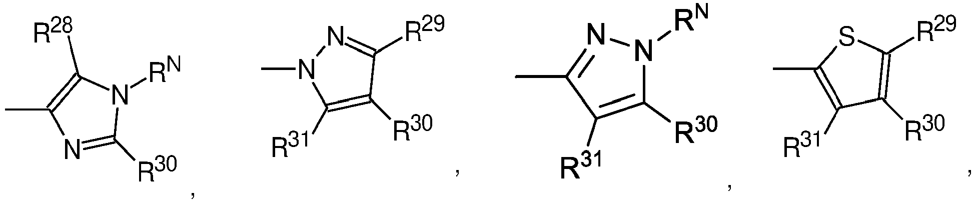 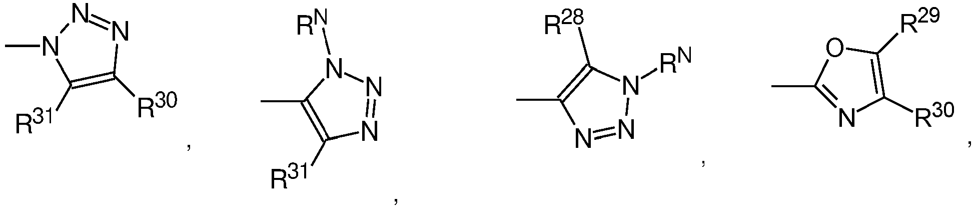 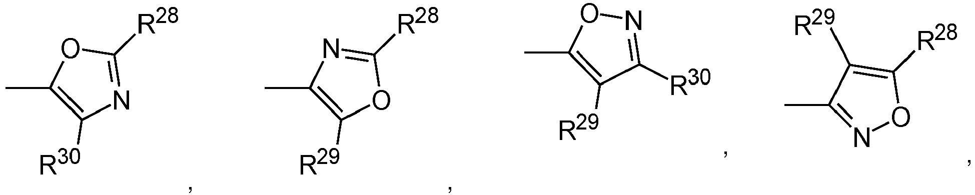 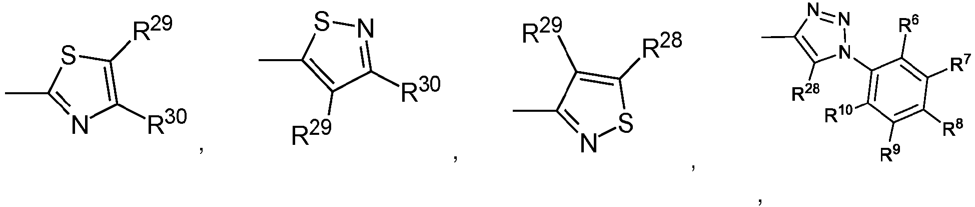 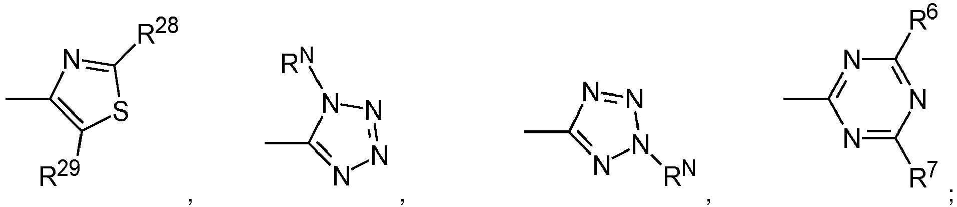 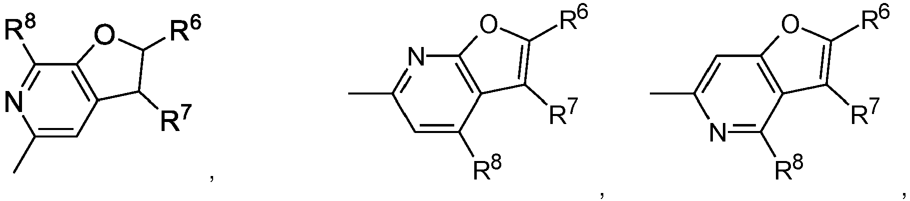 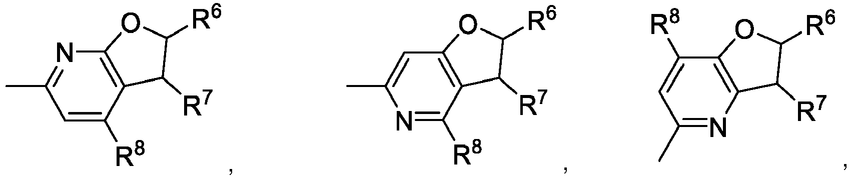 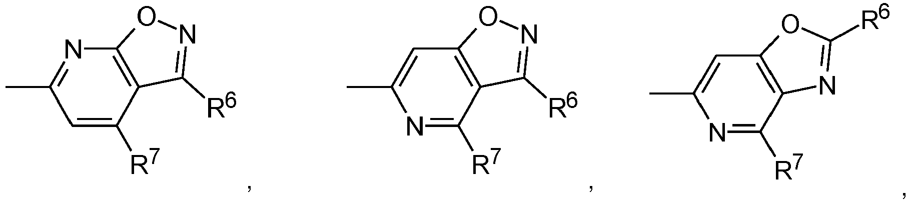 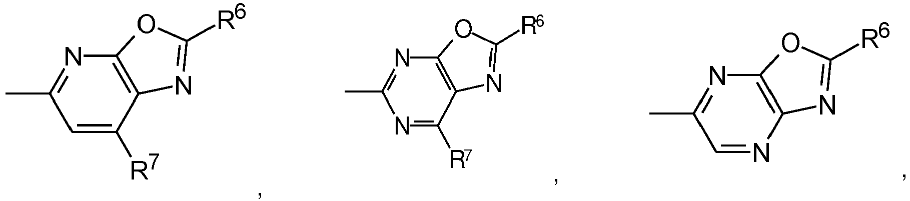 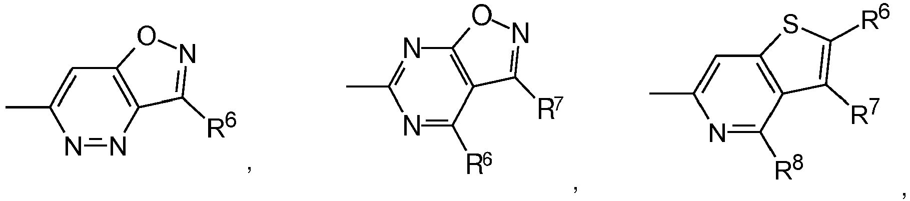 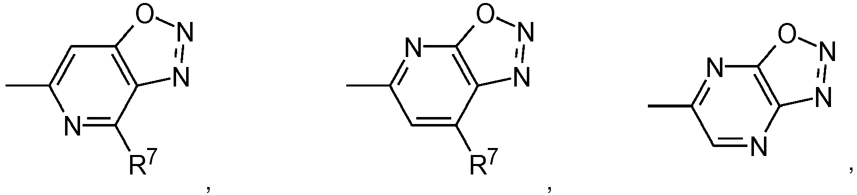 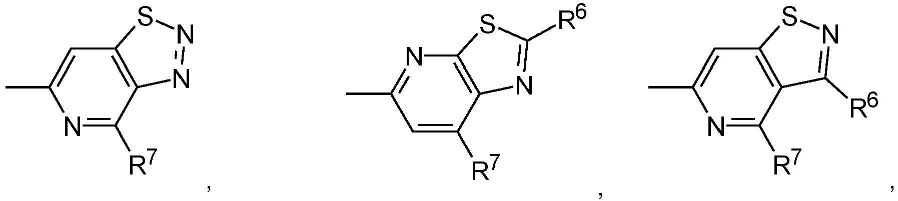 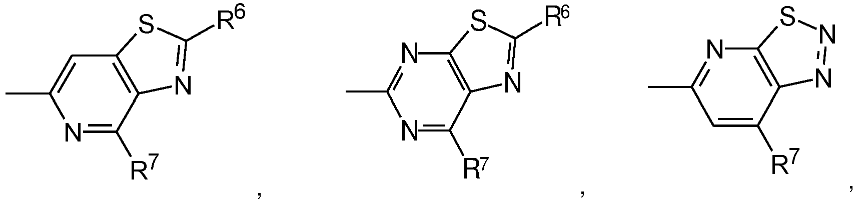 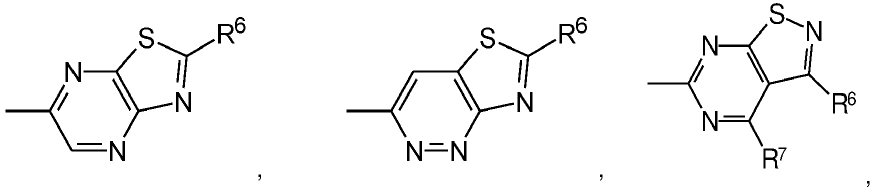 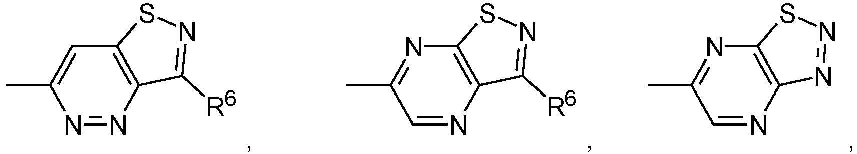 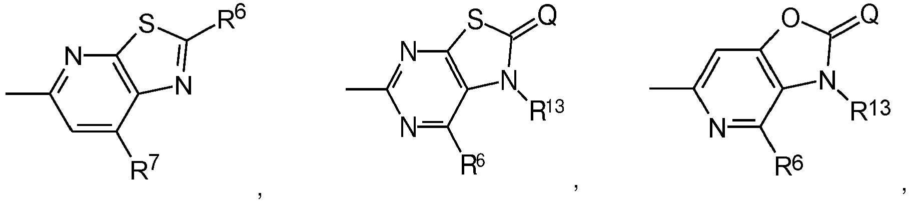 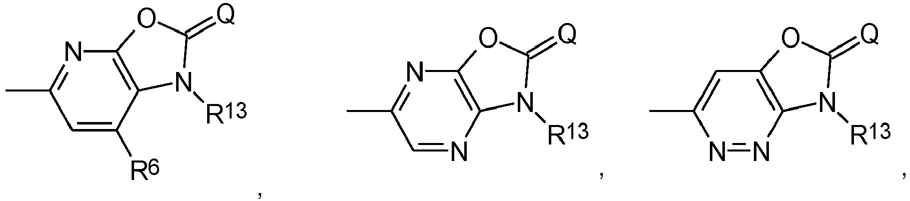 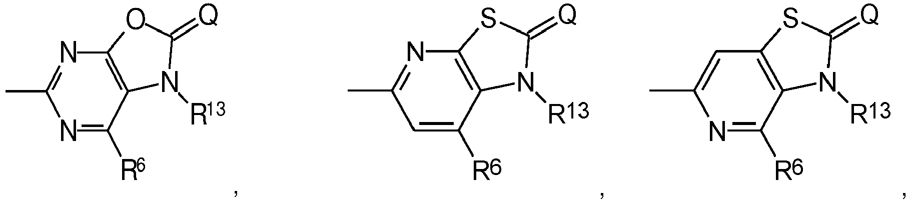 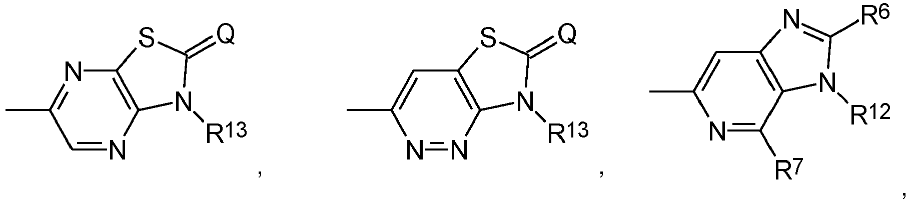 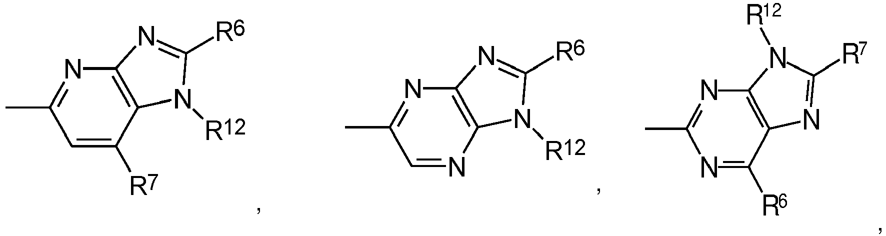 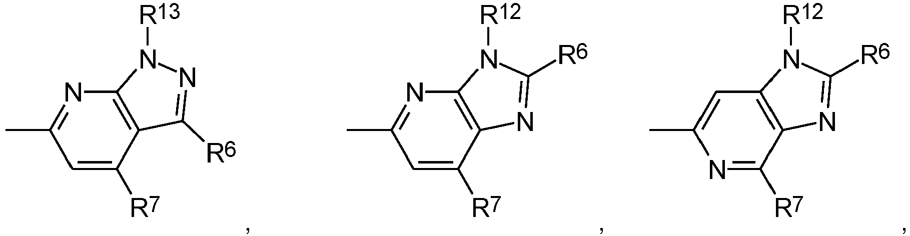 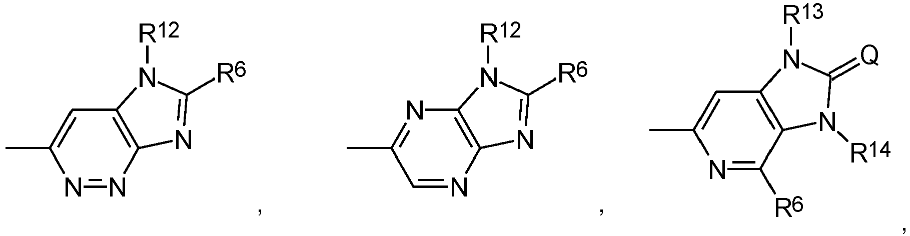 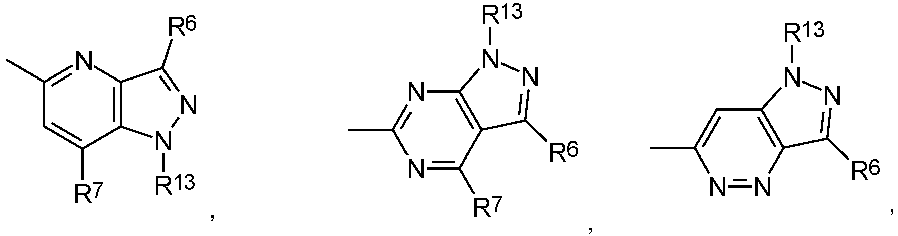 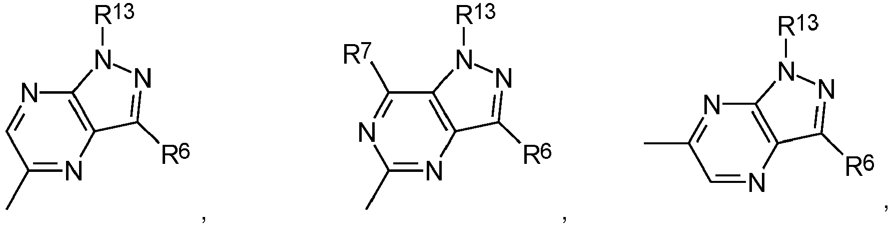 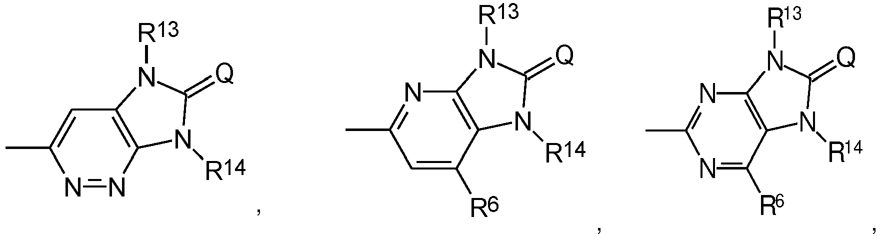 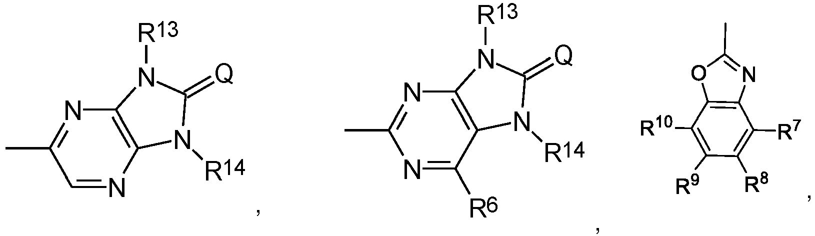 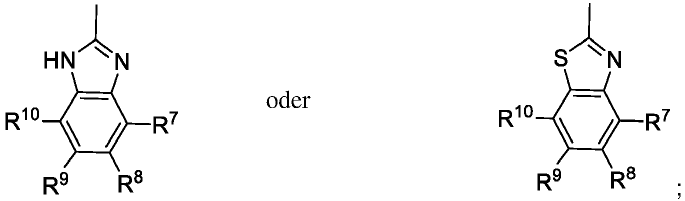
wobei **Q** =O, =S oder =N-R¹² darstellt;
**R^{N}** -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COPh, -COCN, -COOCH₃, -COOC₂H₅, -COOC₃H₇, -COO-cyclo-C₃H₅, -COOCH(CH₃)₂, -COOC(CH₃)₃, -COCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SO₂CH₃, -SO₂C₂H₅, -SO₂CH₂Ph, -SO₂C₃H₇, -SO₂-cyclo-C₃H₅, -SO₂CH(CH₃)₂, -SO₂C(CH₃)₃, -SO₂Ph, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C=C≡CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH₂-C≡C-C₂H₅, -CH=CH-CH=C(CH₃)₂, -CH₂-CH(CH₃)-CH₂-C≡CH, -C₂H₄-C≡C-CH₃, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃ darstellt;
**R^{B}** 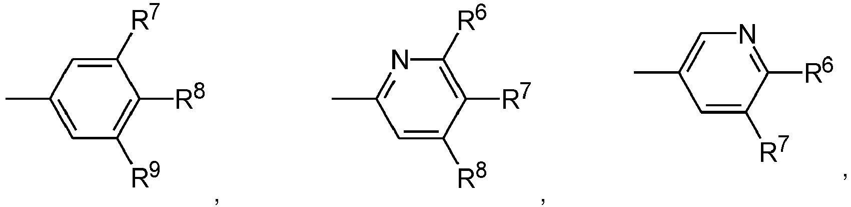 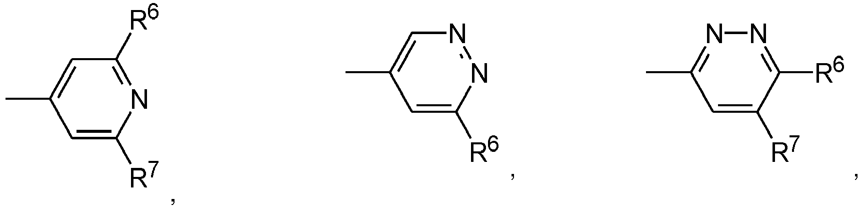 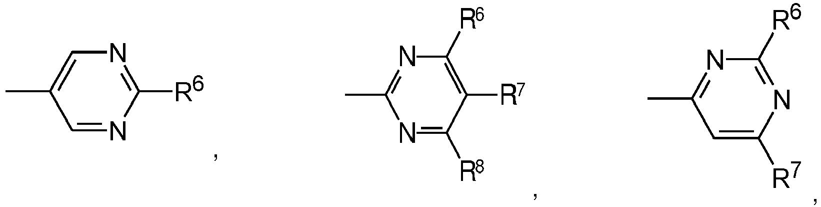 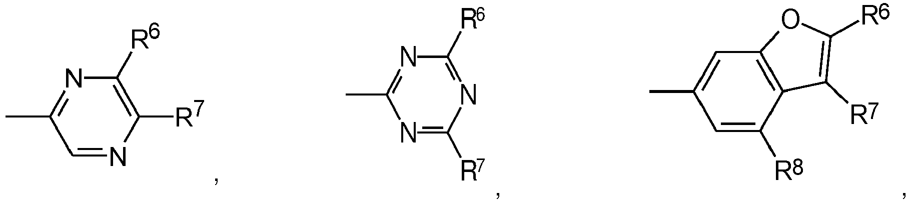 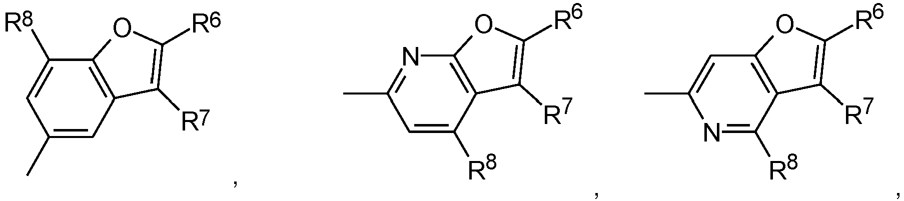 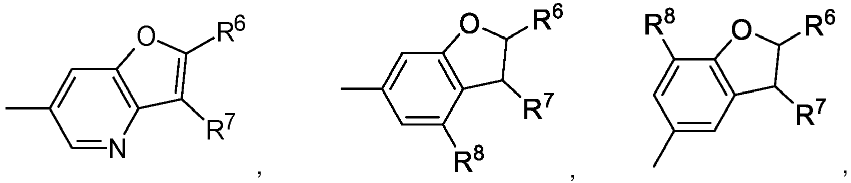 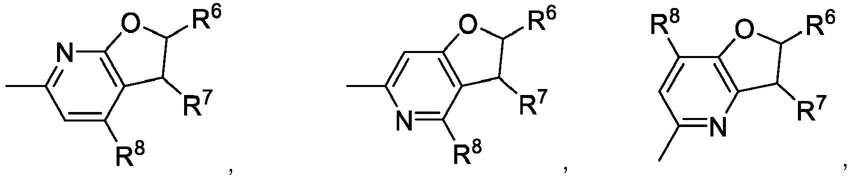 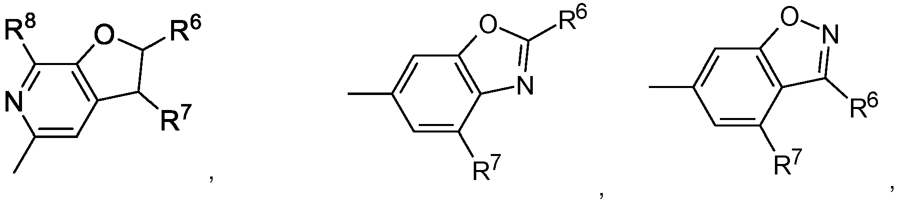 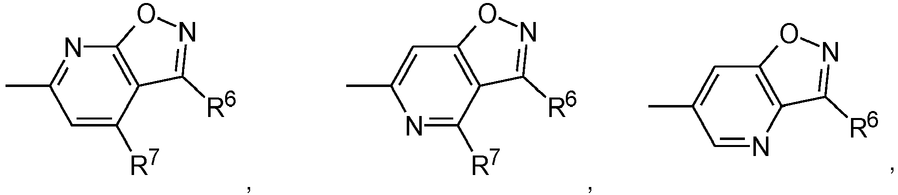 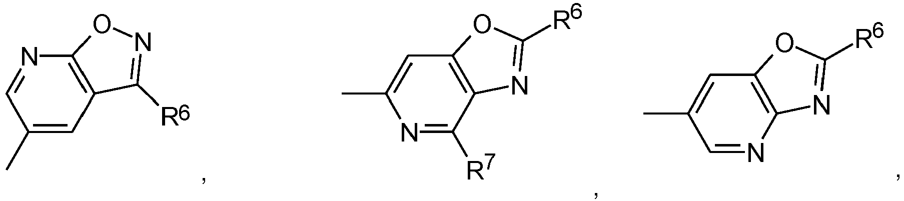 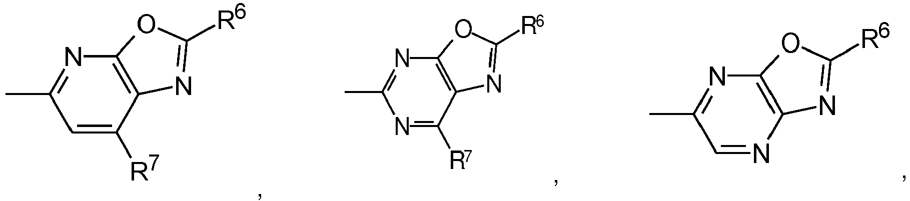 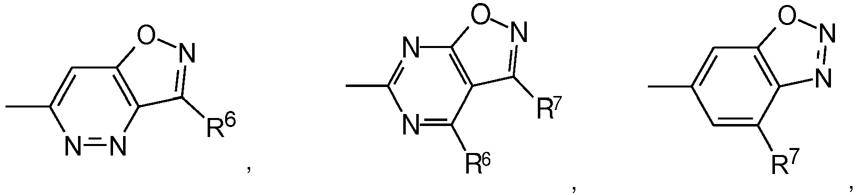 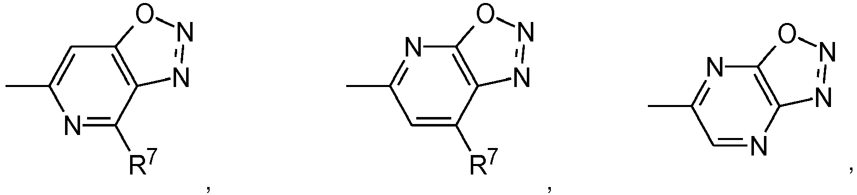 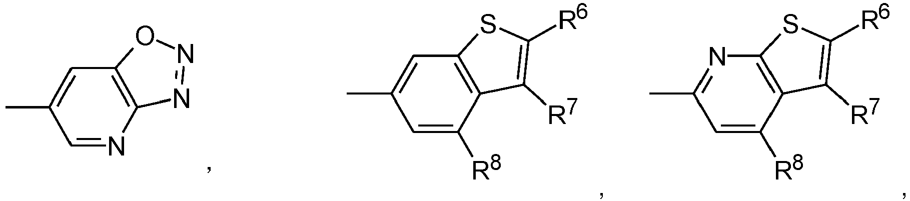 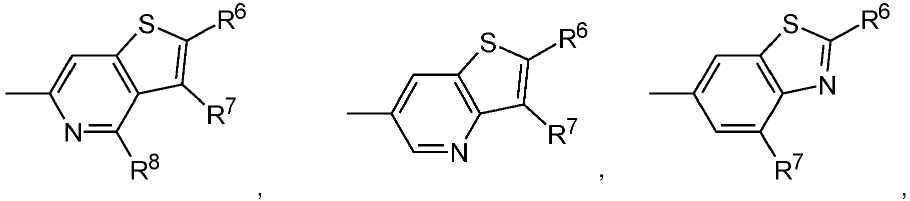 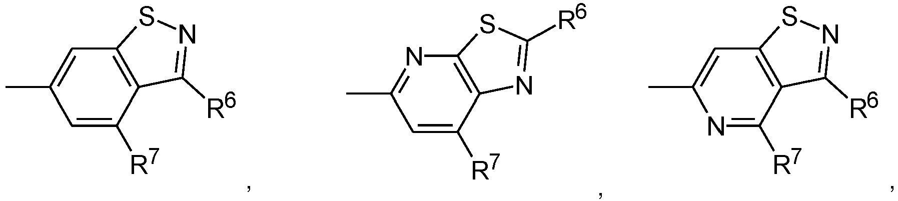 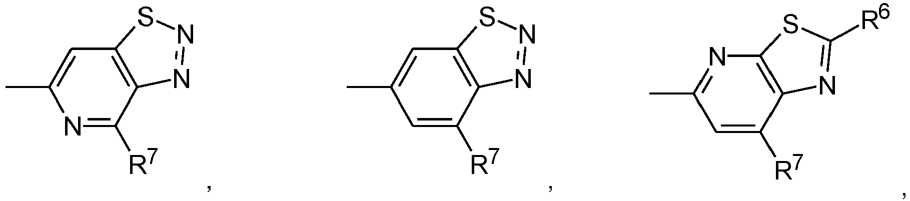 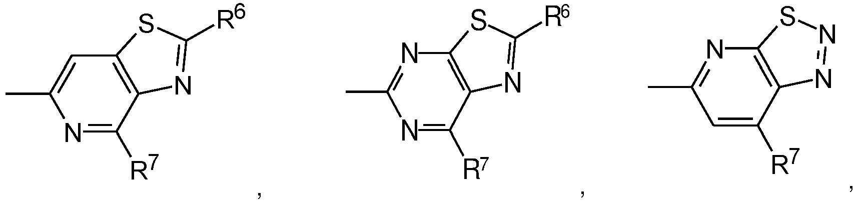 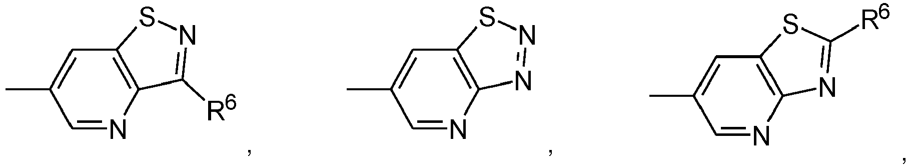 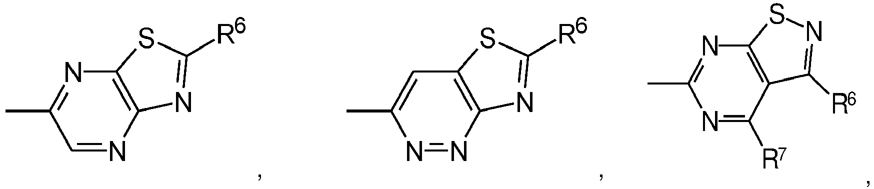 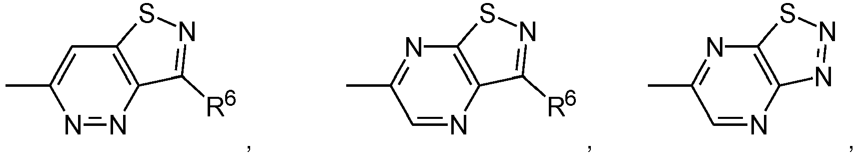 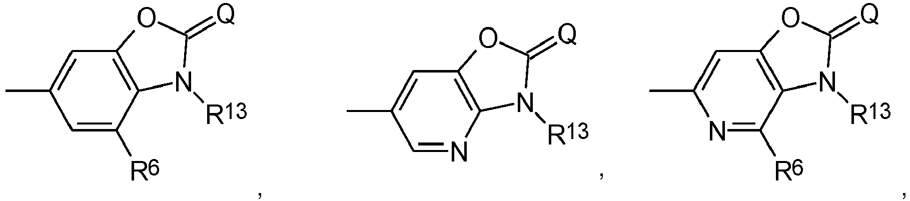 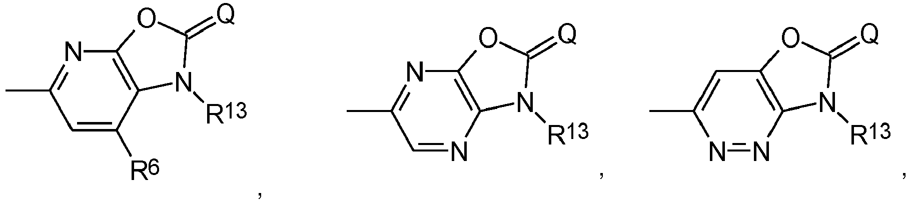 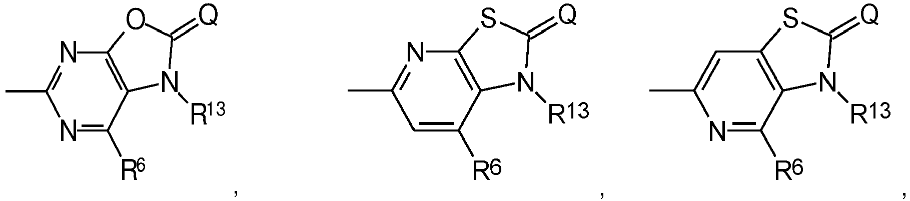 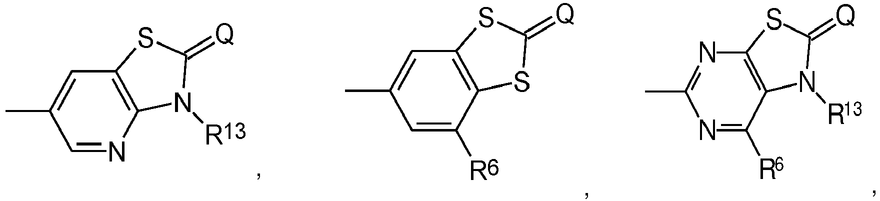 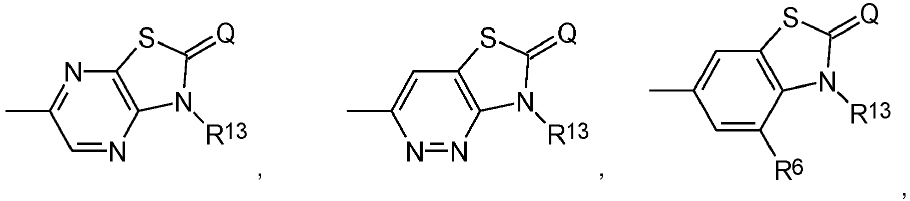 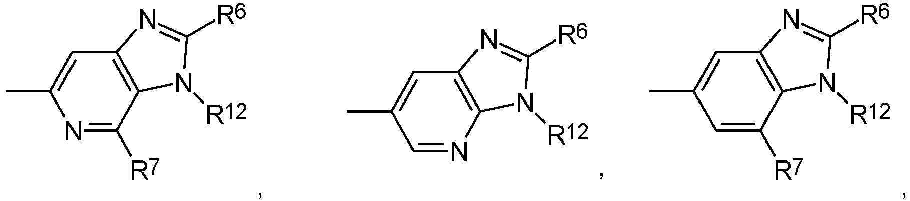 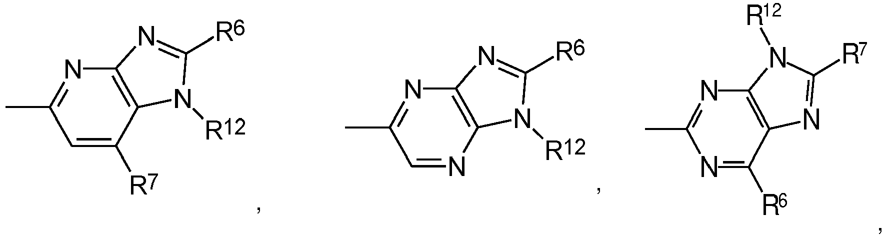 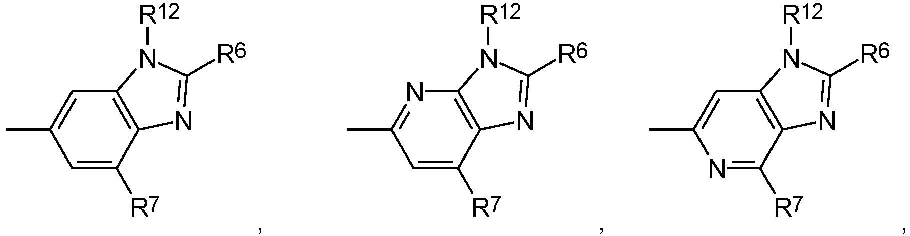 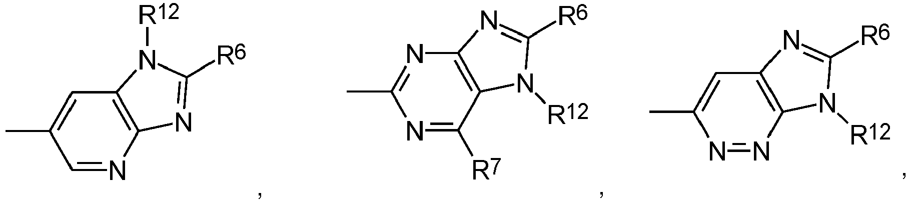 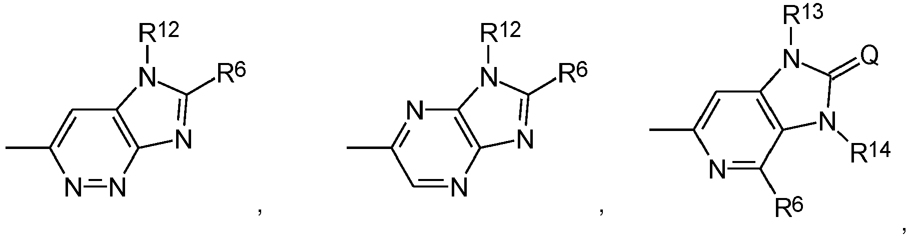 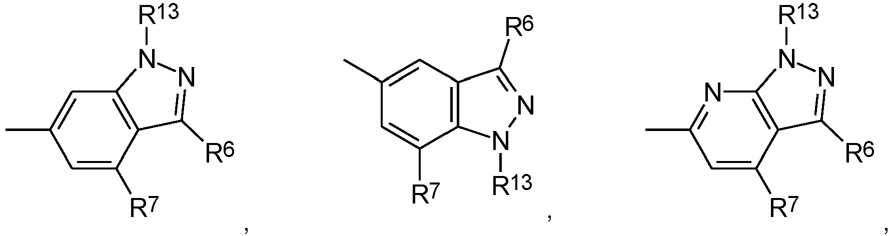 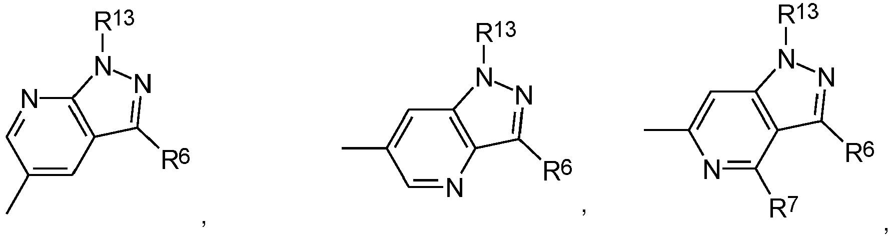 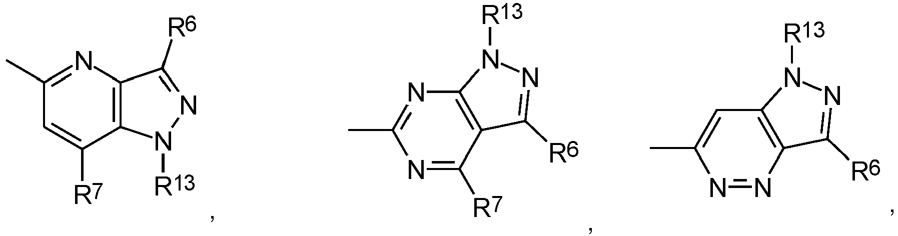 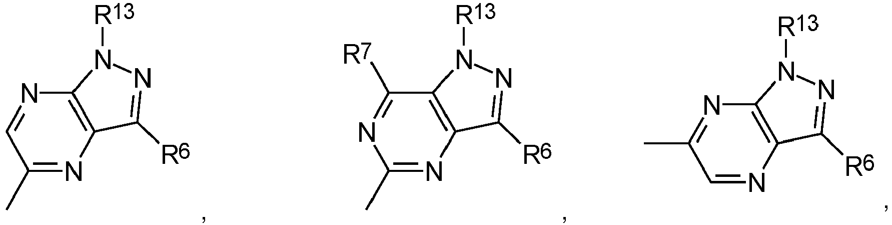 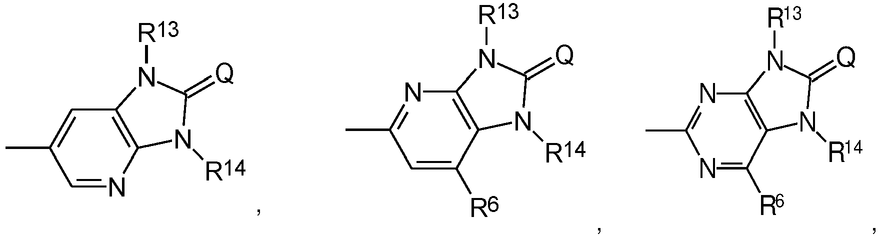 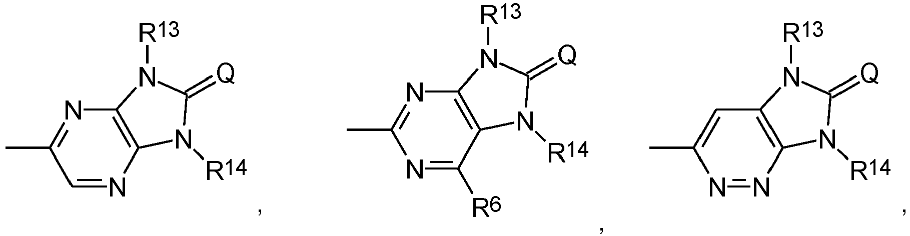 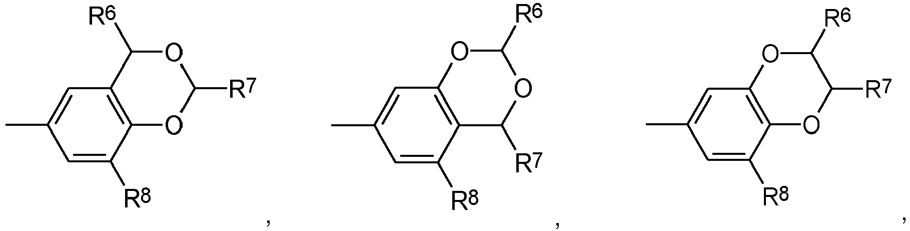 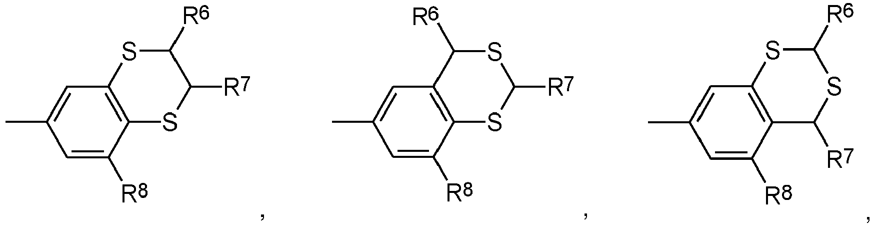 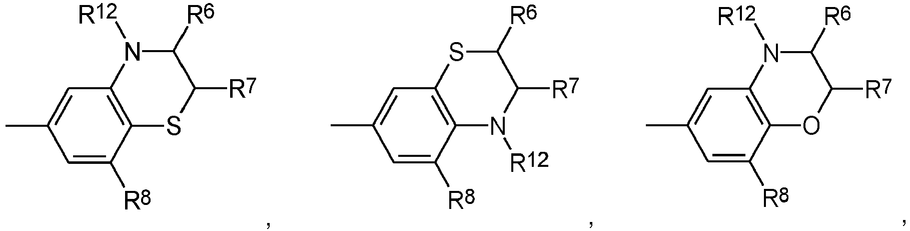 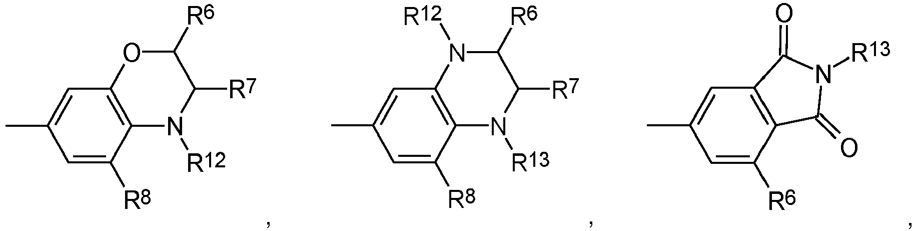 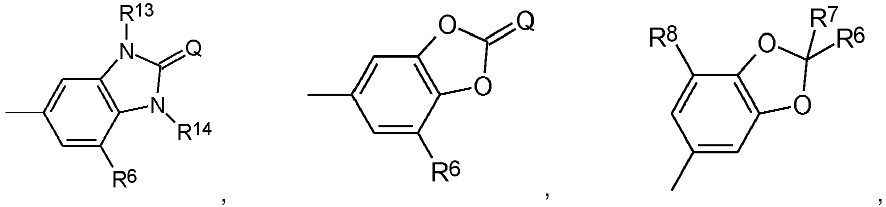 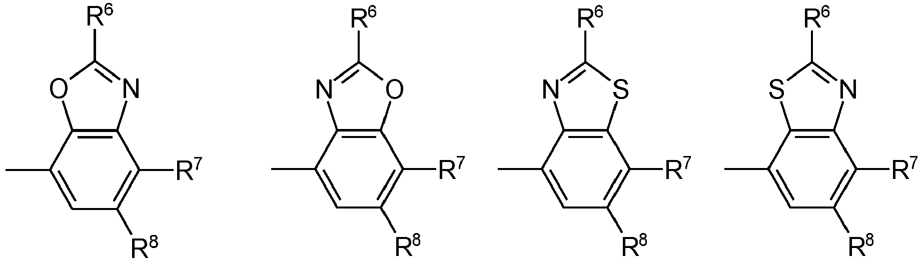 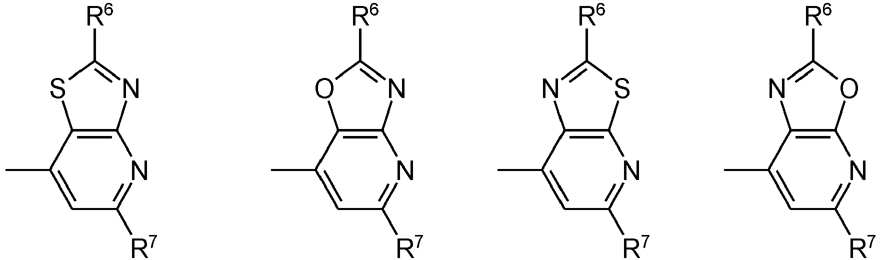 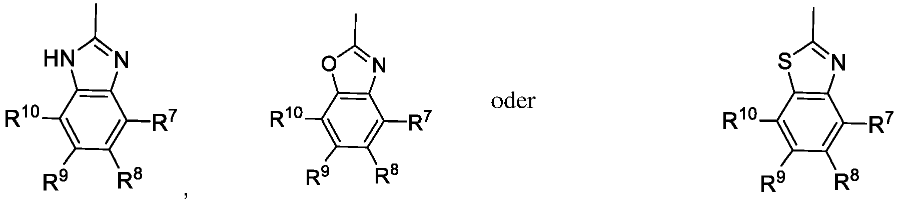
darstellt; wobei **Q** =O, =S oder =N-R¹² darstellt;
**R^{C}** -H,, -OH, -CH₂-OH, -CHO, -CH₂CHO, -CH₂CH₂CHO, -C₂H₄-OH, -C₃H₆-OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -CH(OA_{C})-CH₂OH, -CH(OAc)-CH₂OAc, -CH(OH)-CH₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂H₅)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)CH₂OAc, -CH(NHCH₃)CH₂OAc, -CH(NHC₂H₅)CH₂OAc, -CH(N(CH₃)₂)CH₂OAc, -CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)CH₂OAc, -CH₂-CH(NHCH₃)CH₂OAc, -CH₂-CH(NHC₂H₅)CH₂OAc, -CH₂-CH(N(CH₃)₂)CH₂OAc, -CH₂-CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -NHSO₂CH₃, -NHSO₂CF₃, -NHSO₂CH₂CF₃, -CH₂-NHSO₂CH₃, -CH₂-NHSO₂CF₃, -CH₂-NHSO₂CH₂CF₃, -C₂H₄-NHSO₂CH₃, -C₂H₄-NHSO₂CF₃, -C₂H₄-NHSO₂CH₂CF₃, -CONH₂, -CONHCH₃, -CONHC₂H₅, -CONHC₃H₇, -NH(C₂H₅), -N(C₂H₅)₂, -CH₂-NH(C₂H₅), -CH₂-N(C₂H₅)₂, -C₂H₄-NH(C₂H₅), -C₂H₄-N(C₂H₅)₂, -NO₂, -CH₂-NO₂, -C₂H₄-NO₂, -CH(OH)-NO₂, -CH(NO₂)-OH, -CO₂H, -CH₂-CO₂H, -C₂H₄-CO₂H, -CH=CH-CO₂H, -CO₂CH₃, -CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -O-Si(CH₃)₃, -O-Si(C₂H₅)₃, -CO-CHO, -CO-CO-CH₃, -C(OH)-CO-CH₃, -CO-C(OH)-CH₃, -CO-CH₂-CO-CH₃, -C(OH)-CH₂-CO-CH₃, -CO-CH₂-C(OH)-CH₃, -C(OH)-CH₂-C(OH)-CH₃, -F, -Cl, -Br, -CH₂-F, -CHF₂, -CF₃, -C₂H₄-F, -CH₂-CF₃, -CF₂-CF₃, -O-CHF₂, -O-CF₃, -O-CH₂-CF₃, -O-C₂F₅, -CH₃, -CH₂CH₃, -C₃CH₇, -CH(CH₃)₂, -CH=CH₂, -C≡CH, -CH₂-CH=CH₂ oder -CH₂-C≡CH darstellt;
**R¹-R¹⁰** unabhängig voneinander -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)_{3]}=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡CC₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, 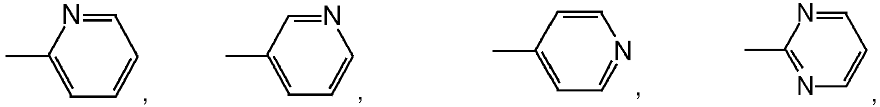 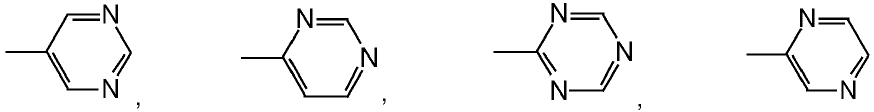
darstellen; **R¹⁵** -**R²⁰**, -CN, -CH₂-CN, -CH₂-OR¹⁷, -CH₂-CH₂-OR¹⁷, -CH₂-NR¹⁷R¹⁸, -CH₂-NR¹⁷COR¹⁹, -CH₂-CH₂-NR¹⁷R¹⁸, -CH₂-CH₂-NR¹⁷COR¹⁹, -CO₂R¹⁷, -CO-NR¹⁷R¹⁸, -CH₂-CO₂R¹⁷ oder -CH₂-CO-NR¹⁷R¹⁸ darstellt;
**R¹⁶, R³⁸, R³⁹** unabhängig voneinander **-R²¹**, -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH₂OH, -CH₂-SH, -CH(OH)CH₃, -C₂H₄OH, -C₃H₆OH, -C₄H₈OH, -CH(CH₃)-C₂H₄OH, -C₅H₁₀OH, -CH₂-S-CH₃, -CH₂-CH₂-S-CH₃, -C₃H₆-S-CH₃, -CH₂OCH₃, -C₂H₄OCH₃, -C₃H₆OCH₃, -C₄H₈OCH₃, -CH(CH₃)-C₂H₄OCH₃, -C₅H₁₀OCH₃, -CH₂NH₂, -C₂H₄NH₂, -C₃H₆NH₂, -C₄H₈NH₂, -CH(CH₃)-C₂H₄NH₂, -C₅H₁₀NH₂, -CH₂-CH₂-CH₂-NH-C(NH)NH₂, -CH₂-CO₂H, -CH₂-CONH₂, -CH₂-CH₂-CO₂H, -CH₂-CH₂-CONH₂, -CH₂-CO₂CH₃, -CH₂-CONHCH₃, -CH₂-CON(CH₃)₂, -CH₂-CH₂-CO₂CH₃, -CH₂-CH₂-CONHCH₃, -CH₂-CH₂-CONH(CH₃)₂, -CH=CH-CO₂H, -CH=CH-CO₂CH₃, -CH=CH-CONHCH₃, -CH=CH-CONHC₂H₅, -CH=CH-CON(CH₃)₂, -CH=CH-CON(C₂H₅)₂, -CH₂-CH=CH-CO₂H, -CH₂-CH=CH-CO₂CH₃, -CH₂-CH=CH-CONHCH₃, -CH₂-CH=CH-CON(CH₃)₂, -CH₂-CH=CH-CONHC₂H₅, -CH₂-CH=CH-CON(C₂H₅)₂, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)_{3]}=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡CC₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-Ph, 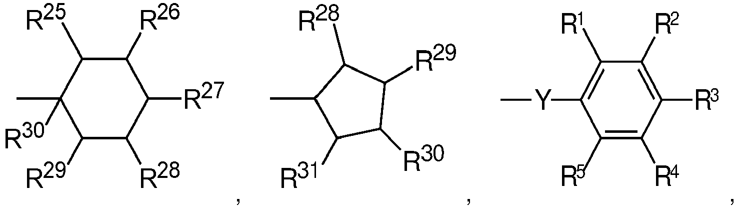 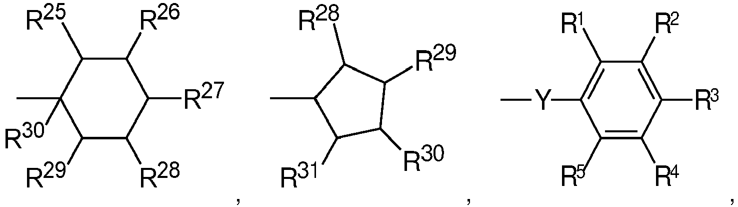 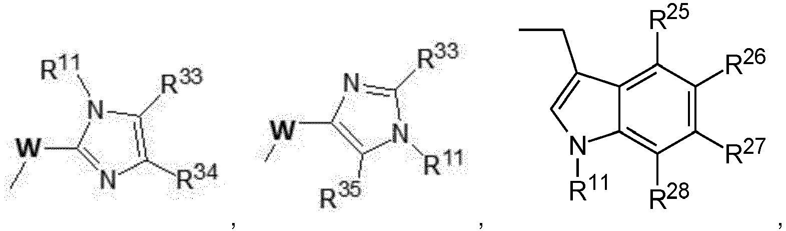 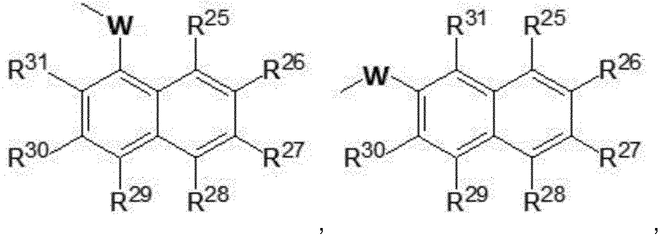 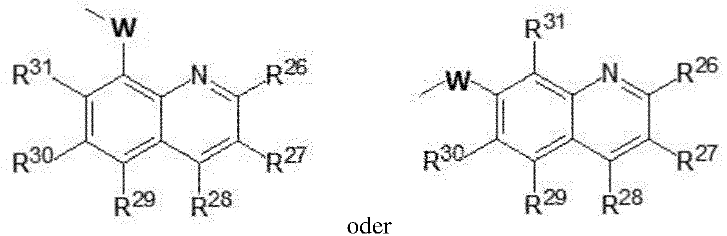
darstellen; wobei W O, N-R¹², S darstellt;
**R¹¹ - R¹⁴** und **R¹⁷ - R²¹** unabhängig voneinander -H, -CH₂F, -CHF₂, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)_{3]}=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡CC₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH oder -CH₂-CH(C≡CH)₂darstellen;
**R²²- R³⁷** unabhängig voneinander -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -S0₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)_{3]}=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 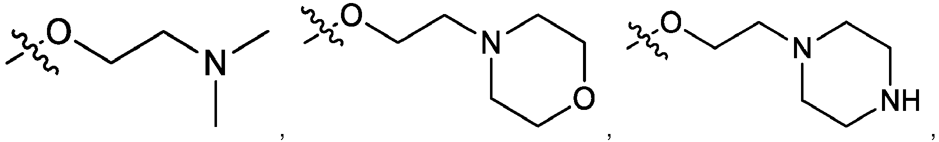 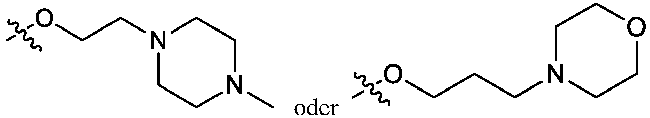 darstellen;
und Anomere, Deoxy-Formen, Tautomere, Hydrate und Solvate von den vorstehend erwähnten Verbindungen und pharmazeutisch verträgliche Salze.

2. Verbindung gemäß Anspruch 1, wobei die Verbindung durch die folgende Formel (V) dargestellt wird: 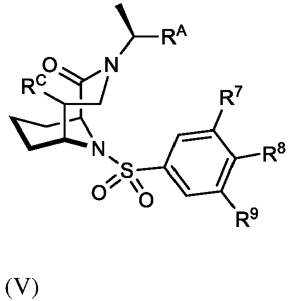
und wobei die Substituenten **R^{A}, R^{B}, R^{C}** und **R⁷** - **R⁹** die Bedeutungen wie für Formel (I) hier definiert haben;
oder wobei die Verbindung durch die folgenden Formeln (VI) oder (VIa) dargestellt wird: 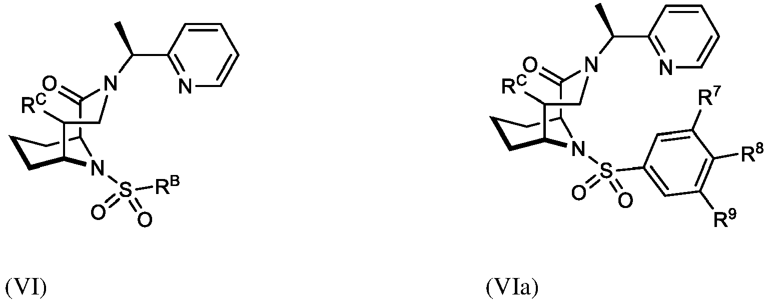
und wobei die Substituenten **R^{B}, R^{C}** und **R⁷** - **R⁹** die Bedeutungen wie für Formel (I) hier definiert haben;
oder wobei die Verbindung durch die folgenden Formeln (VII) oder (VIIa) dargestellt wird: 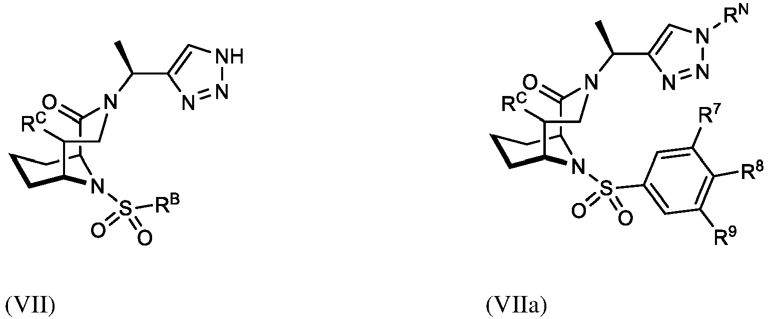
und wobei die Substituenten **R^{B}, R^{C}, R^{N}** und **R⁷** - **R⁹** die Bedeutungen wie für Formel (I) hier definiert haben;
oder wobei die Verbindung durch die folgende Formel (VIII) oder (VIIIa) dargestellt wird: 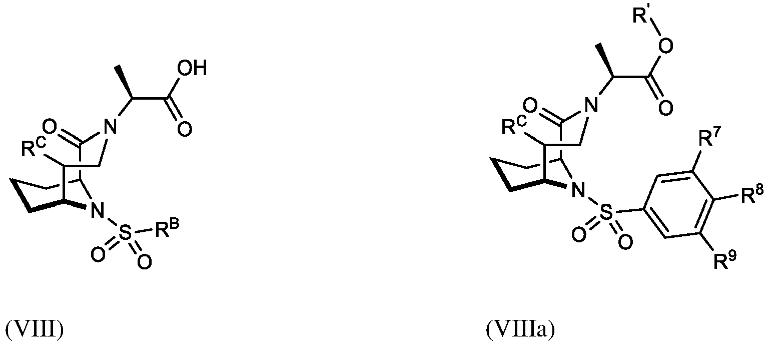
und wobei die Substituenten **R^{B}, R^{C}, R^{N}, R^{'}** und **R⁷** - **R⁹** die Bedeutungen wie für Formel (I) hier definiert haben;
oder wobei die Verbindung durch die folgende Formel (IX) oder (X) dargestellt wird: 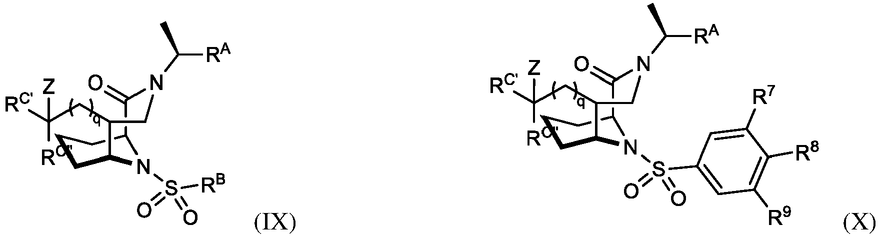
und wobei die Substituenten **R^{A}**, **R^{B}** und **R⁷** - **R⁹** die Bedeutungen wie für Formel (I) hier definiert haben;
R^{C'} und R^{C"} unabhängig voneinander -H, -CH₃, -C₂H₅, -CH₂-OH darstellen;
Z -OH, -OCH₃, -OC₂H₅, -OCH(CH₃)₂, -NH₂, -NH(CH₃), -NH(C₂H₅), -NHCH(CH₃)₂, -N(CH₃)₂ oder -N(C₂H₅)₂ darstellt; und q 0 oder 1 ist;
und Anomere, Deoxy-Formen, Tautomere, Hydrate und Solvate von den vorstehend erwähnten Verbindungen und pharmazeutisch verträgliche Salze.

3. Verbindung gemäß einem der vorhergehenden Ansprüche, wobei die Verbindung durch die folgende Formel (V) dargestellt wird: 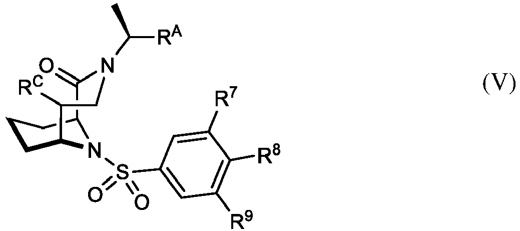
wobei **R^{A}** ausgewählt ist aus: 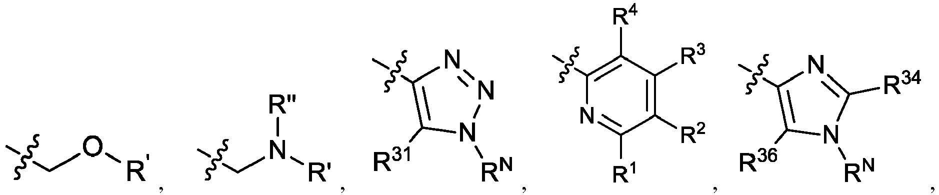 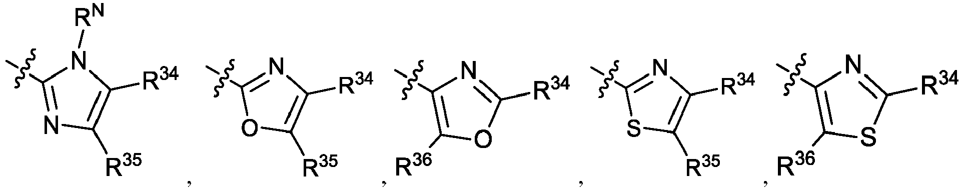 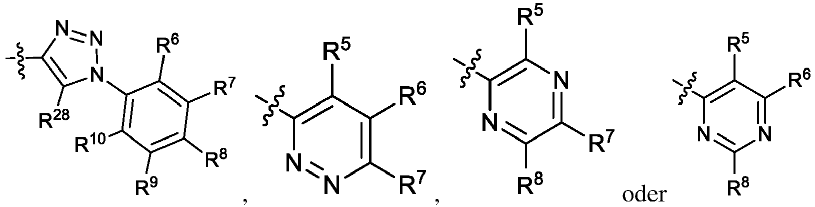
**R^{C}** die Bedeutung wie für Formel (I) hier definiert hat;
**R⁷** - **R⁹** unabhängig voneinander ausgewählt sind aus: -H, -F, -Cl, -Br, -I, -OH, -OCH₃, -OC₂H₅, -OC₃H₇, -CN, -CONH₂, -NHCOCH₃, -NHCOC₂H₅, -NHCOC₃H₇, -COOH, -COOCH₃, -COOC₂H₅, -COOC₃H₇, -Ph, 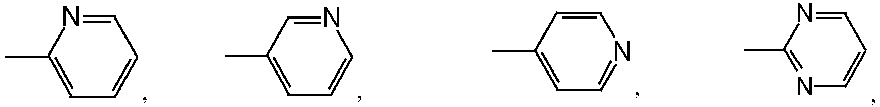 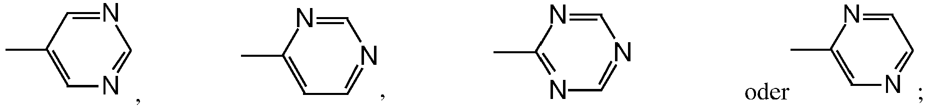
und Anomere, Deoxy-Formen, Tautomere, Hydrate und Solvate von den vorstehend erwähnten Verbindungen und pharmazeutisch verträgliche Salze.

4. Verwendung der Verbindung gemäß einem der vorhergehenden Ansprüche als Inhibitor von FK506-bindenden Proteinen, wobei die Inhibierung außerhalb des menschlichen oder tierischen Körpers stattfindet.

5. Verwendung der Verbindung gemäß den Ansprüchen 1-3 als Inhibitor von FKBP12, FKBP12.6, FKBP51, FKBP52, LpMIP, CpMIP, CtMIP BpMIP und TcMIP, wobei die Inhibierung außerhalb des menschlichen oder tierischen Körpers stattfindet.

6. Verbindung gemäß den Ansprüchen 1-3 zur Verwendung als pharmazeutischer Wirkstoff in Medizin.

7. Verbindung gemäß den Ansprüchen 1-3 zur Verwendung in der Behandlung oder Prophylaxe von psychiatrischen Störungen, neurologischen Störungen, metabolischen Erkrankungen, Krebs, Glucocorticoid-Hyposensibilitätssyndromen, peripherer Glucocorticoid-Resistenz, infektiösen Erkrankungen, Alopezie, anomal erhöhtem intraokulärem Druck, Makuladegeneration, oxidativem Schaden von Augengeweben, Sehstörung, Schlafstörungen, Asthma, Diabetes, traumatischer Hirnverletzung, Nervenverletzung, Alzheimer-Krankheit, Morbus Huntington, Parkinson-Krankheit, Ischämie, Gedächtnisbeeinträchtigung und für Neuroprotektion, Neuroregeneration, Fördern von Haarwachstum, Stimulieren von Neuritwachstum, Wundheilung, Antiglaukom-Medikationen, Verbessern von Sehen, Steigern von Gedächtnisleistung, zur Verwendung in der Behandlung oder Vorbeugung von Mehrfachresistenz gegen Arzneimittel, zur Verwendung bei Schwangerschaftsabbruch und der Inhibierung von Embryoneneinnistung, zur Verwendung beim Einschränken oder Vorbeugen von Blutung oder Neovaskularisation und zur Verwendung in der Behandlung von mit Neurodegeneration in Zusammenhang stehenden Erkrankungen.

8. Verbindung zur Verwendung gemäß Anspruch 7, wobei die psychiatrischen Erkrankungen eine affektive Störung oder eine Angststörung ist, und/oder die metabolischen Erkrankungen lokalisierte Adipositas, metabolisches Syndrom oder Fettleibigkeit ist, und/oder der Krebs Prostatakrebs, akute lymphoblastische Leukämie oder Melanom ist, und/oder wobei die affektive Störung aus der Gruppe ausgewählt ist, welche besteht aus: Depression, bipolarer Störung, Manie, substanzinduzierter Gemütsstörung und jahreszeitlich-bedingter affektiver Störung (SAD), und/oder wobei die Depression aus schwerer Depression oder schwerer depressiver Störung ausgewählt ist, und/oder wobei die Angststörung aus der Gruppe ausgewählt ist, welche umfasst oder besteht aus generalisierter Angststörung, Panikstörung, Panikstörung mit Agoraphobie, Phobien, zwanghafter Verhaltensstörung, posttraumatischer Belastungsstörung, Trennungsangst und Angststörungen der Kindheit, und/oder wobei die infektiöse Erkrankung aus der Gruppe ausgewählt ist, welche aus Chagas-Krankheit, Chlamydien-Infektionen, Burkholderia-Infektionen, Legionärskrankheit, Fettleibigkeit, Diabetes, chronischem Schmerz, neuropathischem Schmerz, Fibromyalgie, hereditärer hämorrhagischer Teleangiektasie, pulmonal-arterieller Hypertonie, Prostatakrebs, Melanom oder Glioblastom besteht.

9. Pharmazeutische Zusammensetzung umfassend mindestens eine Verbindung gemäß den Ansprüchen 1-3 zusammen mit mindestens einem pharmazeutisch verträglichen Träger, Lösungsmittel oder Exzipienten oder zusammen mit mindestens einem pharmazeutisch verträglichen Träger, Lösungsmittel oder Exzipienten und mindestens einem Wirkstoff ausgewählt aus der Gruppe bestehend aus einem Antidepressivum und anderen psychotropen Arzneistoffen, sowie Diabetes-Arzneistoffen, Schmerz-Arzneistoffen und/oder Antibiotika.

10. Pharmazeutische Zusammensetzung gemäß Anspruch 9, wobei das Antidepressivum aus Amitriptylin, Amioxid Clomipramin, Doxepin, Duloxetin, Imipramin Trimipramin, Mirtazapin, Reboxetin, Citalopram, Fluoxetin, Moclobemid und Sertralin ausgewählt ist.

## Revendications

1. Composé de formule générale (!) : 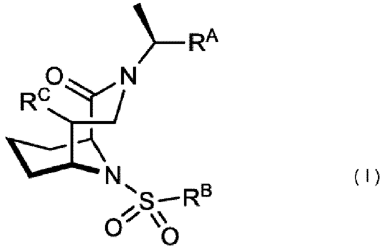
dans lequel le groupe méthyle en position 1 par rapport au point d'ancrage dans la position RA est en configuration S ;
dans lequel **R^{A}** représente : 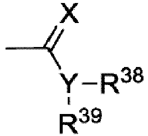
dans lequel **X, Y** représentent indépendamment l'un de l'autre O, N, S ;
ou dans lequel **R^{A}** représente : -CH₂OR¹⁶, -CH₂NR³⁸R³⁹, 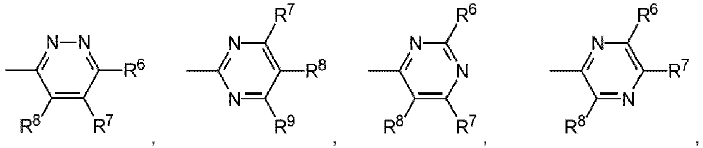 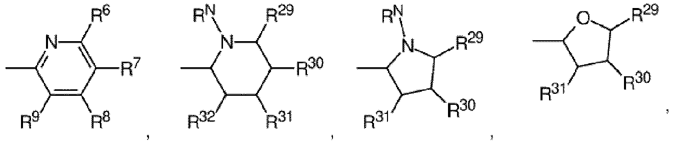 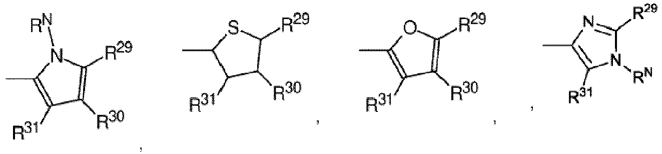 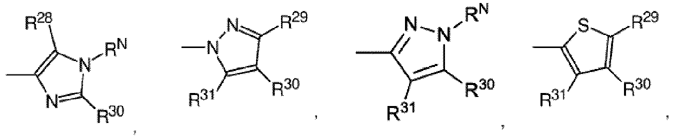 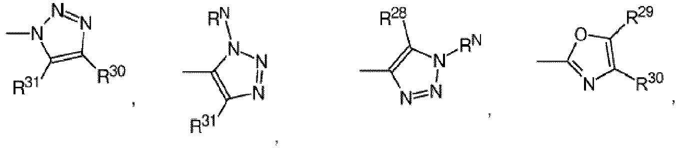 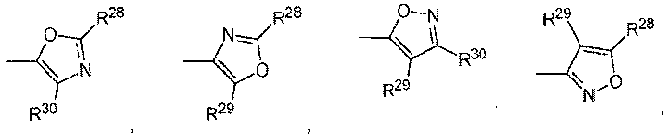 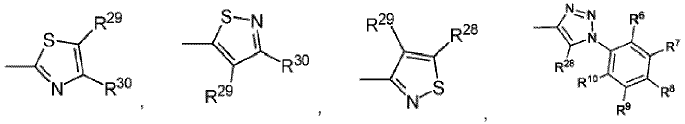 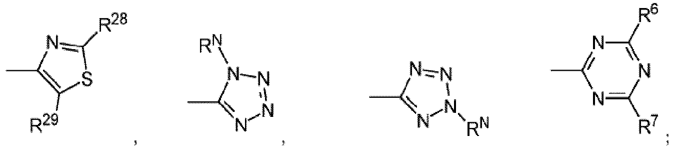 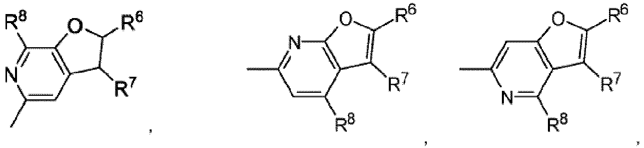 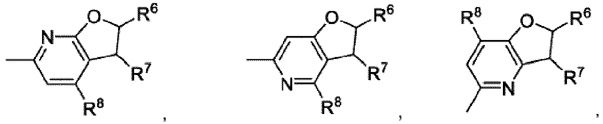 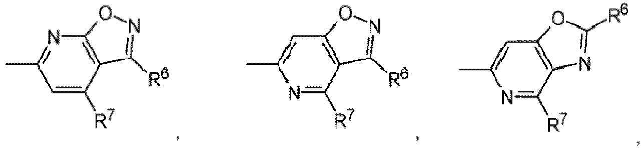 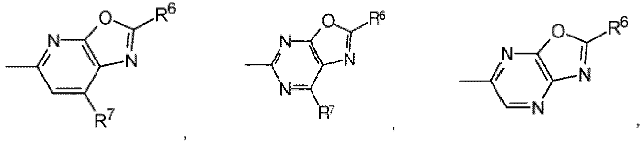 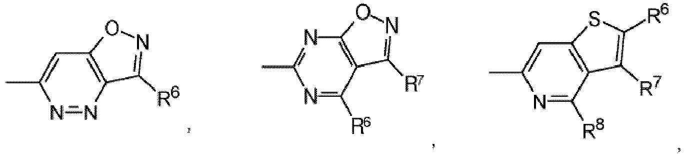 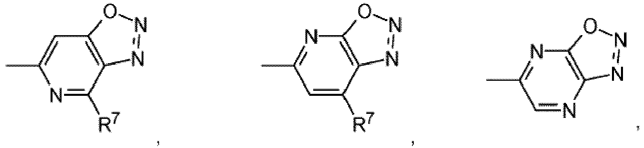 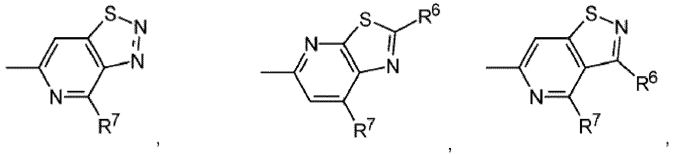 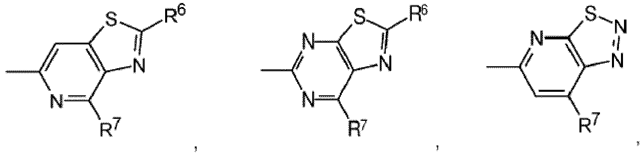 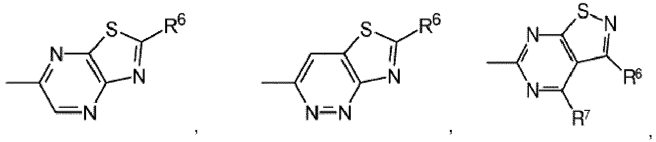 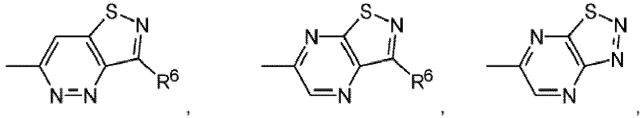 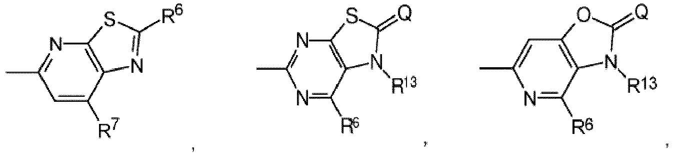 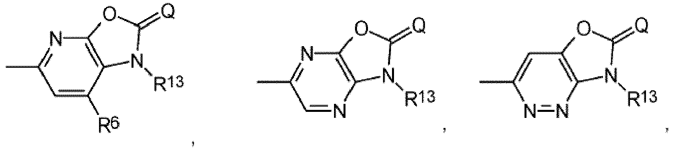 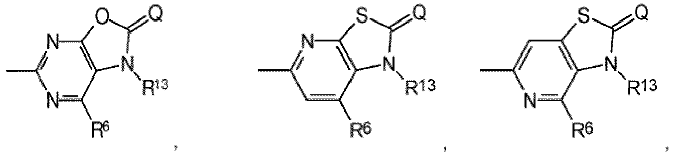 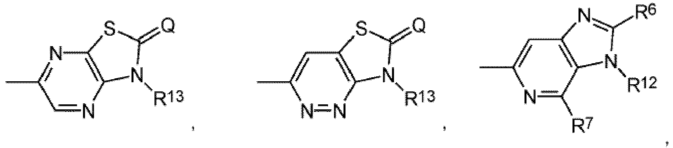 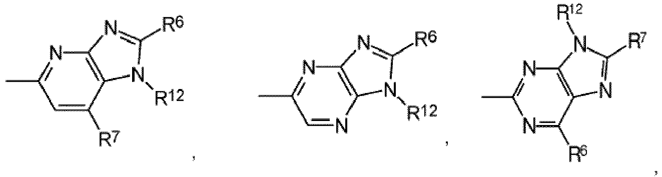 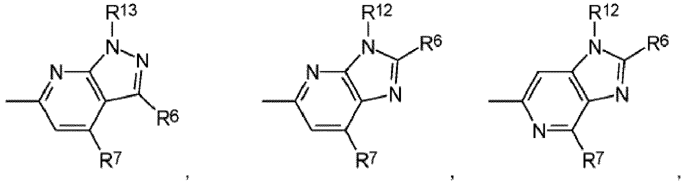 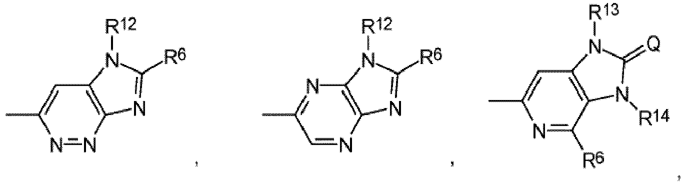 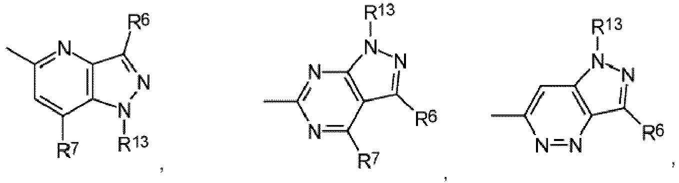 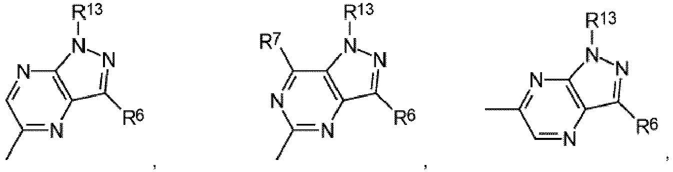 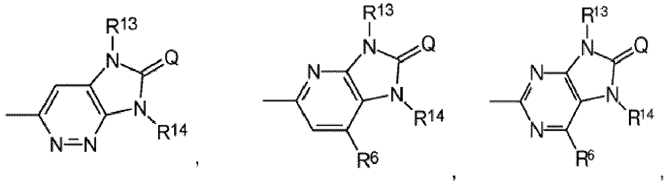 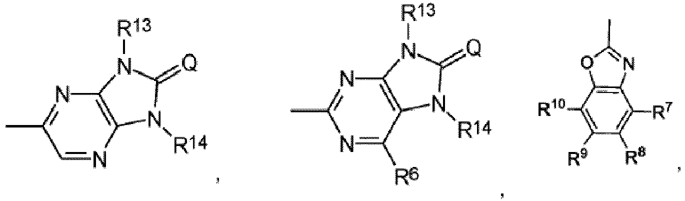 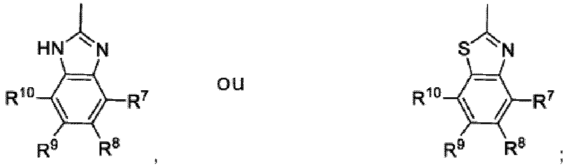
dans lequel Q représente =O, =S, ou =N-R¹² ;
**R^{N}** représente -H, -CH₂-OCH₃, -C₂H₄-OCH₃, -C₃H₆-OCH₃, -CH₂-OC₂H₅, -C₂H₄-OC₂H₅, - C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, - C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, - COC₃H₇, -CO-cyclo-C₃H₅, -COCH(CH₃)₂, -COC(CH₃)₃, -COPh, -COCN, -COOCH₃, -COOC₂H₅, - COOC₃H₇, -COO-cyclo-C₃H₅, -COOCH(CH₃)₂, -COOC(CH₃)₃, -COCH₂Ph, -CONH₂, -CONHCH₃, - CONHC₂H₅, -CONHC₃H₇, -CONH-cyclo-C₃H₅, -CONH[CH(CH₃)₂], -CONH[C(CH₃)₃], - CON(CH₃)₂, -CON(C₂H₅)₂, -CON(C₃H₇)₂, -CON(cyclo-C₃H₅)₂, -CON[CH(CH₃)₂]₂, - CON[C(CH₃)₃]₂, -SO₂CH₃, -SO₂C₂H₅, -SO₂CH₂Ph, -SO₂C₃H₇, -SO₂-cyclo-C₃H₅, -SO₂CH(CH₃)₂, - SO₂C(CH₃)₃, -SO₂Ph, -CH₂-OCF₃, -C₂H₄-OCF₃, -C₃H₆-OCF₃, -OC₂F₅, -CH₂-OC₂F₅, -C₂H₄-OC₂F₅, - C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, - CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, - CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, - CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, - CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, - CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, - CH₂-CH=CH-CH=CH₂, -CH=CH-CH=CH-CH₃, -CH=CH-CH₂-CH=CH₂, -C(CH₃)=CH-CH=CH₂, - CH=C(CH₃)-CH=CH₂, -CH=CH-C(CH₃)=CH₂, -C₂H₄-C(CH₃)=CH₂, -CH₂-CH(CH₃)-CH=CH₂, - CH(CH₃)-CH₂-CH=CH₂, -CH₂-CH=C(CH₃)₂, -CH₂-C(CH₃)=CH-CH₃, -CH(CH₃)-CH=CH-CH₃, - CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, - CH(CH₃)-C(CH₃)=CH₂, -C(CH₃)=CH-CH=CH₂, -CH=C(CH₃)-CH=CH₂, -CH=CH-C(CH₃)=CH₂, - C₄H₈-CH=CH₂, -C₃H₆-CH=CH-CH₃, -C₂H₄-CH=CH-C₂H₅, -CH₂-CH=CH-C₃H₇, -CH=CH-C₄H₉, - C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, - CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, - CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, - C(C₄H₉)=CH₂, -C(C₃H₇)=CH-CH₃, -C(C₂H₅)=CH-C₂H₅, -C(C₂H₅)=C(CH₃)₂, - C[CH(CH₃)(C₂H₅)]=CH₂, -C[CH₂-CH(CH₃)_{2]}=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C=CH, - CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C=CH, -C(CH₃)=CH-CH₂-CH=CH₂, -CH₂-C=C-C₂H₅, -CH=CH-CH=C(CH₃)₂, -CH₂-CH(CH₃)-CH₂-C≡CH, -C₂H₄-C≡C-CH₃, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, - CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C=CH, -C≡C-CH₃, -CH₂-C≡CH, -C₂H₄-C≡CH, -CH₂-C≡C-CH₃, -C≡C-C₂H₅, -C₃H₆-C≡CH, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C=C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡'C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, - CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, - CH(C≡CH)-C≡C-CH₃ ;
**R^{B}** représente 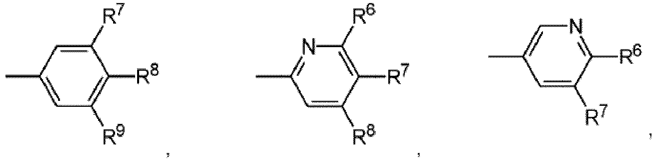 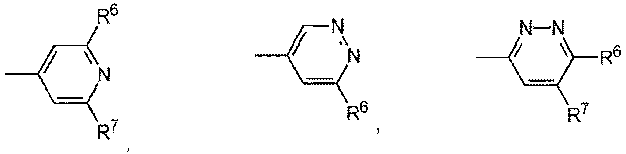 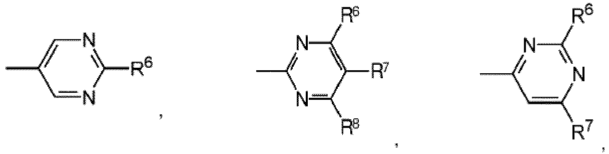 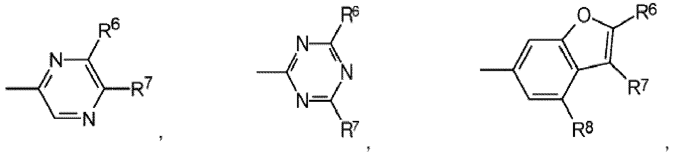 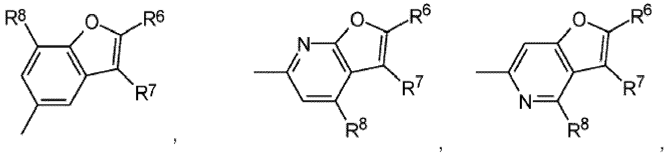 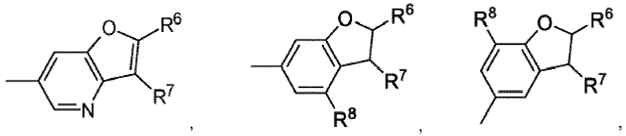 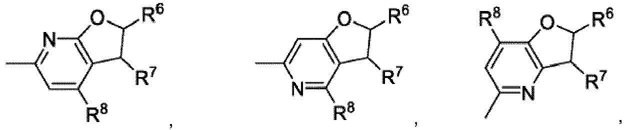 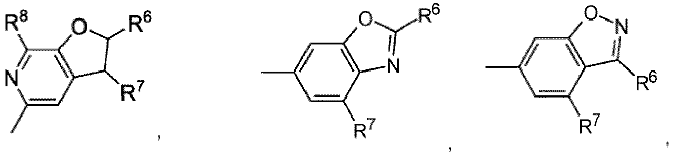 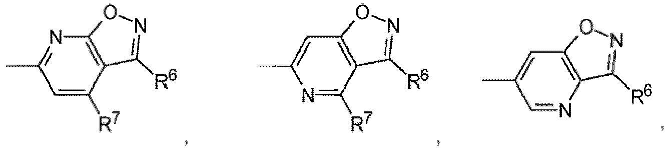 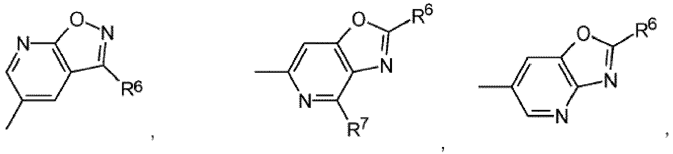 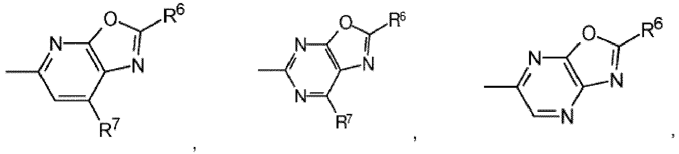 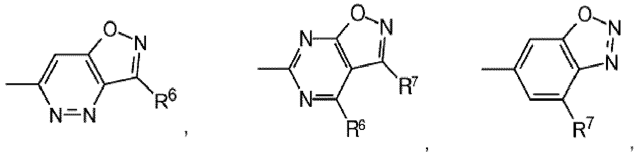 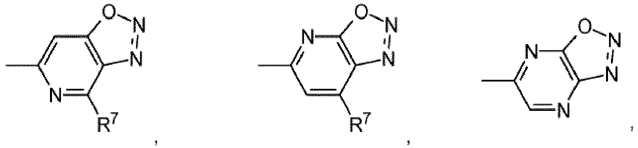 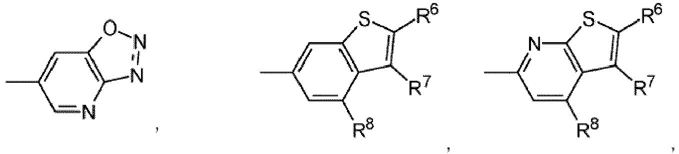 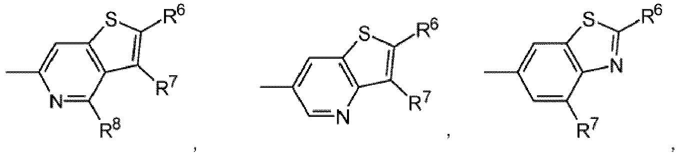 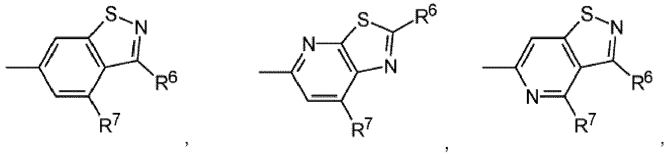 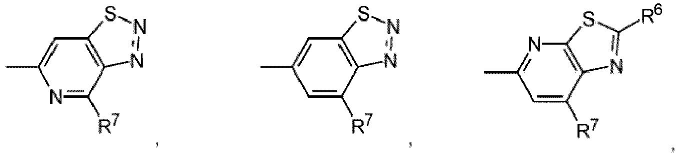 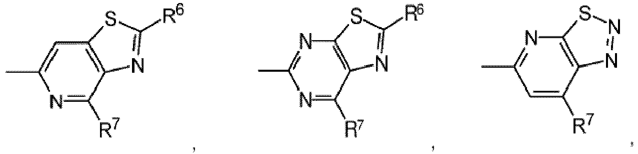 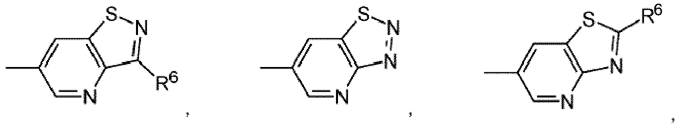 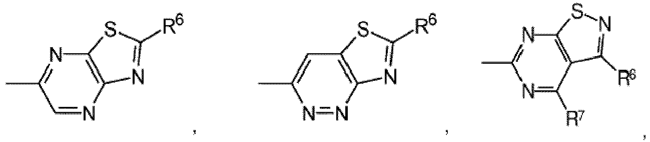 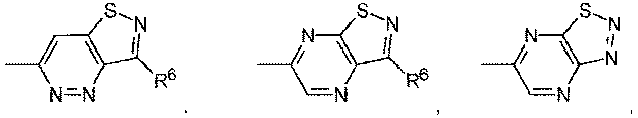 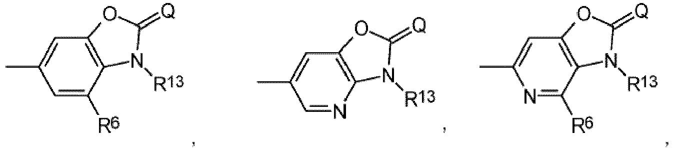 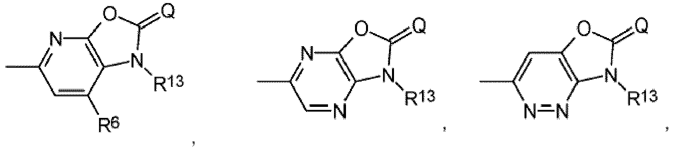 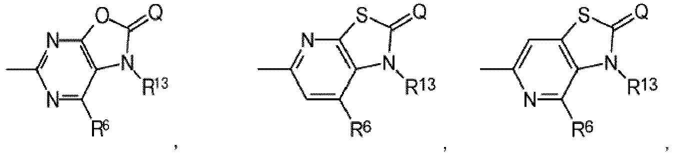 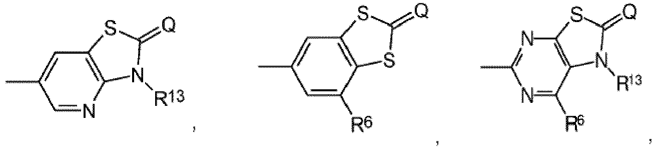 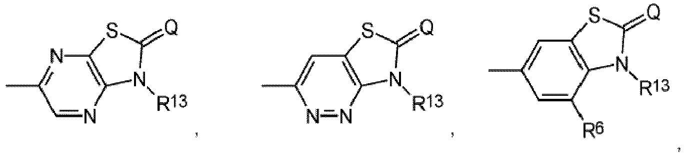 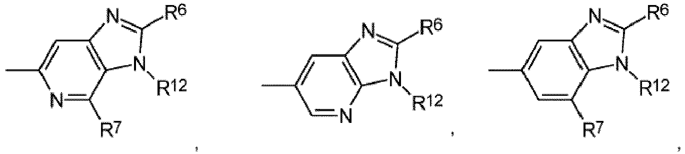 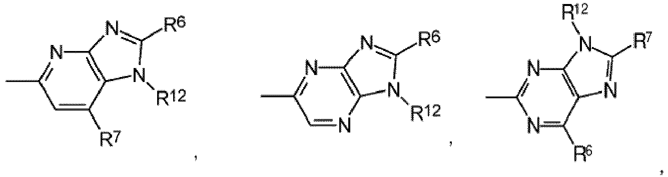 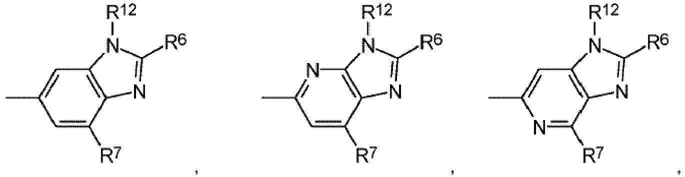 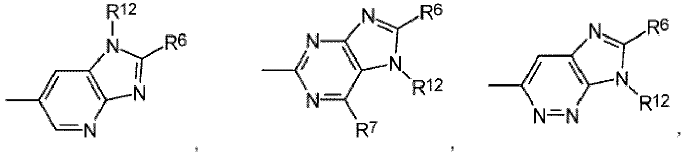 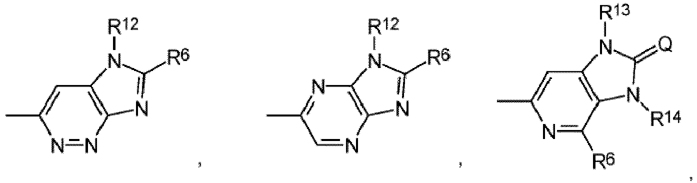 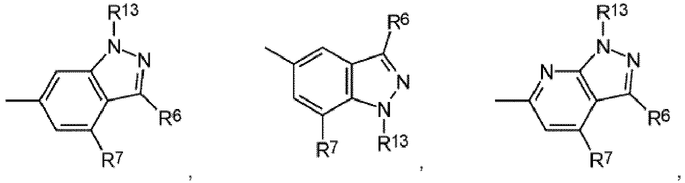 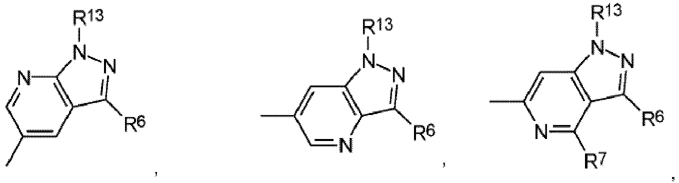 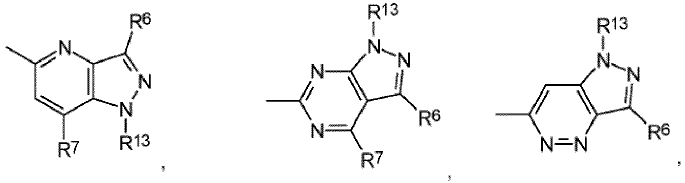 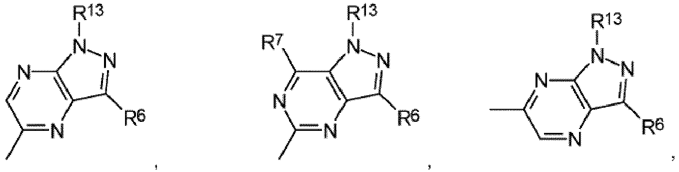 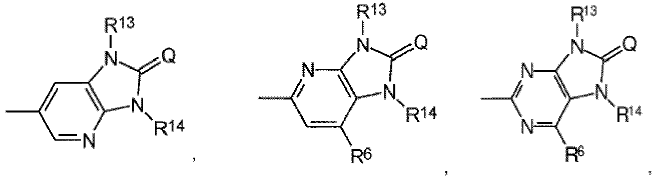 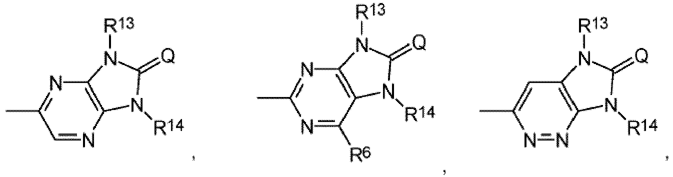 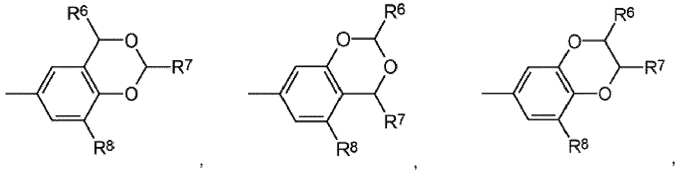 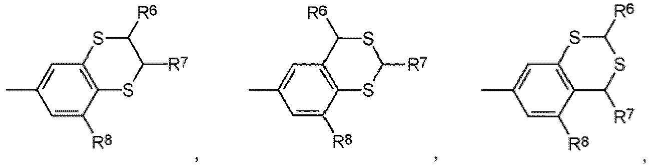 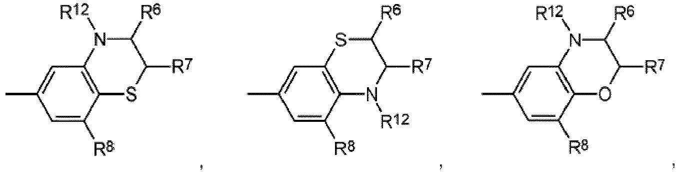 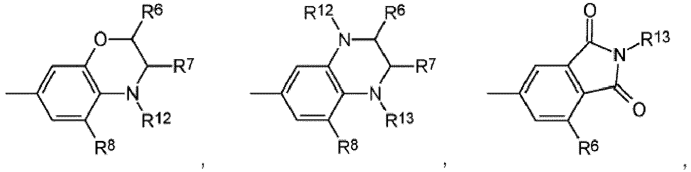 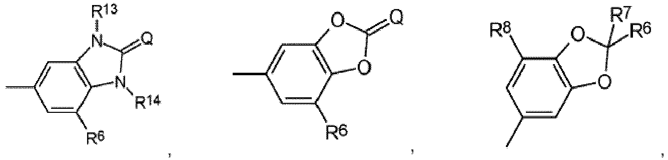 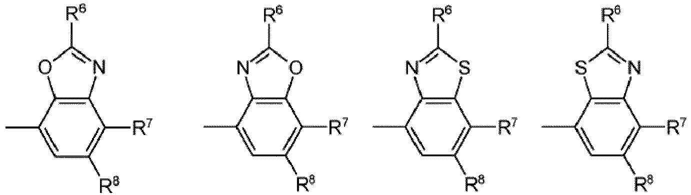 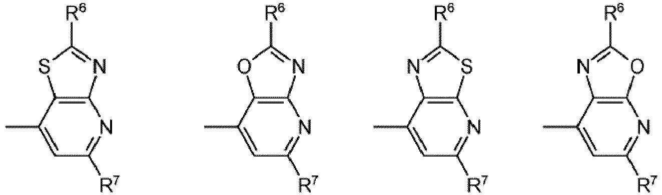 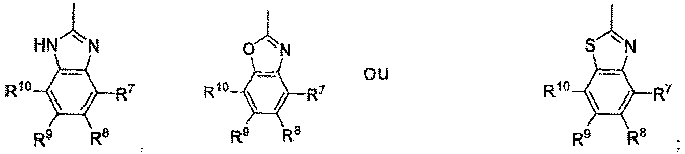
dans lequel **Q** représente =O, =S, ou =N-R¹² ;
**R^{c}** représente -H, -OH, -CH₂-OH, -CHO, -CH₂CHO, -CH₂CH₂CHO, -C₂H₄-OH, - C₃H₆-OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -CH(OAc)-CH₂OH, -CH(OAc)-CH₂OAc, -CH(OH)-CH₂OAc, - CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, - CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, - CH(N(CH₃)₂)CH₃, -CH(N(C₂H₅)₂)CH₃, -CH(NH₂)CH₂OH, -CH(NHCH₃)CH₂OH, - CH(NHC₂H₅)CH₂OH, -CH(N(CH₃)₂)CH₂OH, -CH(N(C₂H₅)₂)CH₂OH, -CH(NH₂)CH₂OCH₃, - CH(NHCH₃)CH₂OCH₃, -CH(NHC₂H₅)CH₂OCH₃, -CH(N(CH₃)₂)CH₂OCH₃, - CH(N(C₂H₅)2)CH₂OCH₃, -CH(NH₂)CH₂OC₂H₅, -CH(NHCH₃)CH₂OC₂H₅, - CH(NHC₂H5)CH₂OC₂H5, -CH(N(CH₃)₂)CH₂OC₂H₅, -CH(N(C₂H₅)₂)CH₂OC₂H₅, -CH(NH₂)CH₂OAc, -CH(NHCH₃)CH₂OAc, -CH(NHC₂H₅)CH₂OAc, -CH(N(CH₃)₂)CH₂OAc, -CH(N(C₂H₅)₂)CH₂OAc, - CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)CH₂OAc, -CH₂-CH(NHCH₃)CH₂OAc, -CH₂-CH(NHC₂H₅)CH₂OA_{C}, -CH₂-CH(N(CH₃)₂)CH₂OAc, -CH₂-CH(N(C₂H₅)₂)CH₂OA_{C}, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -NHSO₂CH₃, -NHSO₂CF₃, -NHSO₂CH₂CF₃, -CH₂-NHSO₂CH₃, -CH₂-NHSO₂CF₃, -CH₂-NHSO₂CH₂CF₃, -C₂H₄-NHSO₂CH₃, -C₂H₄-NHSO₂CF₃, - C₂H₄-NHSO₂CH₂CF₃, -CONH₂, -CONHCH₃, -CONHC₂H₅, -CONHC₃H₇, -NH(C₂H₅), -N(C₂H₅)₂, - CH₂-NH(C₂H₅), -CH₂-N(C₂H₅)₂, -C₂H₄-NH(C₂H₅), -C₂H₄-N(C₂H₅)₂, -NO₂, -CH₂-NO₂, -C₂H₄-NO₂, - CH(OH)-NO₂, -CH(NO₂)-OH, -CO₂H, -CH₂-CO₂H, -C₂H₄-CO₂H, -CH=CH-CO₂H, -CO₂CH₃, - CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, - C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -O-Si(CH₃)₃, -O-Si(C₂H₅)₃, -CO-CHO, -CO-CO-CH₃, -C(OH)-CO-CH₃, -CO-C(OH)-CH₃, -CO-CH₂-CO-CH₃, -C(OH)-CH₂-CO-CH₃, -CO-CH₂-C(OH)-CH₃, -C(OH)-CH₂-C(OH)-CH₃, -F, -Cl, -Br, -CH₂-F, - CHF₂, -CF₃, -C₂H₄-F, -CH₂-CF₃, -CF₂-CF₃, -O-CHF₂, -O-CF₃, -O-CH₂-CF₃, -O-C₂F₅, -CH₃, -CH₂CH₃, -C₃CH₇, -CH(CH₃)₂, -CH=CH₂, -C=CH, -CH₂-CH=CH₂, ou -CH₂-C≡CH ;
**R¹** à **R¹⁰** représentent indépendamment les uns des autres -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, - OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, - C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, - C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, - P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, - Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, - CON(cyclo-C₃H₅)₂, -CON[CH(CH₃)₂]₂, -CON[C(CH₃)₃]₂, -NHCOCH₃, -NHCOC₂H₅, -NHCOC₃H₇, - NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)2, - N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, - SOCH(CH₃)₂, -SOC(CH₃)₃, -SO₂CH₃, -SO₂C₂H₅, -SO₂C₃H₇, -SO₂-cyclo-C₃H₅ , -SO₂CH(CH₃)₂, - SO₂C(CH₃)₃, -SO₃H, -SO₃CH₃, -SO₃C₂H₅, -SO₃C₃H₇, -SO₃-cyclo-C₃H₅, -SO₃CH(CH₃)₂, - SO₃C(CH₃)₃, -SO₂NH₂, -SO₂NHCH₃, -SO₂NHC₂H₅, -SO₂NHC₃H₇, -SO₂NH-cyclo-C₃H₅, - SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -OS(=O)-cyclo-C₃H₅, -O-S(=O)CH(CH₃)₂, -O-S(=O)C(CH₃)₃, -S(=O)(=NH)CH₃, -S(=O)(=NH)C₂H₅, - S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, - C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, - NH-CO-N(C₂H₅)₂, -NH-CO-NH-cyclo-C₃H₅, -NH-CO-N(cyclo-C₃H₅)₂, -NH-CO-N[CH(CH₃)₂]₂, - NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-N H-cyclo-C₃H₅, -NH-C(=NH)-NH-cyclo-C₃H₅, -NH-C(=NH)-NH[CH(CH₃)₂], -O-CO-NH[CH(CH₃)₂], - NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CON(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, - cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, - C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, - CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, - CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, - CH=C(CH₃)-CH=CH₂, -CH=CH-C(CH₃)=CH₂, -C₂H₄-C(CH₃)=CH₂, -CH₂-CH(CH₃)-CH=CH₂, - CH(CH₃)-CH₂-CH=CH₂, -CH₂-CH=C(CH₃)₂, -CH₂-C(CH₃)=CH-CH₃, -CH(CH₃)-CH=CH-CH₃, - CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, - CH(CH₃)-C(CH₃)=CH₂, -C(CH₃)=CH-CH=CH₂, -CH=C(CH₃)-CH=CH₂, -CH=CH-C(CH₃)=CH₂, - C₄H₈-CH=CH₂, -C₃H₆-CH=CH-CH₃, -C₂H₄-CH=CH-C₂H₅, -CH₂-CH=CH-C₃H₇, -CH=CH-C₄H₉, - C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, - CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH2-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, - CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH3)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H5)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C=CH, -C≡C-CH₃, -CH₂-C≡CH, -C₂H₄-C≡CH, -CH₂-C≡C-CH₃, -C=C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C=C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, 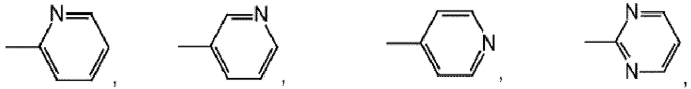 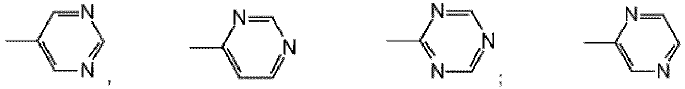
**R¹⁵** représente **-R²⁰,** -CN, -CH₂-CN, -CH₂-OR¹⁷, -CH₂-CH₂-OR¹⁷, -CH₂-NR¹⁷R¹⁸, -CH₂-NR¹⁷COR¹⁹, -CH₂-CH₂-NR¹⁷R¹⁸, -CH₂-CH₂-NR¹⁷COR¹⁹, -CO₂R¹⁷, -CO-NR¹⁷R¹⁸, -CH₂-CO₂R¹⁷, ou -CH₂-CO-NR¹⁷R¹⁸ ;
**R¹⁶, R³⁸, R³⁹** représentent indépendamment les uns des autres **-R²¹,** -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, - CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, - CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, - CH₂OH, -CH₂-SH, -CH(OH)CH₃, -C₂H₄OH, -C₃H₆OH, -C₄H₈OH, -CH(CH₃)-C₂H₄OH, -C₅H₁₀OH, - CH₂-S-CH₃, -CH₂-CH₂-S-CH₃, -C₃H₆-S-CH₃, -CH₂OCH₃, -C₂H₄OCH₃, -C₃H₆OCH₃, -C₄H₈OCH₃, - CH(CH₃)-C₂H₄OCH₃, -C₅H₁₀OCH₃, -CH₂NH₂, -C₂H₄NH₂, -C₃H₆NH₂, -C₄H₈NH₂, -CH(CH₃)-C₂H₄NH₂,-C₅H_{IO}NH₂, -CH₂-CH₂-CH₂-NH-C(NH)NH₂, -CH₂-CO₂H, -CH₂-CONH₂, -CH₂-CH₂-CO₂H, -CH₂-CH₂-CONH₂, -CH₂-CO₂CH₃, -CH₂-CONHCH₃, -CH₂-CON(CH₃)₂, -CH₂-CH₂-CO₂CH₃, -CH₂-CH₂-CONHCH₃, -CH₂-CH₂-CONH(CH₃)₂, -CH=CH-CO₂H, -CH=CH-CO₂CH₃, -CH=CH-CONHCH₃, -CH=CH-CONHC₂H₅, -CH=CH-CON(CH₃)₂, -CH=CH-CON(C₂H₅)₂, -CH₂-CH=CH-CO₂H, -CH₂-CH=CH-CO₂CH₃, -CH₂-CH=CH-CONHCH₃, -CH₂-CH=CH-CON(CH₃)₂, -CH₂-CH=CH-CONHC₂H₅, -CH₂-CH=CH-CON(C₂H₅)₂, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, - CH=CH-C₃H₇, -CH₂-CH=CH-CH=CH₂, -CH=CH-CH=CH-CH₃, -CH=CH-CH₂-CH=CH₂, - C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, - C(CH₃)2-CH=CH₂, -CH(CH₃)-C(CH₃)=CH₂, -C(CH₃)=CH-CH=CH₂, -CH=C(CH₃)-CH=CH₂, - CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH-CH(CH₃)-CH₂-CH=CH₂, - C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)_{3]}=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, - C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, - CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH3)(C₂H₅)]=CH₂, -C[CH₂-CH(CH₃)_{2]}=CH₂, -C₂H₄-CH=CH-CH=CH₂, - CH₂-CH=CH-CH₂-CH=CH₂, -C₃Hₛ-C≡C-CH₃, -CH₂-CH=CH-CH=CH-CH₃, -CH=CH-CH=CH-C₂H₅, - CH₂-CH=CH-C(CH₃)=CH₂, -CH₂-CH=C(CH₃)-CH=CH₂, -CH₂- C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, - CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, - CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, - C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, - CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H_{S})-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, - C(CH₃)(C₂H₅)-C≡CH, -CH₂-Ph, 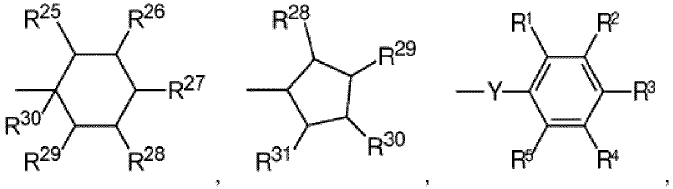 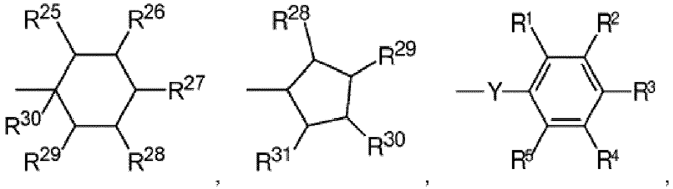 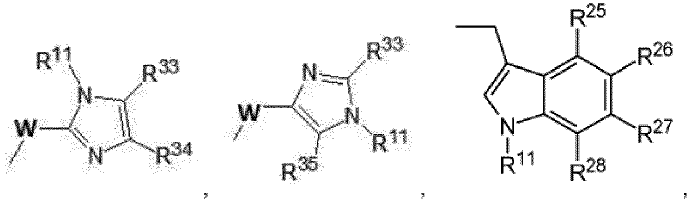 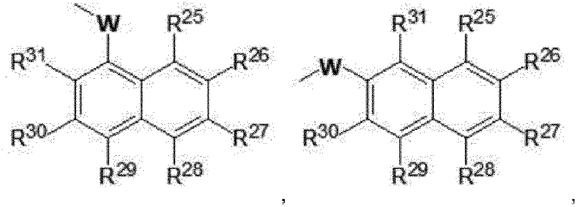 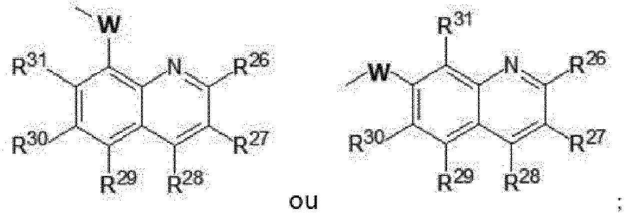
dans lequel **W** représente O, N-R¹², S ;
**R¹¹** à **R¹⁴** et **R¹⁷** à **R²¹** représentent indépendamment les uns des autres -H, -CH₂F, -CHF₂, - CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, - C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, - CH₂-OCH₂-Ph, -C₂H₄-OCH₂-Ph, -C₃H₆-OCH₂-Ph, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, - cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, - C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, - CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, - CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, - CH=C(CH₃)-CH=CH₂, -CH=CH-C(CH₃)=CH₂, -C₂H₄-C(CH₃)=CH₂, -CH₂-CH(CH₃)-CH=CH₂, - CH(CH₃)-CH₂-CH=CH₂, -CH₂-CH=C(CH₃)₂, -CH₂-C(CH₃)=CH-CH₃, -CH(CH₃)-CH=CH-CH₃, - CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, - CH(CH₃)-C(CH₃)=CH₂, -C(CH₃)=CH-CH=CH₂, -CH=C(CH₃)-CH=CH₂, -CH=CH-C(CH₃)=CH₂, - C₄H₈-CH=CH₂, -C₃H₆-CH=CH-CH₃, -C₂H₄-CH=CH-C₂H₅, -CH₂-CH=CH-C₃H₇, -CH=CH-C₄H₉, - C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, - CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH2-CH=CH₂, -CH₂-C(CH₃)=C(CH₃)₂, - CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C=C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, ou -CH₂-CH(C≡CH)₂ ;
**R²² à R³⁷** représentent indépendamment les uns des autres -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, - OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H_{d}-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, - C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, - P(O)(OH)₂, -P(O)(OCH₃)₂, -P(O)(OC₂H₅)₂, -P(O)(OCH(CH₃)₂)₂, -C(OH)[P(O)(OH)₂]₂, - Si(CH₃)₂(C(CH₃)₃), -Si(C₂H₅)₃, -Si(CH₃)₃, -N₃, -CN, -OCN, -NCO, -SON, -NCS, -CHO, -COCH₃, - COC₂H₅, -COC₃H₇, -CO-cyclo-C₃H₅, -COCH(CH₃)₂, -COC(CH₃)₃, -COOH, -COCN, -COOCH₃, - COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, - CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, - CON[C(CH₃)₃]₂, -NHCOCH₃, -NHCOC₂H₅, -NHCOC₃H₇, -NHCO-cyclo-C₃H₅ , -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, - NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, - N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, - SO₂NHC₃H₇, -SO₂N H-cyclo-C₃H₅, -SO₂NHCH(CH₃)₂, -SO₂NHC(CH₃)₃, -SO₂N(CH₃)₂, - SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -OS(=O)CH₃, -O-S(=O)C₂H₅, -O-S(=O)C₃H₇, -O-S(=O)-cyclo-C₃H₅, -O-S(=O)CH(CH₃)₂, -OS(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, - O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)_{2]}, -NH-CS-NH[C(CH₃)_{3]}, -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂]-O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, - NH-C(=NH)-N[CH(CH₃)₂]₂, -NH-C(=NH)-N[C(CH₃)₃]₂, -O-CO-NH2, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CON(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph,-CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, - C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, - C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, - CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, - CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH=CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H5, -CH₂-C(CH₃)=CH-C₂H₅, -CH(CH₃)-CH=CH-C₂H₅, -CH=CH-CH₂-CH(CH₃)₂, - CH=CH-CH(CH₃)-C₂H₅, -CH=C(CH₃)-C₃H₇, -C(CH₃)=CH-C₃H₇, -CH₂-CH(CH₃)-C(CH₃)=CH₂, - C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, - CH=C(CH₃)-CH(CH₃)₂, -C(CH₃)=CH-CH(CH₃)₂, -C(CH₃)=C(CH₃)-C₂H₅, -CH=CH-C(CH₃)₃, - C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, - C(C₃H₇)=CH-CH₃, -C(C₂H₅)=CH-C₂H₅, -C(C₂H₅)=C(CH₃)₂, -C[CH(CH3)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, - C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C=C-C₂H₅, -C₃H₆-C≡CH, -C₂H₄-C≡C-CH₃, -CH₂-C≡C-C₂Hₛ, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH,-CH(CH₃)-C≡C-C≡CH, -CH(C≡CH)-CH₂-C≡CH, -CH(C≡CH)-C≡C-CH₃, 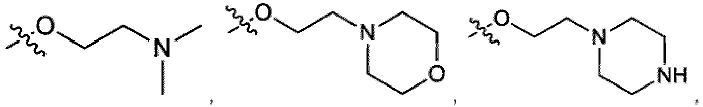 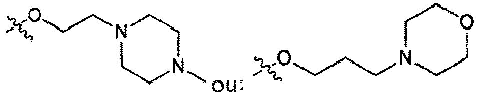
et les anomères, les formes désoxy, les tautomères, les hydrates, et les solvates des composés mentionnés ci-dessus et les sels pharmaceutiquement acceptables.

2. Composé selon la revendication 1, dans lequel le composé est représenté par la formule (V) suivante : 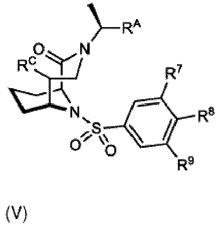
et dans lequel les substituants **R^{A} R^{B}, R^{c}** et **R⁷ à R⁹** ont les mêmes significations que celles définies pour la formule (I) dans ce document ;
ou dans lequel le composé est représenté par les formules (VI) ou (VIa) suivantes : 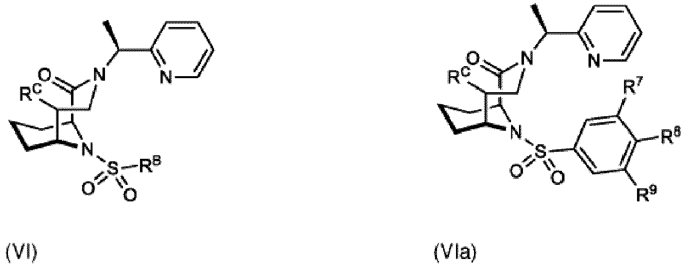
et dans lequel les substituants **R^{B}**, **R^{C}** et **R⁷ à R⁹** ont les mêmes significations que celles définies pour la formule (I) dans ce document ;
ou dans lequel le composé est représenté par les formules (VII) ou (VIIa) suivantes : 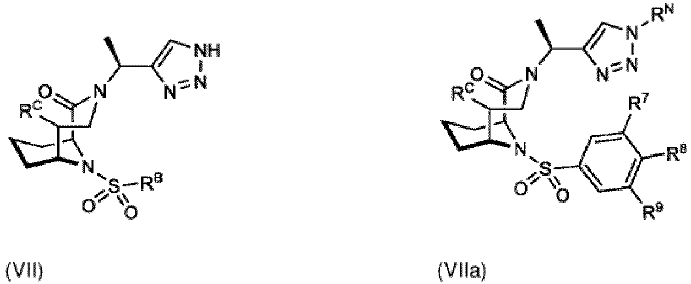
et dans lequel les substituants **R^{B}**, **R^{C}**, **R^{N} et R⁷ à R⁹** ont les mêmes significations que celles définies pour la formule (I) dans ce document ;
ou dans lequel le composé est représenté par les formules (VIII) ou (Villa) suivantes : 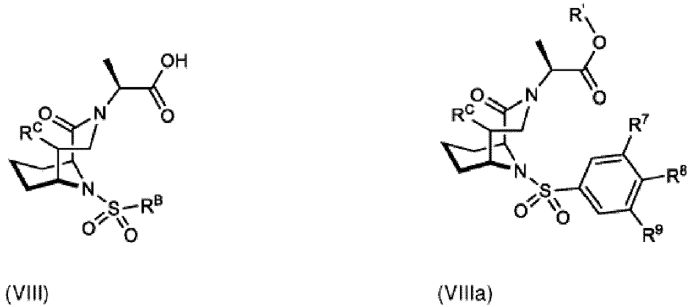
et dans lequel les substituants **R^{B}**, **R^{C}**, **R^{N}**, **R'** et **R⁷ à R⁹** ont les mêmes significations que celles définies pour la formule (I) dans ce document ;
ou dans lequel le composé est représenté par les formules (IX) ou (X) suivantes : 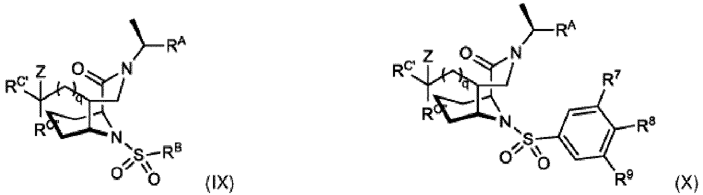
et dans lequel les substituants **R^{A}**, **R^{B}** et **R⁷ à R⁹** ont les mêmes significations que celles définies pour la formule (I) dans ce document ;
R^{c'} et R^{c"} représentent indépendamment l'un de l'autre, -H, -CH₃, -C₂H₅, -CH₂-OH ; Z représente -OH, -OCH₃, -OC₂H₅, -OCH(CH₃)₅, -NH₂, -NH(CH₃), -NH(C₂H₅), -NHCH(CH₃)₂, - N(CH₃)₂, ou -N(O₂H₅) ; et q est 0 ou 1 ;
et les anomères, les formes désoxy, les tautomères, les hydrates et les solvates des composés mentionnés ci-dessus et les sels pharmaceutiquement acceptables.

3. Composé selon l'une quelconque des revendications précédentes, dans lequel le composé est représenté par la formule (V) suivante : 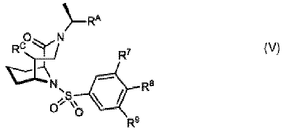
dans lequel **R^{A}** est sélectionné parmi : 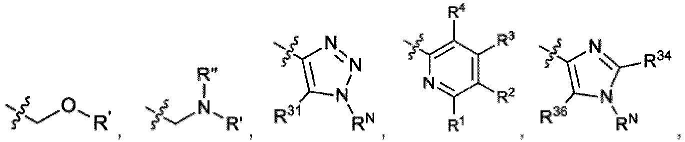 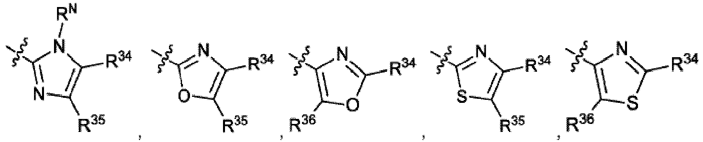 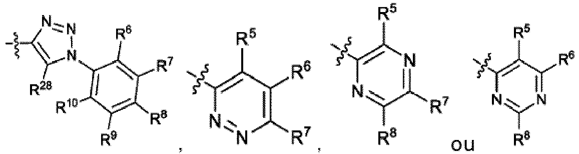
**R^{c}** a la signification définie pour la formule (I) dans ce document ;
**R⁷ à R⁹** sont sélectionnés indépendamment les uns des autres parmi : -H, -F, -Cl, -Br, -l, -OH, -OCH₃, OC₂H₅, -OC₃H₇, -CN, -CONH₂, -NHCOCH₃, -NHCOC₂H₅, -NHCOC₃H₇, -COOH, - COOCH₃, -COOC₂H₅, -COOC₃H₇, -Ph, 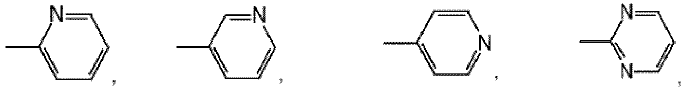 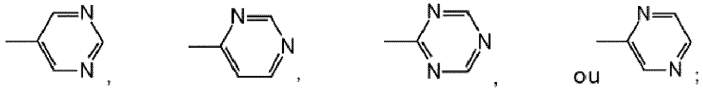
et les anomères, les formes désoxy, les tautomères, les hydrates, et les solvates des composés mentionnés ci-dessus et les sels pharmaceutiquement acceptables.

4. Utilisation du composé selon l'une quelconque des revendications précédentes en tant qu'inhibiteur des protéines de liaison au FK506, dans laquelle l'inhibition a lieu hors du corps humain ou animal.

5. Utilisation selon les revendications 1-3, en tant qu'inhibiteur de FKBP12, FKBP12.6, FKBP51, FKBP52, LpMIP, CpMIP, CtMIP BpMIP, et TcMIP, dans laquelle l'inhibition a lieu hors du corps humain ou animal.

6. Composé selon les revendications 1-3 pour une utilisation tant que principe actif dans un médicament.

7. Composé selon les revendications 1-3 pour une utilisation dans le traitement ou la prophylaxie de troubles psychiatriques, de troubles neurologiques, de maladies métaboliques, de cancers, de syndromes d'hypersensibilité aux glucocorticoïdes, de résistance périphérique aux glucocorticoïdes, de maladies infectieuses, d'une alopécie, d'une pression intraoculaire anormalement élevée, d'une dégénérescence maculaire, d'une lésion oxydative des tissus oculaires, d'un trouble de la vision, de troubles du sommeil, d'un asthme, d'un diabète, d'une lésion cérébrale traumatique, d'une lésion nerveuse, d'une maladie d'Alzheimer, d'une maladie d'Huntington, d'une maladie de Parkinson, d'une ischémie, d'une altération de la mémoire et pour la neuroprotection, la neurorégénération, la promotion de la croissance pileuse, la stimulation de la croissance des neurites, la cicatrisation des plaies, des médicaments antiglaucomateux, l'amélioration de la vision, l'amélioration des performances de mémoire, pour une utilisation dans le traitement ou la prévention de la résistance multi-médicamenteuse, pour une utilisation dans l'interruption de grossesse et l'inhibition de la nidation, pour une utilisation dans la limitation ou la prévention d'une hémorragie ou d'une néovascularisation et pour une utilisation dans le traitement de maladies associées à la neurodégénérescence.

8. Composé pour une utilisation selon la revendication 7, dans lequel la maladie psychiatrique est un trouble affectif ou un trouble anxieux et/ou la maladie métabolique est une adiposité localisée, un syndrome métabolique ou une obésité et/ou le cancer est un cancer de la prostate, une leucémie lymphoblastique aigüe ou un mélanome et/ou dans lequel le trouble affectif est sélectionné dans le groupe constitué de : une dépression, un trouble bipolaire, une manie, un trouble de l'humeur induit par une substance et un trouble affectif saisonnier (TAS) et/ou dans lequel la dépression est sélectionnée parmi une dépression majeure ou un trouble dépressif majeur et/ou dans lequel le trouble anxieux est sélectionné dans le groupe comprenant ou constitué d'un trouble anxieux généralisé, d'un trouble panique, d'un trouble panique avec agoraphobie, de phobies, d'un trouble obsessionnel compulsif, d'un trouble de stress post-traumatique, de troubles de l'angoisse de séparation et anxieux de l'enfance et/ou dans lequel la maladie infectieuse est sélectionnée dans le groupe constitué de la maladie de Chagas, d'infections à Chlamydia, d'infections à Burkholderia, de la légionellose, de l'obésité, du diabète, d'une douleur chronique, d'une douleur neuropathique, d'une fibromyalgie, d'une télangiectasie hémorragique héréditaire, d'une hypertension artérielle pulmonaire, d'un cancer de la prostate, d'un mélanome ou d'un glioblastome.

9. Composition pharmaceutique comprenant au moins un composé selon les revendications 1-3 conjointement à au moins un vecteur, solvant ou excipient pharmaceutiquement acceptable ou conjointement à au moins un vecteur, solvant ou excipient pharmaceutiquement acceptables et au moins un agent actif sélectionné dans le groupe constitué d'un anti-dépresseur et d'autres médicaments psychotropes, ainsi que de médicaments anti-diabétiques, d'analgésiques et/ou d'antibiotiques.

10. Composition pharmaceutique selon la revendication 9, dans lequel l'antidépresseur est sélectionné parmi l'amitriptyline, l'amioxid, la clomipramine, la doxépine, la duloxetine, l'imipramine, la trimipramine, la mirtazapine, la réboxetine, le citaloprame, la fluoxétine, le moclobémide et la sertraline.
